(19) Europäisches Patentamt
European Patent Office
Office européen des brevets

(11) **EP 3 502 699 A1**

(12) **EUROPEAN PATENT APPLICATION**

(43) Date of publication:
**26.06.2019 Bulletin 2019/26**

(51) Int Cl.:
***G01N 33/574*** (2006.01)   ***G01N 33/68*** (2006.01)

(21) Application number: **17209024.3**

(22) Date of filing: **20.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicants:
- **metanomics Health GmbH**
  **10589 Berlin (DE)**
- **Universitätsmedizin Greifswald**
  **17475 Greifswald (DE)**

(72) Inventors:
- **KAMLAGE, Beate**
  **12161 Berlin (DE)**
- **CHRISTIANSEN, Nicole**
  **10589 Berlin (DE)**

- **SCHATZ, Philipp**
  **10435 Berlin (DE)**
- **PILARSKY, Christian**
  **91080 Marloffstein (DE)**
- **GRUETZMANN, Robert**
  **91054 Erlangen (DE)**
- **BAHRA, Marcus**
  **10119 Berlin (DE)**
- **MAYERLE, Julia**
  **80469 München (DE)**
- **LERCH, Markus**
  **17475 Greifswald (DE)**
- **CHROMIK, Ansgar Michael**
  **44791 Bochum (DE)**

(74) Representative: **Herzog, Fiesser & Partner**
**Patentanwälte PartG mbB**
**Dudenstrasse 46**
**68167 Mannheim (DE)**

(54) **METHODS FOR DIAGNOSING PANCREATIC CANCER**

(57)   The present invention relates to a method for diagnosing pancreatic cancer in a subject suspected to suffer from pancreatic cancer comprising the steps of: (a) determining in a sample of said subject the value of (i) at least one biomarker of the categories carnitines, amino acids, and mitochondrial energy metabolites, and (ii) a first and a second sphingolipid, wherein the fatty acid residue of the first sphingolipid is at least four carbon atoms longer than the fatty acid residue of the second sphingolipid, and wherein the sphingosine backbone of the first sphingolipid and the second sphingolipid are identical; and (b) comparing the values of the biomarkers with references, whereby pancreatic cancer is diagnosed; and to a method for identifying whether a subject is in need of a pancreatic cancer therapy related thereto. The present invention further relates to a device for diagnosing pancreatic cancer in a sample of a subject comprising (a) an analyzing unit for the sample of the subject comprising a detector for the amounts of at least one biomarker each of the categories carnitines, amino acids, energy metabolites, and sphingolipids; said detector allowing for the determination of the amounts of the biomarkers of the said group of biomarkers in the sample; and operatively linked thereto, (b) an evaluation unit comprising a data processing unit and a data base, said data base comprising a stored reference and said data processing unit having tangibly embedded an algorithm for carrying out a comparison of the amounts of the biomarkers of the group of biomarkers determined by the analyzing unit and the stored reference and for generating an output information based on which the diagnosis can be established. Further, the present invention relates to a data collection and too a use related to the aforesaid subject matter.

EP 3 502 699 A1

Printed by Jouve, 75001 PARIS (FR)

**Description**

The present invention relates to a method for diagnosing pancreatic cancer in a subject suspected to suffer from pancreatic cancer comprising the steps of: (a) determining in a sample of said subject the value of (i) at least one biomarker of the categories carnitines, amino acids, and mitochondrial energy metabolites, and (ii) a first and a second sphingolipid, wherein the fatty acid residue of the first sphingolipid is at least four carbon atoms longer than the fatty acid residue of the second sphingolipid, and wherein the sphingosine backbone of the first sphingolipid and the second sphingolipid are identical; and (b) comparing the values of the biomarkers with references, whereby pancreatic cancer is diagnosed; and to a method for identifying whether a subject is in need of a pancreatic cancer therapy related thereto. The present invention further relates to a device for diagnosing pancreatic cancer in a sample of a subject comprising (a) an analyzing unit for the sample of the subject comprising a detector for the amounts of at least one biomarker each of the categories carnitines, amino acids, energy metabolites, and sphingolipids; said detector allowing for the determination of the amounts of the biomarkers of the said group of biomarkers in the sample; and operatively linked thereto, (b) an evaluation unit comprising a data processing unit and a data base, said data base comprising a stored reference and said data processing unit having tangibly embedded an algorithm for carrying out a comparison of the amounts of the biomarkers of the group of biomarkers determined by the analyzing unit and the stored reference and for generating an output information based on which the diagnosis can be established. Further, the present invention relates to a data collection and to a use related to the aforesaid subject matter.

Pancreatic cancer has the worst prognosis of all solid tumors, with 5-year survival rates of less than 5% but an increasing incidence (Everhart 2009, Gastroenterology 136:1134-11449). There is a widely acknowledged demand for the establishment of innovative tools and technologies for point-of-care utilization of specific biomarkers and novel molecular imaging tools for early diagnosis, prognostic stratification and differential diagnosis of pancreatic cancer. Advances in these areas are pivotal to improve the prognosis of this malignancy, since timely surgical resection of early stage tumors is currently the only effective means of treatment of this dismal disease.

The mortality of this cancer type is the highest of any cancer type in Europe and the western world. People die soon after diagnosis due to the lack of means for early detection. Early symptoms are rare and uncharacteristic. Thus, pancreatic ductal adenocarcinomas (PDACs) are commonly diagnosed in an advanced stage of the disease. To date, the best imaging technologies to detect PDAC are endoscopic ultrasound (EUS), spiral computer tomography (CT), magnetic resonance cholangiopancreatography (MRCP) or endoscopic retrograde cholangiopancreatography (ERCP) (Dewitt 2006, Gastroenterol Hepatol. (4):717-25). Unfortunately, the resolution of these technologies for detecting neoplastic lesions within the pancreas is in the range of 3-10 mm. Thus, they are not able to detect pancreatic neoplasia at a curable stage. The serum concentration of conventional tumor markers such as CA19-9 is increased in a subset of pancreatic cancer patients (Fry 2008, Langenbecks Arch Surg. (393): 883-90). However, so far all available markers lack sensitivity and tumor specificity (Gupta et al., 1985, Cancer 56 (277-283)). Thus, new approaches are urgently needed to increase the diagnostic sensitivity towards the detection of very small, early stage PDAC and its precursor lesions (PanINs and IPMNs) as well as prognostic subgroups of advanced tumors.

The association between chronic inflammation and the development of malignancies has been recognized for many years. For pancreatic cancer, this association was only recently confirmed and a consensus conference agreed upon a new classification for pancreatic intraepithelial neoplasia as noninvasive precursor lesions (Hruban 2004, Am J Surg Path (28): 977-987). Chronic pancreatitis is defined as recurrent bouts of a sterile inflammatory disease characterized by often progressive and irreversible morphological changes, typically causing pain and permanent impairment of pancreatic function. With an incidence of 8.2, a prevalence of 27.4 per 100 000 population and a 0.04% to 5% frequency in unselected autopsy specimens, chronic pancreatitis represents a frequent disorder of the gastrointestinal tract. Various etiologies are responsible for the development of chronic pancreatitis. An increased risk of patients suffering from of chronic pancreatitis to die from pancreatic cancer was shown in an international cooperative investigation conducted by AB Lowenfels and coworkers as a multicenter historical cohort study of 2015 patients with chronic pancreatitis recruited from clinical centers in 6 countries in 1993. This study found a cumulative risk of pancreatic cancer in patients with chronic pancreatitis of 1.8% after 10 years and of 4% after 20 years with a standardized incidence ratio of 14.4. For patients with a minimum of two years follow up the risk of pancreatic cancer was 16.5 fold higher than that of the general population (Lowenfels 1993, N Engl J Med (328): 1433-1437). The search for an association between chronic pancreatitis and pancreatic cancer intensified when in 1996 a single point mutation in the third exon of the cationic trypsinogen gene on chromosome 7 (7q35) was found to be associated with hereditary pancreatitis and multiple kindreds were subsequently identified and reported. Only very recently the EUROPAC study group presented their work on clinical and genetic characteristics in hereditary pancreatitis. In a multilevel proportional hazard model employing data obtained from the European Registry of Hereditary Pancreatitis this group presented 112 families in 14 countries (418 affected individuals) (Howes 2004, Clinical Gastroenterology and Hepatology (2): 252-261). The cumulative risk (95% CI) of pancreatic cancer was 44.0% (8.0% - 80.0%) at 70 years from symptom onset with a standardized incidence ratio of 67% (50% - 82%). A previous study had also shown an estimated lifetime risk of pancreatic cancer of 40% (Lowenfels 2001, JAMA 286:

169-170, Lowenfels 1997, J Natl Cancer Inst 89: 442-44656).

Diabetes mellitus (DM) and pancreatic cancer (PDAC) are associated diseases that have a not completely understood relationship with each other (Sah et al. 2013, Nat Rev Gastroenterol Hepatol 10:423-433). On the one hand, long-standing DM is a low to moderate risk factor for pancreatic cancer (Muniraj and Chari 2012, Minerva gastroenterologica e dietologica 58:331-345.). On the other hand, new onset of DM at advanced age, can be of paraneoplastic character and the first symptom of pancreatic cancer before other symptoms arise (Chari et al. 2008, Gastroenterology 134:95-101) and therefore could be a valuable clue to detect PDAC early at a still curable stage. This association is temporal for about two to three years following the initial DM diagnosis. Data on incidence of PDAC in new onset of DM is rare, numbers of 0.25% (Munigala et al. 2015 Clin Transl Gastroenterol 6:e118), 0.85% (Chari et al. 2005, Gastroenterology 129:504-511), and 3.6% (Choe et al. 2016, Pancreas 45:730-734) have been reported.

In pancreatic cancer, imaging studies fail to detect early pancreatic malignancies in a curable stage. Thus, the detection of pancreatic malignancy in a high risk cohort would be highly desired.

There are a few reports of metabolic changes in patients suffering from pancreas-associated diseases. Schrader et al (Schrader 2009, Pancreas 38: 416-421) suggests that patients with pancreatic cancer and chronic pancreatitis show significant changes in serum amino acid levels. It has been suggested that sphingolipids on the cell surface of cells takes actively part in cell signaling (Pitson 2011, Trend Biochem Sci 36:97-107). Ceramides are known to induce apoptosis in cancer cells. Low levels of sphingomyelin suggest less responsiveness to gemcitabine treatment (Modrak 2009, Mol Cancer Res 7:890-896). Further single metabolic biomarkers have been reported in WO 2011/151252 and WO 2013/079594.

CA 19-9 blood levels are elevated in many patients with pancreatic cancer. The CA19-9 level is of limited value for pancreatic cancer diagnostic in terms of both sensitivity and specificity. CA19-9 sensitivity for pancreatic cancer diagnostic is impaired by false positives due to other gastrointestinal cancers such as colon cancer, gastric cancer, and liver cancer, as well as breast cancer and other gynecological cancer, lung cancer, and bronchial cancer. Benign diseases such as pancreatitis also result in false positive CA19-9 levels. CA19-9 specificity for pancreatic cancer diagnostic is further impaired by false negatives patients that are negative for Lewis a/b antigen and will therefore not express CA19-9.

In conclusion, with a 5-year survival rate of 0.5-5%, pancreatic cancer carries the most dismal prognosis of all human tumors and represents the 4th leading cause in cancer-related deaths worldwide. It is thus a disease with a major socioeconomic impact. Accurate diagnosis including its differentiation from pancreatitis and timely surgical resection of early tumors currently offer the only realistic prospect for the improvement of patient prognosis.

The technical problem underlying the present invention can be seen as the provision of means and methods for complying with the aforementioned needs. The technical problem is solved by the embodiments characterized in the claims and herein below.

Accordingly, the present invention relates to a method for diagnosing pancreatic cancer in a subject suspected to suffer from pancreatic cancer comprising the steps of:

(a) determining in a sample of said subject the value of (i) at least one biomarker of the categories carnitines, amino acids, and mitochondrial energy metabolites, and (ii) a first and a second sphingolipid, wherein the fatty acid residue of the first sphingolipid is at least four carbon atoms longer than the fatty acid residue of the second sphingolipid, and wherein the sphingosine backbone of the first sphingolipid and the second sphingolipid are identical; and

(b) comparing the values of the biomarkers with references, whereby pancreatic cancer is diagnosed.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value $\pm$ 20%, more preferably $\pm$ 10%, most preferably $\pm$ 5%. Further, the term "essentially" indicates that deviations having influence on the indicated result or use are absent, i.e. potential deviations do not cause the indicated result to deviate by more than $\pm$ 20%, more preferably $\pm$ 10%, most preferably $\pm$ 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1 % by weight of non-specified component(s).

The method as referred to in accordance with the present invention includes a method which essentially consists of the aforementioned steps or a method which includes further steps. However, it is to be understood that the method, in a preferred embodiment, is a method carried out ex vivo, i.e. not practiced on the human or animal body. The method, preferably, is assisted by automation. For example, sample processing or pre-treatment can be automated by robotics. Data processing and comparison is, preferably, assisted by suitable computer programs and databases, preferably as described elsewhere herein. Automation as described herein, preferably, allows using the method of the present invention in high-throughput approaches.

The term "diagnosing" as used herein refers to assessing whether a subject suffers from pancreatic cancer, or not. As will be understood by those skilled in the art, such an assessment, although preferred to be, may usually not be correct for 100% of the investigated subjects. The term, however, requires that a statistically significant portion of subjects can preferably be correctly assessed and, thus, diagnosed. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.1, 0.05, or 0.01. The term includes individual diagnosis of pancreatic cancer or its symptoms as well as continuous monitoring of a patient. Monitoring, i.e. diagnosing the presence or absence of pancreatic cancer or the symptoms accompanying it at various time points, includes monitoring of patients known to suffer from pancreatic cancer as well as monitoring of subjects known to be at risk of developing pancreatic cancer. Preferred subjects known to be at risk of developing pancreatic cancer are subjects suffering from new-onset diabetes, as specified elsewhere herein. Furthermore, monitoring can also be used to determine whether a patient is treated successfully or whether at least symptoms of pancreatic cancer can be ameliorated over time by a certain therapy.

The terms "pancreatic cancer" and "pancreas cancer", as used herein, relate equally to neoplasms which are derived from pancreas cells and, preferably, from pancreatic epithelial cells. Thus, preferably, pancreatic cancer as used herein is pancreatic adenocarcinoma, more preferably pancreatic ductal adenocarcinoma (PDAC). Preferably, the pancreatic cancer is a resectable pancreatic cancer, i.e., preferably, is a pancreatic cancer at a tumor stage permitting, preferably complete, resection of the tumor from the subject. More preferably, said pancreatic cancer is a pancreatic cancer of tumor stage IA-IIB. The symptoms accompanying pancreatic cancer are well known from standard text books of medicine such as Stedmen or Pschyrembl and include severe abdominal pain, lower back pain, and in some cases jaundice.

The term "biomarker", as used herein, refers to a molecular species which serves as an indicator for a disease or effect as referred to in this specification. Said molecular species can be a metabolite itself which is found in a sample of a subject. Moreover, the biomarker may also be a molecular species which is derived from said metabolite. In such a case, the actual metabolite will be chemically modified in the sample or during the determination process and, as a result of said modification, a chemically different molecular species, i.e. an analyte, will be the determined molecular species. Preferred chemical modifications may depend on the analytical method used and are described elsewhere herein. It is to be understood that in such a case, the analyte represents the actual metabolite and has the same potential as an indicator for the respective medical condition. Moreover, a biomarker according to the present invention is not necessarily corresponding to one molecular species. Rather, the biomarker may comprise stereoisomers or enantiomeres of a compound. Further, a biomarker can also represent the sum of isomers of a biological class of isomeric molecules. Said isomers shall exhibit identical analytical characteristics in some cases and are, therefore, not distinguishable by various analytical methods including those applied in the accompanying Examples described below. However, the isomers will share at least identical sum formula parameters and, thus, in the case of, e.g., lipids an identical chain length and identical numbers of double bonds in the fatty acid and/or sphingo base moieties. Further, depending on sample treatment, a biomarker may also represent a group of metabolites, e.g. after transmethanolysis, a specific fatty acid methylester may represent all metabolites originally present in a sample being or comprising said specific fatty acid.

A "metabolite", as used herein, refers to at least one molecule of a specific metabolite up to a plurality of molecules of the said specific metabolite. It is to be understood further that a group of metabolites means a plurality of chemically different molecules wherein for each metabolite at least one molecule up to a plurality of molecules may be present. A metabolite in accordance with the present invention may be from all classes of organic or inorganic chemical compounds including those being comprised by biological material such as organisms. Preferably, the metabolite in accordance with the present invention is a small molecule compound, the term "small molecule" referring to chemical compounds with a molecular mass of less than 5000 u (5 kDa, 1 u = 1.66 x 10$^{-27}$ kg), more preferably less than 1000 u. Thus, preferably, the biomarker is a small molecule biomarker, more preferably of less than 5 kDa, most preferably less than 1 kDa. In case the value of a biomarker is a ratio or another value derived from the amount of more than one biomarker, a value of a small molecule biomarker is derived exclusively from small molecule biomarkers.

The term "determining the amount" as used herein refers to determining at least one characteristic feature of a biomarker to be determined by the method of the present invention in the sample. Characteristic features in accordance with the present invention are features which characterize the physical and/or chemical properties including biochemical properties of a biomarker. Such properties include, e.g., molecular weight, viscosity, density, electrical charge, spin, optical activity, color, fluorescence, chemoluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the m/z information, as well as an intensity value being related to the abundance of the said biomarker (i.e. its amount) in the sample.

The amount of a biomarker comprised by a sample may be, preferably, determined quantitatively or semi-quantitatively. For quantitative determination, either the absolute or precise amount of the biomarker will be determined or the relative amount of the biomarker will be determined based on the value determined for the characteristic feature(s) referred to herein above. The relative amount may be determined in a case were the precise amount of a biomarker can or shall not be determined. In said case, it can preferably be determined whether the amount in which the biomarker is present is enlarged or diminished with respect to a second sample comprising said biomarker in a second amount. Quantitatively analysing a biomarker, thus, also includes what is sometimes referred to as semi-quantitative analysis of a biomarker. As is understood by the skilled person, the above applies to other measures of an amount of a compound, in particular a concentration, mutatis mutandis.

Preferably, determining comprises a sample pre-treatment step. Preferably, such pre-treatment may include treatments required to release or separate the compounds or to remove excessive material or waste. Suitable techniques comprise centrifugation, extraction, fractioning, ultrafiltration, protein precipitation followed by filtration and purification and/or enrichment of compounds. Preferably, other pre-treatments are carried out in order to provide the compounds in a form or concentration suitable for compound analysis. For example, if gas-chromatography coupled mass spectrometry is used in the method of the present invention, it may be required to derivatize the compounds prior to the said gas chromatography. Suitable and necessary pre-treatments depend on the means used for carrying out the method of the invention and are well known to the person skilled in the art. Samples pre-treated as described herein are also comprised by the term "sample" specified herein below.

Preferably, determining as used in the method of the present invention, includes using at least one compound separation step prior to the analysis step referred to before. Preferably, the compound separation step is a precipitation step, preferably causing precipitation of macromolecules comprised in the sample, in particular polypeptides. Preferably, said step comprises addition of from one to five volumes of a solution comprising an organic solvent to a liquid sample. Preferably, said solution comprising an organic solvent comprises methanol and or acetonitrile, more preferably is acetonitrile. Preferably, precipitated sample constituents are removed from the sample, preferably by centrifugation. Preferably, determination is performed on the supernatant of such precipitation. Also preferably, the compound separation step comprises a step yielding a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado. Appropriate techniques are disclosed in, e.g., Nissen 1995, Journal of Chromatography A, 703: 37-57, US 4,540,884 or US 5,397,894. More preferably, said mass spectrometry is liquid chromatography (LC) MS and/or gas chromatography (GC) MS. Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatized prior to gas chromatography. Suitable techniques for derivatization are well known in the art. Preferably, derivatization in accordance with the present invention relates to methoxymation and trimethylsilylation of, preferably, polar compounds and transmethylation, methoxymation and trimethylsilylation of, preferably, non-polar (i.e. lipophilic) compounds.

The sample may also, preferably, be pre-treated by lipid fractionation. Lipid fractionation as used in this context refers to a process as, preferably, described in the accompanying Examples below. In particular, lipid fractionation can be achieved by extracting the total lipids from serum or, preferably, plasma by liquid/liquid extraction using chloroform/methanol. The lipid extracts obtained thereby are subsequently fractionated by normal phase liquid chromatography (NPLC) into eleven different lipid groups according to Christie (Journal of Lipid Research (26), 1985, 507-512). The fractions are, preferably, analyzed by LC-MS/MS using electrospray ionization (ESI) and atmospheric pressure chemical ionization (APCI) with detection of specific multiple reaction monitoring (MRM) transitions for cholesterol esters (CE), free sterols (FS), sphingomyelins (SM), and ceramides (CER), respectively. Sphingosines and sphingosine-1-phosphates (SP) are preferably analyzed by LC-MS/MS using electrospray ionization (ESI) with detection of specific multiple reaction monitoring (MRM) transitions as described by Schmidt H et.al., Prostaglandins & other Lipid Mediators 81(2006), 162-170. The fractions are preferably further analyzed by GC-MS after derivatization with TMSH (Trimethyl sulfonium hydroxide), yielding the fatty acid methyl esters (FAME) corresponding to the acyl moieties of the class-separated lipids. Preferably, lipid fractionation is used for determining ceramides and/or sphingomyelins as biomarkers in accordance with the present invention.

Preferably, the at least one biomarker is determined by a specific chemical or biological assay. Said assay shall comprise means which allow to specifically detect the at least one biomarker in the sample. Preferably, said means are capable of specifically recognizing the chemical structure of the biomarker or are capable of specifically identifying the biomarker based on its capability to react with other compounds or its capability to elicit a response in a biological read out system (e.g., induction of a reporter gene). Means which are capable of specifically recognizing the chemical structure of a biomarker are, preferably, antibodies or other proteins which specifically interact with chemical structures, such as receptors or enzymes, or aptamers. Specific antibodies, for instance, may be obtained using the biomarker as antigen by methods well known in the art. Antibodies as referred to herein include both polyclonal and monoclonal antibodies, as well as fragments thereof, such as Fv, Fab and F(ab)2 fragments that are capable of binding the antigen or hapten. The present invention also includes humanized hybrid antibodies wherein amino acid sequences of a non-human donor antibody exhibiting a desired antigen-specificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Suitable proteins which are capable of specifically recognizing the biomarker are, preferably, enzymes which are involved in the metabolic conversion of the said biomarker. Said enzymes may either use the biomarker as a substrate or may convert a substrate into the biomarker. Moreover, said antibodies may be used as a basis to generate oligopeptides which specifically recognize the biomarker. These oligopeptides shall, for example, comprise the enzyme's binding domains or pockets for the said biomarker. Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, the biomarker may also be determined based on its capability to react with other compounds, i.e. by a specific chemical reaction. Further, the biomarker may be determined in a sample due to its capability to elicit a response in a biological read out system. The biological response shall be detected as read out indicating the presence and/or the amount of the biomarker comprised by the sample. The biological response may be, e.g., the induction of gene expression or a phenotypic response of a cell or an organism. In a preferred embodiment the determination of the least one biomarker is a quantitative process, e.g., allowing also the determination of the amount of the at least one biomarker in the sample. Also preferably, the determining step comprises at least one of the following techniques: nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado.

More preferably, determining of the at least one biomarker comprises mass spectrometry (MS). For mass spectrometry, the analytes in the sample are ionized in order to generate charged molecules or molecule fragments. Afterwards, the mass-to-charge of the ionized analyte, in particular of the ionized biomarkers, or fragments thereof is measured. Thus, the mass spectrometry step preferably comprises an ionization step in which the biomarkers to be determined are ionized. Of course, other compounds present in the sample/eluate are ionized as well. Ionization of the biomarkers can be carried out by any method deemed appropriate, in particular by electron impact ionization, fast atom bombardment, electrospray ionization (ESI), atmospheric pressure chemical ionization (APCI), matrix assisted laser desorption ionization (MALDI). More preferably, the ionization step (for mass spectrometry) is carried out by electrospray ionization (ESI). Accordingly, the mass spectrometry is preferably ESI-MS (or if tandem MS is carried out: ESI-MS/MS). Electrospray is a soft ionization method which results in the formation of ions without breaking any chemical bonds. Electrospray ionization (ESI) is a technique used in mass spectrometry to produce ions using an electrospray in which a high voltage is applied to the sample to create an aerosol. It is especially useful in producing ions from macromolecules because it overcomes the propensity of these molecules to fragment when ionized. Preferably, the electrospray ionization is positive ion mode electrospray ionization. Thus, the ionization is preferably a protonation (or an adduct formation with positive charged ions such as $NH_4^+$, $Na^+$, or $K^+$, in particular $NH_4^+$). According a suitable cation, preferably, a proton ($H^+$) is added to the biomarkers to be determined (and of course to any compound in the sample, i.e. in the eluate from the chromatography column). Therefore, the determination of the amounts of the diagnostic biomarkers might be the determination of the amount of protonated biomarkers.

Mass spectrometry as used herein encompasses all techniques which allow for the determination of the molecular weight (i.e. the mass) or a mass variable corresponding to a compound, i.e. a biomarker, to be determined in accordance with the present invention. Preferably, mass spectrometry is used, in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionization in a first analytical quadrupole of the mass spectrometer, b) fragmentation of the ion selected in step a) by applying an acceleration voltage in an additional subsequent quadrupole which is filled with a collision gas and acts as a collision chamber, c) selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionization process, whereby the quadrupole is filled with collision gas but no acceleration voltage is applied during the analysis. Details on said most preferred mass spectrometry to be used in accordance with the present invention can be found in WO 03/073464.

The term "value of a biomarker", as used herein, relates to a numerical value based on an amount of a biomarker determined in a sample or a reference. Thus, preferably, the value of a biomarker is an amount of a biomarker, e.g. of a metabolite bearing the same designation, or is a ratio of concentrations of at least two biomarkers, e.g. at least two metabolites bearing the respective designations. Thus, as used herein, the term "determining a value of a biomarker" comprises determining the amount of the biomarker and, optionally, calculating a numerical value based on said amount. In accordance, the term "ratio of two shingolipids" relates to a ratio calculated from at least two values of sphingolipids, preferably as specified elsewhere herein.

The term "numerical value based on the amount of a biomarker", as used herein, relates to a numerical value derived from the amount of a biomarker by mathematical procedures; Preferably, said procedures include normalization, weighting and/or comparing an amount to a reference. Preferably, the measured amounts are scaled by first subtracting a predetermined, biomarker-specific subtrahend from said amount and then dividing the resulting value by a predetermined biomarker-specific divisor. More preferably, numerical value based on the amount of a biomarker is calculated by scaling the $log_{10}$-transformed input data x by first subtracting an analyte-specific constant $m_i$ and then dividing by a analyte-specific constant $s_i$, resulting in $log_{10}$-transformed and scaled input data $\hat{x}$ as in formula (I):

$$\hat{x}_i = \frac{x_i - m_i}{s_i} \quad (I).$$

More preferably, the method further comprises a further step of calculating a score from the values of the biomarkers, e.g. a prediction score. Methods for determining a score are known in the art and include, e.g. calculating a sum, a difference, a product, and/or a ratio over the values of biomarker or any other standard mathematical operations. Preferably, calculating a score comprises calculating a measure for the probability that a subject is afflicted with pancreatic cancer, more preferably a prediction score is calculated by weighting the individual biomarkers as specified above and summing up the weighted values for all biomarkers determined, preferably, followed by assigning a bias value to said sum and, preferably, scaling the bias-corrected sum, preferably to a value between 0 and 1. Preferably, scaling of measured values comprises $\log_{10}$ transforming the measured values, preferably after subtracting said predetermined, diagnostic biomarker-specific subtrahend. More preferably, the prediction score is calculated according to the following formula (II)

$$p = \frac{1}{1 + e^{-(\omega_0 + \sum_i^n \omega_i \hat{x}_i)}} \quad \text{(II)},$$

wherein

  p = prediction score;
  e = Euler's number;
  $\omega_0$ = bias value;
  $\omega_i$ = diagnostic biomarker-specific weight value for diagnostic biomarker i; and
  $\hat{x}_i$ = scaled amount for diagnostic biomarker i, calculated as specified above, preferably according to formula (I).

As will be appreciated, the score may be a direct predictor of the probability of disease, in particular in case calculating the value of the biomarker or of the score already comprises comparing the biomarkers to references. Thus, the score may directly indicate the probability of disease, e.g. as a percent probability.

More preferably, the score is an indirect indicator of disease requiring comparison to a reference score. Thus, preferably, the score may indicate the probability of disease in comparison to a reference. Preferably, optimized values of the predetermined, diagnostic biomarker-specific subtrahend, the predetermined, diagnostic biomarker-specific divisor, the diagnostic biomarker-specific weight value, and the bias value are determined by optimizing the differentiation between subjects suffering from a specific disease and subjects not suffering from said disease in such case. Thus preferably, the group of diagnostic biomarkers is, preferably, trained on data obtained from two groups of subjects with known disease state. E.g., preferably, one of said groups of subjects is a group of subjects suffering from pancreatitis, and the second group is a group of subjects suffering from pancreatic cancer; this way, optimized parameters for a differentiation between pancreatitis and pancreatic cancer are obtained.

Thus, preferably, the method of diagnosing pancreatic cancer comprises the steps of

  (a) semiquantitatively or, preferably, quantitatively determining the amounts of a group of diagnostic biomarkers according to the present invention in at least one sample of a subject,
  (b1) for each amount determined in step (a), calculating a scaled amount by first subtracting a predetermined, diagnostic biomarker-specific subtrahend from said amount and then dividing the resulting value by a predetermined, diagnostic biomarker-specific divisor,
  (b2) calculating a prediction score by

    (i) assigning a diagnostic biomarker-specific weight value to each scaled amount of (b1), thereby providing a weighed amount,
    (ii) summing up said weighed amounts for all diagnostic biomarkers, providing a sum of weighted amounts,
    (iii) preferably, assigning a bias value to the sum of weighted amounts of step (ii) to provide a bias-corrected sum,
    (iv) preferably, scaling the bias-corrected sum of step (iii), preferably to a value between 0 and 1, and

  (b3) determining the probability for a subject to suffer from pancreatic cancer based on the prediction score determined in step (b2).

According to the method disclosed herein, determining comprises determining the value of at least one biomarker each of the categories carnitines, amino acids, mitochondrial energy metabolites, and sphingolipids.

The term "carnitine" is known to the skilled person. As used herein, the term relates to carnitine (3-Hydroxy-4-(trimethylazaniumyl)butanoate, CAS-No: 541-15-1) and its 3-hydroxy fatty acid esters, preferably its C2 to C10 fatty acid esters. More preferably, the carnitine is selected from the list consisting of Propionyl-carnitine, Butyryl-carnitine, and Octenoyl-carnitine.

The term "amino acid" is known to the skilled person as well. Preferably, the term relates to alpha-amino acids, more preferably I-alpha amino acids, most preferably to proteinogenic amino acids. Preferably the amino acid is selected from the list consisting of Histidine and Proline.

The term "mitochondrial energy metabolite biomarker", as used herein, relates to compounds indicating increased flux through the energy generating system of mitochondria. Thus, preferably, the term includes compounds of the citric acid cycle and cofactors of the respiratory chain, more preferably citrate, cis-aconitate, isocitrate, 2-Oxoglutarate, succinate, fumarate, malate, oxalacetate, coenzyme Q10, and Coenzyme Q9. More preferably, the mitochondrial energy metabolite biomarker is selected from 2-Oxoglutarate or Coenzyme Q9.

The term "sphingolipid" is known to the skilled person to relate to a class of lipids having a sphingoid base backbone and a fatty acid side chain. According to the method disclosed herein, a first and a second sphingolipid are determined, wherein the fatty acid residue of the first sphingolipid is at least four, preferably at least five, more preferably at least six, most preferably at least seven carbon atoms longer than the fatty acid residue of the second sphingolipid. The expression "at least X carbon atoms longer" is understood by the skilled person to mean that the first sphingolipid has at least X ($CH_2$) or (CH) units more than the second sphingolipid. Thus, preferably, the method comprises determining a value of a sphingolipid comprising a long-chain fatty (C14 to C22) acid and/or a sphingolipid comprising a very-long-chain fatty acid (>C22). Moreover, according to the method disclosed herein, the sphingosine backbone of the first sphingolipid and the second sphingolipid are identical; accordingly, the first sphingolipid and the second sphingolipid are from the same subclass, preferably both are ceramides or both are sphingomyelins. Preferably, the fatty acid side chain of the sphingolipid is a saturated fatty acid side chain. Preferably, a ratio is calculated from the first and the second sphingolipid biomarker, preferably wherein a ratio of at least one sphingolipid comprising a long-chain fatty acid to at least one sphingolipid comprising a very-long-chain fatty acid. More preferably, the sphingolipid biomarker is selected from the list consisting of Ceramide (Cer) d18:1,C16:0) to Cer (18:1,C24:0) ratio; Cer (d18:1,C18:0) to Cer (d18:1,C24:0) ratio; (Cer (d18:1,C16:0) plus Cer d18:1,C18:0)) to Cer (d18:1,C24:0) ratio; Sphingomyelin (SM) (d16:1,C18:0) to SM (d16:1,C24:0) ratio; SM (d17:1,C18:0) to SM (d17:1,C24:0) ratio; and SM (d18:2,C18:0) to SM (d18:2,C24:0) ratio.

Preferably, the amount of CA19-9 is additionally determined according to the method disclosed. The term CA19-9 is known to the skilled person and diagnostic tests are commercially available. Preferably, the amount of CA19-9 is determined at essentially the same time as the small molecule biomarker disclosed herein are determined, i.e. preferably, is determined from the same sample as the small molecule biomarkers. More preferably, the sample used for determining CA19-9 and the sample or samples used for determining the remaining biomarkers are obtained within a time frame of at most one year, more preferably at most three months, even more preferably at most two months, most preferably, at most one month. Thus, preferably, the term "determining the amount of CA19-9" includes providing a concentration value for CA19-9 determined earlier for said subject, e.g., preferably, for deciding whether said subject is suspected to suffer from pancreatic cancer. Preferably, at least two, at least three, at least four, at least five, at least six, at least seven, at least eight, at least nine, or at least ten biomarkers are determined in a common assay from the same sample, i.e., an assay providing measured values for said number of biomarkers as an output. The skilled person is aware that some biomarkers are preferably determined in specific assays, e.g., a biomarker having a complex structure, in particular CA19-9 is, preferably, determined in an immunological assay, e.g. preferably, a radioimmunoassay (RIA).

In an embodiment, the biomarkers are determined from the same sample, wherein said sample is, preferably split into at least two subsamples, of which in one subsample the small molecule biomarkers are determined and of which in a second subsample CA19-9 is determined. Preferably, small molecule biomarkers are determined in a first sample and CA19-9 is determined in a second sample, wherein, preferably, said samples are taken at the same time, or, preferably, at different times as specified above. In a further embodiment, in the method of diagnosing pancreatic cancer, CA19-9 is not determined; in such case, preferably, the subject is a subject known or suspected to be a subject with a low CA19-9 value as specified elsewhere herein, more preferably a subject which is Lewis a/b antigen negative, as specified elsewhere herein. Thus, preferably, in a subject with a very low CA19-9 value, preferably less than 2 U/mL, and therefore most probably being a Lewis a/b antigen negative subject, the biomarkers from Table 1 are applied without CA19-9.

In view of the above, the method for diagnosing pancreatic cancer in a subject of the present invention includes, preferably, a method comprising the steps of

(a1) determining in a sample of said subject the value of at least one biomarker each of the categories carnitines, amino acids, mitochondrial energy metabolites, and sphingolipids;
(a2) providing a value of an amount of the diagnostic biomarker CA19-9 in a sample of said subject; and
(b) comparing said amounts of said diagnostic biomarkers of (a1) and (a2) with a reference or references, whereby pancreatic cancer is diagnosed.

Preferably, the method comprises determining still further biomarkers as well. In such case, preferably, each further biomarker determined decreases the false-positive rate and/or the false negative rate of the method by at least 0.1 %, preferably 1 %. More preferably, each further biomarker determined significantly increases the AUC value of the method. Accordingly, the method, preferably, avoids determining biomarkers not contributing to improvement of diagnosis. It is, however, also envisaged that further biomarkers are determined which are diagnostic markers of other diseases, e.g. as a part of a global health screening method.

Preferred groups (panels) of biomarkers which comprise the aforementioned biomarkers are shown in Tables 1. These groups of biomarkers may be preferably determined as biomarker groups in accordance with the present invention.

Table 1: Preferred biomarker combinations (panels)

| Panel designation | Biomarkers | AUC pancreatic cancer relative to diabetes | AUC resectable pancreatic cancer relative to diabetes |
|---|---|---|---|
| ACELS_29 | Propionylcarnitine, alpha-Ketoglutarate, Histidine, (Cer d18:1,C18:0) ratio to (Cer d18:1,C24:0), Lysophosphatidylethanolamine (C18:2), | 0.936 | 0.945 |
| ACELS_3 | Octenoylcarnitine, Coenzyme Q9, Proline, (Cer d18:1,C16:0) ratio to (Cer d18:1,C24:0), Lysophosphatidylethanolamine (C18:2), | 0.900 | 0.921 |
| ACELS_30 | Butyrylcarnitine, alpha-Ketoglutarate, Histidine, (Cer d18:1,C18:0) ratio to (Cer d18:1,C24:0), Lysophosphatidylethanolamine (C18:2), | 0.920 | 0.925 |
| ACELS_31 | Octenoylcarnitine, alpha-Ketoglutarate, Histidine, (Cer d18:1,C18:0) ratio to (Cer d18:1,C24:0), Lysophosphatidylethanolamine (C18:2), | 0.907 | 0.910 |
| ACELS_33 | Propionylcarnitine, Coenzyme Q9, Proline, (Cer d18:1,C16:0 plus Cer d18:1,C18:0) ratio to (Cer d18:1,C24:0), Lysophosphatidylethanolamine (C18:2), | 0.926 | 0.936 |
| ACELS_34 | Butyrylcarnitine, Coenzyme Q9, Proline, (Cer d18:1,C16:0 plus Cer d18:1,C18:0) ratio to (Cer d18:1,C24:0), Lysophosphatidylethanolamine (C18:2), | 0.910 | 0.923 |
| ACELS_35 | Octenoylcarnitine, Coenzyme Q9, Proline, (Cer d18:1,C16:0 plus Cer d18:1,C18:0) ratio to (Cer d18:1,C24:0), Lysophosphatidylethanolamine (C18:2), | 0.902 | 0.920 |
| ACELS_37 | Propionylcarnitine, Coenzyme Q9, Histidine, (Cer d18:1,C16:0 plus Cer d18:1,C18:0) ratio to (Cer d18:1,C24:0), Lysophosphatidylethanolamine (C18:2), | 0.930 | 0.937 |
| ACELS_38 | Butyrylcarnitine, Coenzyme Q9, Histidine, (Cer d18:1,C16:0 plus Cer d18:1,C18:0) ratio to (Cer d18:1,C24:0), Lysophosphatidylethanolamine (C18:2), | 0.910 | 0.918 |
| ACELS_39 | Octenoylcarnitine, Coenzyme Q9, Histidine, (Cer d18:1,C16:0 plus Cer d18:1,C18:0) ratio to (Cer d18:1,C24:0), Lysophosphatidylethanolamine (C18:2), | 0.897 | 0.908 |
| ACELS_41 | Propionylcarnitine, alpha-Ketoglutarate, Proline, (Cer d18:1,C16:0 plus Cer d18:1,C18:0) ratio to (Cer d18:1,C24:0), Lysophosphatidylethanolamine (C18:2), | 0.933 | 0.945 |

| ACELS_42 | Butyrylcarnitine, alpha-Ketoglutarate, Proline, (Cer d18:1,C16:0 plus Cer d18:1,C18:0) ratio to (Cer d18:1,C24:0), Lysophosphatidylethanolamine (C18:2), | 0.917 | 0.931 |
|---|---|---|---|
| ACELS_43 | Octenoylcarnitine, alpha-Ketoglutarate, Proline, (Cer d18:1,C16:0 plus Cer d18:1,C18:0) ratio to (Cer d18:1,C24:0), Lysophosphatidylethanolamine (C18:2), | 0.908 | 0.924 |
| ACELS_45 | Propionylcarnitine, alpha-Ketoglutarate, Histidine, (Cer d18:1,C16:0 plus Cer d18:1,C18:0) ratio to (Cer d18:1,C24:0), Lysophosphatidylethanolamine (C18:2), | 0.936 | 0.946 |
| ACELS_46 | Butyrylcarnitine, alpha-Ketoglutarate, Histidine, (Cer d18:1,C16:0 plus Cer d18:1,C18:0) ratio to (Cer d18:1,C24:0), Lysophosphatidylethanolamine (C18:2), | 0.921 | 0.927 |
| ACELS_47 | Octenoylcarnitine, alpha-Ketoglutarate, Histidine, (Cer d18:1,C16:0 plus Cer d18:1,C18:0) ratio to (Cer d18:1,C24:0), Lysophosphatidylethanolamine (C18:2), | 0.907 | 0.913 |
| ACELS_49 | Propionylcarnitine, Coenzyme Q9, Proline, (SM d17:1,C18:0) ratio to (SM d17:1,C24:0), Lysophosphatidylethanolamine (C18:2), | 0.916 | 0.930 |
| ACELS_5 | Propionylcarnitine, Coenzyme Q9, Histidine, (Cer d18:1,C16:0) ratio to (Cer d18:1,C24:0), Lysophosphatidylethanolamine (C18:2), | 0.926 | 0.934 |
| ACELS_50 | Butyrylcarnitine, Coenzyme Q9, Proline, (SM d17:1,C18:0) ratio to (SM d17:1,C24:0), Lysophosphatidylethanolamine (C18:2), | 0.897 | 0.912 |
| ACELS_51 | Octenoylcarnitine, Coenzyme Q9, Proline, (SM d17:1,C18:0) ratio to (SM d17:1,C24:0), Lysophosphatidylethanolamine (C18:2), | 0.893 | 0.911 |
| ACELS_53 | Propionylcarnitine, Coenzyme Q9, Histidine, (SM d17:1,C18:0) ratio to (SM d17:1,C24:0), Lysophosphatidylethanolamine (C18:2), | 0.920 | 0.932 |
| ACELS_54 | Butyrylcarnitine, Coenzyme Q9, Histidine, (SM d17:1,C18:0) ratio to (SM d17:1,C24:0), Lysophosphatidylethanolamine (C18:2), | 0.899 | 0.910 |
| ACELS_55 | Octenoylcarnitine, Coenzyme Q9, Histidine, (SM d17:1,C18:0) ratio to (SM d17:1,C24:0), Lysophosphatidylethanolamine (C18:2), | 0.892 | 0.905 |
| ACELS_57 | Propionylcarnitine, alpha-Ketoglutarate, Proline, (SM d17:1,C18:0) ratio to (SM d17:1,C24:0), Lysophosphatidylethanolamine (C18:2), | 0.923 | 0.937 |
| ACELS_58 | Butyrylcarnitine, alpha-Ketoglutarate, Proline, (SM d17:1,C18:0) ratio to (SM d17:1,C24:0), Lysophosphatidylethanolamine (C18:2), | 0.902 | 0.916 |
| ACELS_59 | Octenoylcarnitine, alpha-Ketoglutarate, Proline, (SM | 0.896 | 0.914 |

| | | | | |
|---|---|---|---|---|
| | d17:1,C18:0) ratio to (SM d17:1,C24:0), Lysophosphatidylethanolamine (C18:2), | | | |
| ACELS_6 | Butyrylcarnitine, Coenzyme Q9, Histidine, (Cer d18:1,C16:0) ratio to (Cer d18:1,C24:0), Lysophosphatidylethanolamine (C18:2), | 0.901 | 0.914 | |
| ACELS_61 | Propionylcarnitine, alpha-Ketoglutarate, Histidine, (SM d17:1,C18:0) ratio to (SM d17:1,C24:0), Lysophosphatidylethanolamine (C18:2), | 0.927 | 0.940 | |
| ACELS_62 | Butyrylcarnitine, alpha-Ketoglutarate, Histidine, (SM d17:1,C18:0) ratio to (SM d17:1,C24:0), Lysophosphatidylethanolamine (C18:2), | 0.905 | 0.914 | |
| ACELS_63 | Octenoylcarnitine, alpha-Ketoglutarate, Histidine, (SM d17:1,C18:0) ratio to (SM d17:1,C24:0), Lysophosphatidylethanolamine (C18:2), | 0.898 | 0.909 | |
| ACELS_65 | Propionylcarnitine, Coenzyme Q9, Proline, (SM d18:2,C18:0) ratio to (SM d18:2,C24:0), Lysophosphatidylethanolamine (C18:2), | 0.905 | 0.925 | |
| ACELS_66 | Butyrylcarnitine, Coenzyme Q9, Proline, (SM d18:2,C18:0) ratio to (SM d18:2,C24:0), Lysophosphatidylethanolamine (C18:2), | 0.883 | 0.909 | |
| ACELS_67 | Octenoylcarnitine, Coenzyme Q9, Proline, (SM d18:2,C18:0) ratio to (SM d18:2,C24:0), Lysophosphatidylethanolamine (C18:2), | 0.882 | 0.905 | |
| ACELS_69 | Propionylcarnitine, Coenzyme Q9, Histidine, (SM d18:2,C18:0) ratio to (SM d18:2,C24:0), Lysophosphatidylethanolamine (C18:2), | 0.912 | 0.928 | |
| ACELS_7 | Octenoylcarnitine, Coenzyme Q9, Histidine, (Cer d18:1,C16:0) ratio to (Cer d18:1,C24:0), Lysophosphatidylethanolamine (C18:2), | 0.893 | 0.905 | |
| ACELS_70 | Butyrylcarnitine, Coenzyme Q9, Histidine, (SM d18:2,C18:0) ratio to (SM d18:2,C24:0), Lysophosphatidylethanolamine (C18:2), | 0.883 | 0.908 | |
| ACELS_71 | Octenoylcarnitine, Coenzyme Q9, Histidine, (SM d18:2,C18:0) ratio to (SM d18:2,C24:0), Lysophosphatidylethanolamine (C18:2), | 0.882 | 0.900 | |
| ACELS_73 | Propionylcarnitine, alpha-Ketoglutarate, Proline, (SM d18:2,C18:0) ratio to (SM d18:2,C24:0), Lysophosphatidylethanolamine (C18:2), | 0.915 | 0.935 | |
| ACELS_74 | Butyrylcarnitine, alpha-Ketoglutarate, Proline, (SM d18:2,C18:0) ratio to (SM d18:2,C24:0), Lysophosphatidylethanolamine (C18:2), | 0.888 | 0.912 | |
| ACELS_75 | Octenoylcarnitine, alpha-Ketoglutarate, Proline, (SM d18:2,C18:0) ratio to (SM d18:2,C24:0), | 0.886 | 0.908 | |

| | | | | |
|---|---|---|---|---|
| | | Lysophosphatidylethanolamine (C18:2), | | |
| | ACELS_77 | Propionylcarnitine, alpha-Ketoglutarate, Histidine, (SM d18:2,C18:0) ratio to (SM d18:2,C24:0), Lysophosphatidylethanolamine (C18:2), | 0.919 | 0.938 |
| | ACELS_78 | Butyrylcarnitine, alpha-Ketoglutarate, Histidine, (SM d18:2,C18:0) ratio to (SM d18:2,C24:0), Lysophosphatidylethanolamine (C18:2), | 0.890 | 0.912 |
| | ACELS_79 | Octenoylcarnitine, alpha-Ketoglutarate, Histidine, (SM d18:2,C18:0) ratio to (SM d18:2,C24:0), Lysophosphatidylethanolamine (C18:2), | 0.889 | 0.904 |
| | ACELS_81 | Propionylcarnitine, Coenzyme Q9, Proline, (SM d16:1,C18:0) ratio to (SM d16:1,C24:0), Lysophosphatidylethanolamine (C18:2), | 0.912 | 0.928 |
| | ACELS_82 | Butyrylcarnitine, Coenzyme Q9, Proline, (SM d16:1,C18:0) ratio to (SM d16:1,C24:0), Lysophosphatidylethanolamine (C18:2), | 0.889 | 0.910 |
| | ACELS_83 | Octenoylcarnitine, Coenzyme Q9, Proline, (SM d16:1,C18:0) ratio to (SM d16:1,C24:0), Lysophosphatidylethanolamine (C18:2), | 0.883 | 0.905 |
| | ACELS_85 | Propionylcarnitine, Coenzyme Q9, Histidine, (SM d16:1,C18:0) ratio to (SM d16:1,C24:0), Lysophosphatidylethanolamine (C18:2), | 0.917 | 0.930 |
| | ACELS_86 | Butyrylcarnitine, Coenzyme Q9, Histidine, (SM d16:1,C18:0) ratio to (SM d16:1,C24:0), Lysophosphatidylethanolamine (C18:2), | 0.893 | 0.907 |
| | ACELS_87 | Octenoylcarnitine, Coenzyme Q9, Histidine, (SM d16:1,C18:0) ratio to (SM d16:1,C24:0), Lysophosphatidylethanolamine (C18:2), | 0.885 | 0.903 |
| | ACELS_89 | Propionylcarnitine, alpha-Ketoglutarate, Proline, (SM d16:1,C18:0) ratio to (SM d16:1,C24:0), Lysophosphatidylethanolamine (C18:2), | 0.919 | 0.936 |
| | ACELS_9 | Propionylcarnitine, alpha-Ketoglutarate, Proline, (Cer d18:1,C16:0) ratio to (Cer d18:1,C24:0), Lysophosphatidylethanolamine (C18:2), | 0.928 | 0.945 |
| | ACELS_90 | Butyrylcarnitine, alpha-Ketoglutarate, Proline, (SM d16:1,C18:0) ratio to (SM d16:1,C24:0), Lysophosphatidylethanolamine (C18:2), | 0.897 | 0.915 |
| | ACELS_91 | Octenoylcarnitine, alpha-Ketoglutarate, Proline, (SM d16:1,C18:0) ratio to (SM d16:1,C24:0), Lysophosphatidylethanolamine (C18:2), | 0.888 | 0.909 |
| | ACELS_93 | Propionylcarnitine, alpha-Ketoglutarate, Histidine, (SM d16:1,C18:0) ratio to (SM d16:1,C24:0), Lysophosphatidylethanolamine (C18:2), | 0.924 | 0.938 |

| ACELS_94 | Butyrylcarnitine, alpha-Ketoglutarate, Histidine, (SM d16:1,C18:0) ratio to (SM d16:1,C24:0), Lysophosphatidylethanolamine (C18:2), | 0.902 | 0.915 |
|---|---|---|---|
| ACELS_95 | Octenoylcarnitine, alpha-Ketoglutarate, Histidine, (SM d16:1,C18:0) ratio to (SM d16:1,C24:0), Lysophosphatidylethanolamine (C18:2), | 0.894 | 0.903 |
| ACELS_97 | Propionylcarnitine, Coenzyme Q9, Proline, (Cer d18:1,C16:0) ratio to (Cer d18:1,C24:0), Lysophosphatidylethanolamine (C18:2), | 0.924 | 0.934 |
| ACELS_98 | Butyrylcarnitine, Coenzyme Q9, Proline, (Cer d18:1,C16:0) ratio to (Cer d18:1,C24:0), Lysophosphatidylethanolamine (C18:2), | 0.904 | 0.920 |
| ACELS_99 | Octenoylcarnitine, Coenzyme Q9, Proline, (Cer d18:1,C16:0) ratio to (Cer d18:1,C24:0), Lysophosphatidylethanolamine (C18:2), | 0.900 | 0.921 |
| ACES_1 | Propionylcarnitine, Coenzyme Q9, Proline, (Cer d18:1,C16:0) ratio to (Cer d18:1,C24:0), | 0.919 | 0.931 |
| ACES_10 | Butyrylcarnitine, alpha-Ketoglutarate, Proline, (Cer d18:1,C16:0) ratio to (Cer d18:1,C24:0), | 0.917 | 0.935 |
| ACES_11 | Octenoylcarnitine, alpha-Ketoglutarate, Proline, (Cer d18:1,C16:0) ratio to (Cer d18:1,C24:0), | 0.919 | 0.928 |
| ACES_13 | Propionylcarnitine, alpha-Ketoglutarate, Histidine, (Cer d18:1,C16:0) ratio to (Cer d18:1,C24:0), | 0.933 | 0.945 |
| ACES_14 | Butyrylcarnitine, alpha-Ketoglutarate, Histidine, (Cer d18:1,C16:0) ratio to (Cer d18:1,C24:0), | 0.917 | 0.928 |
| ACES_15 | Octenoylcarnitine, alpha-Ketoglutarate, Histidine, (Cer d18:1,C16:0) ratio to (Cer d18:1,C24:0), | 0.911 | 0.918 |
| ACES_17 | Propionylcarnitine, Coenzyme Q9, Proline, (Cer d18:1,C18:0) ratio to (Cer d18:1,C24:0), | 0.922 | 0.931 |
| ACES_18 | Butyrylcarnitine, Coenzyme Q9, Proline, (Cer d18:1,C18:0) ratio to (Cer d18:1,C24:0), | 0.908 | 0.917 |
| ACES_19 | Octenoylcarnitine, Coenzyme Q9, Proline, (Cer d18:1,C18:0) ratio to (Cer d18:1,C24:0), | 0.910 | 0.920 |
| ACES_2 | Butyrylcarnitine, Coenzyme Q9, Proline, (Cer d18:1,C16:0) ratio to (Cer d18:1,C24:0), | 0.909 | 0.918 |
| ACES_21 | Propionylcarnitine, Coenzyme Q9, Histidine, (Cer d18:1,C18:0) ratio to (Cer d18:1,C24:0), | 0.930 | 0.934 |
| ACES_22 | Butyrylcarnitine, Coenzyme Q9, Histidine, (Cer d18:1,C18:0) ratio to (Cer d18:1,C24:0), | 0.919 | 0.918 |
| ACES_23 | Octenoylcarnitine, Coenzyme Q9, Histidine, (Cer d18:1,C18:0) ratio to (Cer d18:1,C24:0), | 0.917 | 0.915 |
| ACES_25 | Propionylcarnitine, alpha-Ketoglutarate, Proline, (Cer d18:1,C18:0) ratio to (Cer d18:1,C24:0), | 0.929 | 0.942 |

| ACES_26 | Butyrylcarnitine, alpha-Ketoglutarate, Proline, (Cer d18:1,C18:0) ratio to (Cer d18:1,C24:0), | 0.915 | 0.928 |
|---|---|---|---|
| ACES_27 | Octenoylcarnitine, alpha-Ketoglutarate, Proline, (Cer d18:1,C18:0) ratio to (Cer d18:1,C24:0), | 0.913 | 0.920 |
| ACES_29 | Propionylcarnitine, alpha-Ketoglutarate, Histidine, (Cer d18:1,C18:0) ratio to (Cer d18:1,C24:0), | 0.939 | 0.944 |
| ACES_3 | Octenoylcarnitine, Coenzyme Q9, Proline, (Cer d18:1,C16:0) ratio to (Cer d18:1,C24:0), | 0.914 | 0.926 |
| ACES_30 | Butyrylcarnitine, alpha-Ketoglutarate, Histidine, (Cer d18:1,C18:0) ratio to (Cer d18:1,C24:0), | 0.920 | 0.924 |
| ACES_31 | Octenoylcarnitine, alpha-Ketoglutarate, Histidine, (Cer d18:1,C18:0) ratio to (Cer d18:1,C24:0), | 0.910 | 0.912 |
| ACES_33 | Propionylcarnitine, Coenzyme Q9, Proline, (Cer d18:1,C16:0 plus Cer d18:1,C18:0) ratio to (Cer d18:1,C24:0), | 0.922 | 0.932 |
| ACES_34 | Butyrylcarnitine, Coenzyme Q9, Proline, (Cer d18:1,C16:0 plus Cer d18:1,C18:0) ratio to (Cer d18:1,C24:0), | 0.911 | 0.919 |
| ACES_35 | Octenoylcarnitine, Coenzyme Q9, Proline, (Cer d18:1,C16:0 plus Cer d18:1,C18:0) ratio to (Cer d18:1,C24:0), | 0.914 | 0.923 |
| ACES_37 | Propionylcarnitine, Coenzyme Q9, Histidine, (Cer d18:1,C16:0 plus Cer d18:1,C18:0) ratio to (Cer d18:1,C24:0), | 0.930 | 0.934 |
| ACES_38 | Butyrylcarnitine, Coenzyme Q9, Histidine, (Cer d18:1,C16:0 plus Cer d18:1,C18:0) ratio to (Cer d18:1,C24:0), | 0.919 | 0.918 |
| ACES_39 | Octenoylcarnitine, Coenzyme Q9, Histidine, (Cer d18:1,C16:0 plus Cer d18:1,C18:0) ratio to (Cer d18:1,C24:0), | 0.918 | 0.916 |
| ACES_41 | Propionylcarnitine, alpha-Ketoglutarate, Proline, (Cer d18:1,C16:0 plus Cer d18:1,C18:0) ratio to (Cer d18:1,C24:0), | 0.931 | 0.944 |
| ACES_42 | Butyrylcarnitine, alpha-Ketoglutarate, Proline, (Cer d18:1,C16:0 plus Cer d18:1,C18:0) ratio to (Cer d18:1,C24:0), | 0.920 | 0.931 |
| ACES_43 | Octenoylcarnitine, alpha-Ketoglutarate, Proline, (Cer d18:1,C16:0 plus Cer d18:1,C18:0) ratio to (Cer d18:1,C24:0), | 0.918 | 0.923 |
| ACES_45 | Propionylcarnitine, alpha-Ketoglutarate, Histidine, (Cer d18:1,C16:0 plus Cer d18:1,C18:0) ratio to (Cer d18:1,C24:0), | 0.938 | 0.946 |
| ACES_46 | Butyrylcarnitine, alpha-Ketoglutarate, Histidine, (Cer d18:1,C16:0 plus Cer d18:1,C18:0) ratio to (Cer | 0.923 | 0.928 |

| | | | |
|---|---|---|---|
| | d18:1,C24:0), | | |
| ACES_47 | Octenoylcarnitine, alpha-Ketoglutarate, Histidine, (Cer d18:1,C16:0 plus Cer d18:1,C18:0) ratio to (Cer d18:1,C24:0), | 0.912 | 0.915 |
| ACES_49 | Propionylcarnitine, Coenzyme Q9, Proline, (SM d17:1,C18:0) ratio to (SM d17:1,C24:0), | 0.917 | 0.927 |
| ACES_5 | Propionylcarnitine, Coenzyme Q9, Histidine, (Cer d18:1,C16:0) ratio to (Cer d18:1,C24:0), | 0.925 | 0.930 |
| ACES_50 | Butyrylcarnitine, Coenzyme Q9, Proline, (SM d17:1,C18:0) ratio to (SM d17:1,C24:0), | 0.902 | 0.913 |
| ACES_51 | Octenoylcarnitine, Coenzyme Q9, Proline, (SM d17:1,C18:0) ratio to (SM d17:1,C24:0), | 0.904 | 0.916 |
| ACES_53 | Propionylcarnitine, Coenzyme Q9, Histidine, (SM d17:1,C18:0) ratio to (SM d17:1,C24:0), | 0.923 | 0.928 |
| ACES_54 | Butyrylcarnitine, Coenzyme Q9, Histidine, (SM d17:1,C18:0) ratio to (SM d17:1,C24:0), | 0.911 | 0.913 |
| ACES_55 | Octenoylcarnitine, Coenzyme Q9, Histidine, (SM d17:1,C18:0) ratio to (SM d17:1,C24:0), | 0.908 | 0.914 |
| ACES_57 | Propionylcarnitine, alpha-Ketoglutarate, Proline, (SM d17:1,C18:0) ratio to (SM d17:1,C24:0), | 0.922 | 0.939 |
| ACES_58 | Butyrylcarnitine, alpha-Ketoglutarate, Proline, (SM d17:1,C18:0) ratio to (SM d17:1,C24:0), | 0.904 | 0.924 |
| ACES_59 | Octenoylcarnitine, alpha-Ketoglutarate, Proline, (SM d17:1,C18:0) ratio to (SM d17:1,C24:0), | 0.901 | 0.917 |
| ACES_6 | Butyrylcarnitine, Coenzyme Q9, Histidine, (Cer d18:1,C16:0) ratio to (Cer d18:1,C24:0), | 0.914 | 0.914 |
| ACES_61 | Propionylcarnitine, alpha-Ketoglutarate, Histidine, (SM d17:1,C18:0) ratio to (SM d17:1,C24:0), | 0.930 | 0.942 |
| ACES_62 | Butyrylcarnitine, alpha-Ketoglutarate, Histidine, (SM d17:1,C18:0) ratio to (SM d17:1,C24:0), | 0.910 | 0.922 |
| ACES_63 | Octenoylcarnitine, alpha-Ketoglutarate, Histidine, (SM d17:1,C18:0) ratio to (SM d17:1,C24:0), | 0.898 | 0.910 |
| ACES_65 | Propionylcarnitine, Coenzyme Q9, Proline, (SM d18:2,C18:0) ratio to (SM d18:2,C24:0), | 0.912 | 0.922 |
| ACES_66 | Butyrylcarnitine, Coenzyme Q9, Proline, (SM d18:2,C18:0) ratio to (SM d18:2,C24:0), | 0.891 | 0.902 |
| ACES_67 | Octenoylcarnitine, Coenzyme Q9, Proline, (SM d18:2,C18:0) ratio to (SM d18:2,C24:0), | 0.896 | 0.910 |
| ACES_69 | Propionylcarnitine, Coenzyme Q9, Histidine, (SM d18:2,C18:0) ratio to (SM d18:2,C24:0), | 0.919 | 0.925 |
| ACES_7 | Octenoylcarnitine, Coenzyme Q9, Histidine, (Cer d18:1,C16:0) ratio to (Cer d18:1,C24:0), | 0.916 | 0.918 |

| ACES_70 | Butyrylcarnitine, Coenzyme Q9, Histidine, (SM d18:2,C18:0) ratio to (SM d18:2,C24:0), | 0.898 | 0.904 |
|---|---|---|---|
| ACES_71 | Octenoylcarnitine, Coenzyme Q9, Histidine, (SM d18:2,C18:0) ratio to (SM d18:2,C24:0), | 0.898 | 0.907 |
| ACES_73 | Propionylcarnitine, alpha-Ketoglutarate, Proline, (SM d18:2,C18:0) ratio to (SM d18:2,C24:0), | 0.914 | 0.937 |
| ACES_74 | Butyrylcarnitine, alpha-Ketoglutarate, Proline, (SM d18:2,C18:0) ratio to (SM d18:2,C24:0), | 0.892 | 0.914 |
| ACES_75 | Octenoylcarnitine, alpha-Ketoglutarate, Proline, (SM d18:2,C18:0) ratio to (SM d18:2,C24:0), | 0.895 | 0.907 |
| ACES_77 | Propionylcarnitine, alpha-Ketoglutarate, Histidine, (SM d18:2,C18:0) ratio to (SM d18:2,C24:0), | 0.923 | 0.939 |
| ACES_78 | Butyrylcarnitine, alpha-Ketoglutarate, Histidine, (SM d18:2,C18:0) ratio to (SM d18:2,C24:0), | 0.900 | 0.914 |
| ACES_79 | Octenoylcarnitine, alpha-Ketoglutarate, Histidine, (SM d18:2,C18:0) ratio to (SM d18:2,C24:0), | 0.890 | 0.902 |
| ACES_81 | Propionylcarnitine, Coenzyme Q9, Proline, (SM d16:1,C18:0) ratio to (SM d16:1,C24:0), | 0.913 | 0.925 |
| ACES_82 | Butyrylcarnitine, Coenzyme Q9, Proline, (SM d16:1,C18:0) ratio to (SM d16:1,C24:0), | 0.894 | 0.906 |
| ACES_83 | Octenoylcarnitine, Coenzyme Q9, Proline, (SM d16:1,C18:0) ratio to (SM d16:1,C24:0), | 0.901 | 0.914 |
| ACES_85 | Propionylcarnitine, Coenzyme Q9, Histidine, (SM d16:1,C18:0) ratio to (SM d16:1,C24:0), | 0.918 | 0.928 |
| ACES_86 | Butyrylcarnitine, Coenzyme Q9, Histidine, (SM d16:1,C18:0) ratio to (SM d16:1,C24:0), | 0.901 | 0.909 |
| ACES_87 | Octenoylcarnitine, Coenzyme Q9, Histidine, (SM d16:1,C18:0) ratio to (SM d16:1,C24:0), | 0.902 | 0.909 |
| ACES_89 | Propionylcarnitine, alpha-Ketoglutarate, Proline, (SM d16:1,C18:0) ratio to (SM d16:1,C24:0), | 0.918 | 0.936 |
| ACES_9 | Propionylcarnitine, alpha-Ketoglutarate, Proline, (Cer d18:1,C16:0) ratio to (Cer d18:1,C24:0), | 0.929 | 0.945 |
| ACES_90 | Butyrylcarnitine, alpha-Ketoglutarate, Proline, (SM d16:1,C18:0) ratio to (SM d16:1,C24:0), | 0.899 | 0.917 |
| ACES_91 | Octenoylcarnitine, alpha-Ketoglutarate, Proline, (SM d16:1,C18:0) ratio to (SM d16:1,C24:0), | 0.901 | 0.909 |
| ACES_93 | Propionylcarnitine, alpha-Ketoglutarate, Histidine, (SM d16:1,C18:0) ratio to (SM d16:1,C24:0), | 0.927 | 0.939 |
| ACES_94 | Butyrylcarnitine, alpha-Ketoglutarate, Histidine, (SM d16:1,C18:0) ratio to (SM d16:1,C24:0), | 0.904 | 0.917 |
| ACES_95 | Octenoylcarnitine, alpha-Ketoglutarate, Histidine, (SM d16:1,C18:0) ratio to (SM d16:1,C24:0), | 0.895 | 0.905 |

| ACLS_1 | Propionylcarnitine, Proline, (Cer d18:1,C16:0) ratio to (Cer d18:1,C24:0), Lysophosphatidylethanolamine (C18:2), | 0.902 | 0.930 |
|---|---|---|---|
| ACLS_10 | Butyrylcarnitine, Proline, (Cer d18:1,C18:0) ratio to (Cer d18:1,C24:0), Lysophosphatidylethanolamine (C18:2), | 0.901 | 0.917 |
| ACLS_11 | Octenoylcarnitine, Proline, (Cer d18:1,C18:0) ratio to (Cer d18:1,C24:0), Lysophosphatidylethanolamine (C18:2), | 0.906 | 0.917 |
| ACLS_13 | Propionylcarnitine, Histidine, (Cer d18:1,C18:0) ratio to (Cer d18:1,C24:0), Lysophosphatidylethanolamine (C18:2), | 0.917 | 0.931 |
| ACLS_14 | Butyrylcarnitine, Histidine, (Cer d18:1,C18:0) ratio to (Cer d18:1,C24:0), Lysophosphatidylethanolamine (C18:2), | 0.902 | 0.912 |
| ACLS_15 | Octenoylcarnitine, Histidine, (Cer d18:1,C18:0) ratio to (Cer d18:1,C24:0), Lysophosphatidylethanolamine (C18:2), | 0.900 | 0.903 |
| ACLS_17 | Propionylcarnitine, Proline, (Cer d18:1,C16:0 plus Cer d18:1,C18:0) ratio to (Cer d18:1,C24:0), Lysophosphatidylethanolamine (C18:2), | 0.910 | 0.931 |
| ACLS_18 | Butyrylcarnitine, Proline, (Cer d18:1,C16:0 plus Cer d18:1,C18:0) ratio to (Cer d18:1,C24:0), Lysophosphatidylethanolamine (C18:2), | 0.904 | 0.921 |
| ACLS_19 | Octenoylcarnitine, Proline, (Cer d18:1,C16:0 plus Cer d18:1,C18:0) ratio to (Cer d18:1,C24:0), Lysophosphatidylethanolamine (C18:2), | 0.908 | 0.922 |
| ACLS_2 | Butyrylcarnitine, Proline, (Cer d18:1,C16:0) ratio to (Cer d18:1,C24:0), Lysophosphatidylethanolamine (C18:2), | 0.900 | 0.921 |
| ACLS_21 | Propionylcarnitine, Histidine, (Cer d18:1,C16:0 plus Cer d18:1,C18:0) ratio to (Cer d18:1,C24:0), Lysophosphatidylethanolamine (C18:2), | 0.914 | 0.930 |
| ACLS_22 | Butyrylcarnitine, Histidine, (Cer d18:1,C16:0 plus Cer d18:1,C18:0) ratio to (Cer d18:1,C24:0), Lysophosphatidylethanolamine (C18:2), | 0.904 | 0.914 |
| ACLS_23 | Octenoylcarnitine, Histidine, (Cer d18:1,C16:0 plus Cer d18:1,C18:0) ratio to (Cer d18:1,C24:0), Lysophosphatidylethanolamine (C18:2), | 0.902 | 0.908 |
| ACLS_25 | Propionylcarnitine, Proline, (SM d17:1,C18:0) ratio to (SM d17:1,C24:0), Lysophosphatidylethanolamine (C18:2), | 0.900 | 0.925 |
| ACLS_26 | Butyrylcarnitine, Proline, (SM d17:1,C18:0) ratio to (SM d17:1,C24:0), Lysophosphatidylethanolamine (C18:2), | 0.890 | 0.910 |
| ACLS_27 | Octenoylcarnitine, Proline, (SM d17:1,C18:0) ratio to (SM d17:1,C24:0), Lysophosphatidylethanolamine (C18:2), | 0.893 | 0.911 |
| ACLS_29 | Propionylcarnitine, Histidine, (SM d17:1,C18:0) ratio to (SM d17:1,C24:0), Lysophosphatidylethanolamine (C18:2), | 0.908 | 0.927 |
| ACLS_3 | Octenoylcarnitine, Proline, (Cer d18:1,C16:0) ratio to (Cer | 0.908 | 0.924 |

| | | | | |
|---|---|---|---|---|
| | d18:1,C24:0), Lysophosphatidylethanolamine (C18:2), | | | |
| ACLS_30 | Butyrylcarnitine, Histidine, (SM d17:1,C18:0) ratio to (SM d17:1,C24:0), Lysophosphatidylethanolamine (C18:2), | 0.892 | 0.911 |
| ACLS_31 | Octenoylcarnitine, Histidine, (SM d17:1,C18:0) ratio to (SM d17:1,C24:0), Lysophosphatidylethanolamine (C18:2), | 0.888 | 0.902 |
| ACLS_33 | Propionylcarnitine, Proline, (SM d18:2,C18:0) ratio to (SM d18:2,C24:0), Lysophosphatidylethanolamine (C18:2), | 0.884 | 0.918 |
| ACLS_34 | Butyrylcarnitine, Proline, (SM d18:2,C18:0) ratio to (SM d18:2,C24:0), Lysophosphatidylethanolamine (C18:2), | 0.865 | 0.897 |
| ACLS_35 | Octenoylcarnitine, Proline, (SM d18:2,C18:0) ratio to (SM d18:2,C24:0), Lysophosphatidylethanolamine (C18:2), | 0.881 | 0.902 |
| ACLS_37 | Propionylcarnitine, Histidine, (SM d18:2,C18:0) ratio to (SM d18:2,C24:0), Lysophosphatidylethanolamine (C18:2), | 0.893 | 0.919 |
| ACLS_38 | Butyrylcarnitine, Histidine, (SM d18:2,C18:0) ratio to (SM d18:2,C24:0), Lysophosphatidylethanolamine (C18:2), | 0.866 | 0.894 |
| ACLS_39 | Octenoylcarnitine, Histidine, (SM d18:2,C18:0) ratio to (SM d18:2,C24:0), Lysophosphatidylethanolamine (C18:2), | 0.873 | 0.890 |
| ACLS_41 | Propionylcarnitine, Proline, (SM d16:1,C18:0) ratio to (SM d16:1,C24:0), Lysophosphatidylethanolamine (C18:2), | 0.889 | 0.921 |
| ACLS_42 | Butyrylcarnitine, Proline, (SM d16:1,C18:0) ratio to (SM d16:1,C24:0), Lysophosphatidylethanolamine (C18:2), | 0.876 | 0.905 |
| ACLS_43 | Octenoylcarnitine, Proline, (SM d16:1,C18:0) ratio to (SM d16:1,C24:0), Lysophosphatidylethanolamine (C18:2), | 0.888 | 0.905 |
| ACLS_45 | Propionylcarnitine, Histidine, (SM d16:1,C18:0) ratio to (SM d16:1,C24:0), Lysophosphatidylethanolamine (C18:2), | 0.897 | 0.922 |
| ACLS_46 | Butyrylcarnitine, Histidine, (SM d16:1,C18:0) ratio to (SM d16:1,C24:0), Lysophosphatidylethanolamine (C18:2), | 0.880 | 0.901 |
| ACLS_47 | Octenoylcarnitine, Histidine, (SM d16:1,C18:0) ratio to (SM d16:1,C24:0), Lysophosphatidylethanolamine (C18:2), | 0.884 | 0.896 |
| ACLS_5 | Propionylcarnitine, Histidine, (Cer d18:1,C16:0) ratio to (Cer d18:1,C24:0), Lysophosphatidylethanolamine (C18:2), | 0.906 | 0.927 |
| ACLS_6 | Butyrylcarnitine, Histidine, (Cer d18:1,C16:0) ratio to (Cer d18:1,C24:0), Lysophosphatidylethanolamine (C18:2), | 0.897 | 0.912 |
| ACLS_7 | Octenoylcarnitine, Histidine, (Cer d18:1,C16:0) ratio to (Cer d18:1,C24:0), Lysophosphatidylethanolamine (C18:2), | 0.899 | 0.907 |
| ACLS_9 | Propionylcarnitine, Proline, (Cer d18:1,C18:0) ratio to (Cer d18:1,C24:0), Lysophosphatidylethanolamine (C18:2), | 0.913 | 0.930 |
| ACS_1 | Propionylcarnitine, Proline, (Cer d18:1,C16:0) ratio to (Cer d18:1,C24:0), | 0.921 | 0.929 |
| ACS_10 | Butyrylcarnitine, Proline, (Cer d18:1,C18:0) ratio to (Cer | 0.911 | 0.920 |

| | | | | |
|---|---|---|---|---|
| | d18:1,C24:0), | | | |
| ACS_11 | Octenoylcarnitine, Proline, (Cer d18:1,C18:0) ratio to (Cer d18:1,C24:0), | 0.906 | 0.921 |
| ACS_13 | Propionylcarnitine, Histidine, (Cer d18:1,C18:0) ratio to (Cer d18:1,C24:0), | 0.930 | 0.933 |
| ACS_14 | Butyrylcarnitine, Histidine, (Cer d18:1,C18:0) ratio to (Cer d18:1,C24:0), | 0.912 | 0.913 |
| ACS_15 | Octenoylcarnitine, Histidine, (Cer d18:1,C18:0) ratio to (Cer d18:1,C24:0), | 0.903 | 0.905 |
| ACS_17 | Propionylcarnitine, Proline, (Cer d18:1,C16:0 plus Cer d18:1,C18:0) ratio to (Cer d18:1,C24:0), | 0.924 | 0.931 |
| ACS_18 | Butyrylcarnitine, Proline, (Cer d18:1,C16:0 plus Cer d18:1,C18:0) ratio to (Cer d18:1,C24:0), | 0.915 | 0.923 |
| ACS_19 | Octenoylcarnitine, Proline, (Cer d18:1,C16:0 plus Cer d18:1,C18:0) ratio to (Cer d18:1,C24:0), | 0.911 | 0.923 |
| ACS_2 | Butyrylcarnitine, Proline, (Cer d18:1,C16:0) ratio to (Cer d18:1,C24:0), | 0.910 | 0.923 |
| ACS_21 | Propionylcarnitine, Histidine, (Cer d18:1,C16:0 plus Cer d18:1,C18:0) ratio to (Cer d18:1,C24:0), | 0.930 | 0.934 |
| ACS_22 | Butyrylcarnitine, Histidine, (Cer d18:1,C16:0 plus Cer d18:1,C18:0) ratio to (Cer d18:1,C24:0), | 0.913 | 0.913 |
| ACS_23 | Octenoylcarnitine, Histidine, (Cer d18:1,C16:0 plus Cer d18:1,C18:0) ratio to (Cer d18:1,C24:0), | 0.905 | 0.907 |
| ACS_25 | Propionylcarnitine, Proline, (SM d17:1,C18:0) ratio to (SM d17:1,C24:0), | 0.917 | 0.922 |
| ACS_26 | Butyrylcarnitine, Proline, (SM d17:1,C18:0) ratio to (SM d17:1,C24:0), | 0.898 | 0.910 |
| ACS_27 | Octenoylcarnitine, Proline, (SM d17:1,C18:0) ratio to (SM d17:1,C24:0), | 0.890 | 0.914 |
| ACS_29 | Propionylcarnitine, Histidine, (SM d17:1,C18:0) ratio to (SM d17:1,C24:0), | 0.920 | 0.927 |
| ACS_3 | Octenoylcarnitine, Proline, (Cer d18:1,C16:0) ratio to (Cer d18:1,C24:0), | 0.912 | 0.926 |
| ACS_30 | Butyrylcarnitine, Histidine, (SM d17:1,C18:0) ratio to (SM d17:1,C24:0), | 0.899 | 0.905 |
| ACS_31 | Octenoylcarnitine, Histidine, (SM d17:1,C18:0) ratio to (SM d17:1,C24:0), | 0.878 | 0.897 |
| ACS_33 | Propionylcarnitine, Proline, (SM d18:2,C18:0) ratio to (SM d18:2,C24:0), | 0.907 | 0.917 |
| ACS_34 | Butyrylcarnitine, Proline, (SM d18:2,C18:0) ratio to (SM d18:2,C24:0), | 0.875 | 0.897 |
| ACS_35 | Octenoylcarnitine, Proline, (SM d18:2,C18:0) ratio to (SM | 0.878 | 0.903 |

| | | | | |
|---|---|---|---|---|
| | d18:2,C24:0), | | | |
| ACS_37 | Propionylcarnitine, Histidine, (SM d18:2,C18:0) ratio to (SM d18:2,C24:0), | 0.911 | 0.919 |
| ACS_38 | Butyrylcarnitine, Histidine, (SM d18:2,C18:0) ratio to (SM d18:2,C24:0), | 0.868 | 0.889 |
| ACS_39 | Octenoylcarnitine, Histidine, (SM d18:2,C18:0) ratio to (SM d18:2,C24:0), | 0.865 | 0.883 |
| ACS_41 | Propionylcarnitine, Proline, (SM d16:1,C18:0) ratio to (SM d16:1,C24:0), | 0.908 | 0.917 |
| ACS_42 | Butyrylcarnitine, Proline, (SM d16:1,C18:0) ratio to (SM d16:1,C24:0), | 0.887 | 0.904 |
| ACS_43 | Octenoylcarnitine, Proline, (SM d16:1,C18:0) ratio to (SM d16:1,C24:0), | 0.883 | 0.904 |
| ACS_45 | Propionylcarnitine, Histidine, (SM d16:1,C18:0) ratio to (SM d16:1,C24:0), | 0.913 | 0.921 |
| ACS_46 | Butyrylcarnitine, Histidine, (SM d16:1,C18:0) ratio to (SM d16:1,C24:0), | 0.882 | 0.895 |
| ACS_47 | Octenoylcarnitine, Histidine, (SM d16:1,C18:0) ratio to (SM d16:1,C24:0), | 0.872 | 0.887 |
| ACS_5 | Propionylcarnitine, Histidine, (Cer d18:1,C16:0) ratio to (Cer d18:1,C24:0), | 0.922 | 0.929 |
| ACS_6 | Butyrylcarnitine, Histidine, (Cer d18:1,C16:0) ratio to (Cer d18:1,C24:0), | 0.907 | 0.910 |
| ACS_7 | Octenoylcarnitine, Histidine, (Cer d18:1,C16:0) ratio to (Cer d18:1,C24:0), | 0.903 | 0.905 |
| ACS_9 | Propionylcarnitine, Proline, (Cer d18:1,C18:0) ratio to (Cer d18:1,C24:0), | 0.926 | 0.931 |
| AEL_1 | Coenzyme Q9, Proline, Lysophosphatidylethanolamine (C18:2), | 0.865 | 0.884 |
| AEL_2 | alpha-Ketoglutarate, Proline, Lysophosphatidylethanolamine (C18:2), | 0.871 | 0.905 |
| AEL_3 | Coenzyme Q9, Histidine, Lysophosphatidylethanolamine (C18:2), | 0.859 | 0.875 |
| AEL_4 | alpha-Ketoglutarate, Histidine, Lysophosphatidylethanolamine (C18:2), | 0.866 | 0.896 |
| AELS_1 | Coenzyme Q9, Proline, (Cer d18:1,C16:0) ratio to (Cer d18:1,C24:0), Lysophosphatidylethanolamine (C18:2), | 0.904 | 0.908 |
| AELS_10 | alpha-Ketoglutarate, Proline, (Cer d18:1,C16:0 plus Cer d18:1,C18:0) ratio to (Cer d18:1,C24:0), Lysophosphatidylethanolamine (C18:2), | 0.908 | 0.912 |
| AELS_11 | Coenzyme Q9, Histidine, (Cer d18:1,C16:0 plus Cer d18:1,C18:0) ratio to (Cer d18:1,C24:0), | 0.908 | 0.899 |

| | | | | |
|---|---|---|---|---|
| | Lysophosphatidylethanolamine (C18:2), | | | |
| AELS_12 | alpha-Ketoglutarate, Histidine, (Cer d18:1,C16:0 plus Cer d18:1,C18:0) ratio to (Cer d18:1,C24:0), Lysophosphatidylethanolamine (C18:2), | 0.900 | 0.898 |
| AELS_13 | Coenzyme Q9, Proline, (SM d17:1,C18:0) ratio to (SM d17:1,C24:0), Lysophosphatidylethanolamine (C18:2), | 0.900 | 0.905 |
| AELS_14 | alpha-Ketoglutarate, Proline, (SM d17:1,C18:0) ratio to (SM d17:1,C24:0), Lysophosphatidylethanolamine (C18:2), | 0.896 | 0.906 |
| AELS_15 | Coenzyme Q9, Histidine, (SM d17:1,C18:0) ratio to (SM d17:1,C24:0), Lysophosphatidylethanolamine (C18:2), | 0.899 | 0.900 |
| AELS_16 | alpha-Ketoglutarate, Histidine, (SM d17:1,C18:0) ratio to (SM d17:1,C24:0), Lysophosphatidylethanolamine (C18:2), | 0.891 | 0.898 |
| AELS_17 | Coenzyme Q9, Proline, (SM d18:2,C18:0) ratio to (SM d18:2,C24:0), Lysophosphatidylethanolamine (C18:2), | 0.889 | 0.896 |
| AELS_18 | alpha-Ketoglutarate, Proline, (SM d18:2,C18:0) ratio to (SM d18:2,C24:0), Lysophosphatidylethanolamine (C18:2), | 0.883 | 0.897 |
| AELS_19 | Coenzyme Q9, Histidine, (SM d18:2,C18:0) ratio to (SM d18:2,C24:0), Lysophosphatidylethanolamine (C18:2), | 0.887 | 0.891 |
| AELS_2 | alpha-Ketoglutarate, Proline, (Cer d18:1,C16:0) ratio to (Cer d18:1,C24:0), Lysophosphatidylethanolamine (C18:2), | 0.904 | 0.915 |
| AELS_20 | alpha-Ketoglutarate, Histidine, (SM d18:2,C18:0) ratio to (SM d18:2,C24:0), Lysophosphatidylethanolamine (C18:2), | 0.879 | 0.890 |
| AELS_21 | Coenzyme Q9, Proline, (SM d16:1,C18:0) ratio to (SM d16:1,C24:0), Lysophosphatidylethanolamine (C18:2), | 0.893 | 0.901 |
| AELS_22 | alpha-Ketoglutarate, Proline, (SM d16:1,C18:0) ratio to (SM d16:1,C24:0), Lysophosphatidylethanolamine (C18:2), | 0.890 | 0.900 |
| AELS_23 | Coenzyme Q9, Histidine, (SM d16:1,C18:0) ratio to (SM d16:1,C24:0), Lysophosphatidylethanolamine (C18:2), | 0.896 | 0.896 |
| AELS_24 | alpha-Ketoglutarate, Histidine, (SM d16:1,C18:0) ratio to (SM d16:1,C24:0), Lysophosphatidylethanolamine (C18:2), | 0.887 | 0.890 |
| AELS_3 | Coenzyme Q9, Histidine, (Cer d18:1,C16:0) ratio to (Cer d18:1,C24:0), Lysophosphatidylethanolamine (C18:2), | 0.903 | 0.897 |
| AELS_4 | alpha-Ketoglutarate, Histidine, (Cer d18:1,C16:0) ratio to (Cer d18:1,C24:0), Lysophosphatidylethanolamine (C18:2), | 0.894 | 0.900 |
| AELS_5 | Coenzyme Q9, Proline, (Cer d18:1,C18:0) ratio to (Cer d18:1,C24:0), Lysophosphatidylethanolamine (C18:2), | 0.908 | 0.907 |
| AELS_6 | alpha-Ketoglutarate, Proline, (Cer d18:1,C18:0) ratio to (Cer d18:1,C24:0), Lysophosphatidylethanolamine (C18:2), | 0.907 | 0.907 |
| AELS_7 | Coenzyme Q9, Histidine, (Cer d18:1,C18:0) ratio to (Cer d18:1,C24:0), Lysophosphatidylethanolamine (C18:2), | 0.908 | 0.898 |

| AELS_8 | alpha-Ketoglutarate, Histidine, (Cer d18:1,C18:0) ratio to (Cer d18:1,C24:0), Lysophosphatidylethanolamine (C18:2), | 0.901 | 0.894 |
|---|---|---|---|
| AELS_9 | Coenzyme Q9, Proline, (Cer d18:1,C16:0 plus Cer d18:1,C18:0) ratio to (Cer d18:1,C24:0), Lysophosphatidylethanolamine (C18:2), | 0.909 | 0.909 |
| AES_1 | Coenzyme Q9, Proline, (Cer d18:1,C16:0) ratio to (Cer d18:1,C24:0), | 0.908 | 0.907 |
| AES_10 | alpha-Ketoglutarate, Proline, (Cer d18:1,C16:0 plus Cer d18:1,C18:0) ratio to (Cer d18:1,C24:0), | 0.913 | 0.914 |
| AES_11 | Coenzyme Q9, Histidine, (Cer d18:1,C16:0 plus Cer d18:1,C18:0) ratio to (Cer d18:1,C24:0), | 0.912 | 0.896 |
| AES_12 | alpha-Ketoglutarate, Histidine, (Cer d18:1,C16:0 plus Cer d18:1,C18:0) ratio to (Cer d18:1,C24:0), | 0.909 | 0.905 |
| AES_13 | Coenzyme Q9, Proline, (SM d17:1,C18:0) ratio to (SM d17:1,C24:0), | 0.897 | 0.900 |
| AES_14 | alpha-Ketoglutarate, Proline, (SM d17:1,C18:0) ratio to (SM d17:1,C24:0), | 0.900 | 0.909 |
| AES_15 | Coenzyme Q9, Histidine, (SM d17:1,C18:0) ratio to (SM d17:1,C24:0), | 0.898 | 0.896 |
| AES_16 | alpha-Ketoglutarate, Histidine, (SM d17:1,C18:0) ratio to (SM d17:1,C24:0), | 0.898 | 0.901 |
| AES_17 | Coenzyme Q9, Proline, (SM d18:2,C18:0) ratio to (SM d18:2,C24:0), | 0.885 | 0.896 |
| AES_18 | alpha-Ketoglutarate, Proline, (SM d18:2,C18:0) ratio to (SM d18:2,C24:0), | 0.886 | 0.902 |
| AES_19 | Coenzyme Q9, Histidine, (SM d18:2,C18:0) ratio to (SM d18:2,C24:0), | 0.883 | 0.886 |
| AES_2 | alpha-Ketoglutarate, Proline, (Cer d18:1,C16:0) ratio to (Cer d18:1,C24:0), | 0.912 | 0.916 |
| AES_20 | alpha-Ketoglutarate, Histidine, (SM d18:2,C18:0) ratio to (SM d18:2,C24:0), | 0.885 | 0.891 |
| AES_21 | Coenzyme Q9, Proline, (SM d16:1,C18:0) ratio to (SM d16:1,C24:0), | 0.892 | 0.897 |
| AES_22 | alpha-Ketoglutarate, Proline, (SM d16:1,C18:0) ratio to (SM d16:1,C24:0), | 0.893 | 0.902 |
| AES_23 | Coenzyme Q9, Histidine, (SM d16:1,C18:0) ratio to (SM d16:1,C24:0), | 0.889 | 0.891 |
| AES_24 | alpha-Ketoglutarate, Histidine, (SM d16:1,C18:0) ratio to (SM d16:1,C24:0), | 0.892 | 0.893 |
| AES_3 | Coenzyme Q9, Histidine, (Cer d18:1,C16:0) ratio to (Cer d18:1,C24:0), | 0.907 | 0.896 |

| AES_4 | alpha-Ketoglutarate, Histidine, (Cer d18:1,C16:0) ratio to (Cer d18:1,C24:0), | 0.906 | 0.906 |
|---|---|---|---|
| AES_5 | Coenzyme Q9, Proline, (Cer d18:1,C18:0) ratio to (Cer d18:1,C24:0), | 0.909 | 0.903 |
| AES_6 | alpha-Ketoglutarate, Proline, (Cer d18:1,C18:0) ratio to (Cer d18:1,C24:0), | 0.909 | 0.911 |
| AES_7 | Coenzyme Q9, Histidine, (Cer d18:1,C18:0) ratio to (Cer d18:1,C24:0), | 0.909 | 0.895 |
| AES_8 | alpha-Ketoglutarate, Histidine, (Cer d18:1,C18:0) ratio to (Cer d18:1,C24:0), | 0.907 | 0.902 |
| AES_9 | Coenzyme Q9, Proline, (Cer d18:1,C16:0 plus Cer d18:1,C18:0) ratio to (Cer d18:1,C24:0), | 0.909 | 0.906 |
| CEL_1 | Propionylcarnitine, Coenzyme Q9, Lysophosphatidylethanolamine (C18:2), | 0.905 | 0.918 |
| CEL_2 | Propionylcarnitine, alpha-Ketoglutarate, Lysophosphatidylethanolamine (C18:2), | 0.917 | 0.943 |
| CEL_3 | Butyrylcarnitine, Coenzyme Q9, Lysophosphatidylethanolamine (C18:2), | 0.863 | 0.885 |
| CEL_4 | Butyrylcarnitine, alpha-Ketoglutarate, Lysophosphatidylethanolamine (C18:2), | 0.878 | 0.921 |
| CEL_5 | Octenoylcarnitine, Coenzyme Q9, Lysophosphatidylethanolamine (C18:2), | 0.867 | 0.895 |
| CEL_6 | Octenoylcarnitine, alpha-Ketoglutarate, Lysophosphatidylethanolamine (C18:2), | 0.874 | 0.906 |
| CELS_1 | Propionylcarnitine, Coenzyme Q9, (Cer d18:1,C16:0) ratio to (Cer d18:1,C24:0), Lysophosphatidylethanolamine (C18:2), | 0.919 | 0.929 |
| CELS_10 | Propionylcarnitine, alpha-Ketoglutarate, (Cer d18:1,C18:0) ratio to (Cer d18:1,C24:0), Lysophosphatidylethanolamine (C18:2), | 0.933 | 0.943 |
| CELS_11 | Butyrylcarnitine, Coenzyme Q9, (Cer d18:1,C18:0) ratio to (Cer d18:1,C24:0), Lysophosphatidylethanolamine (C18:2), | 0.912 | 0.916 |
| CELS_12 | Butyrylcarnitine, alpha-Ketoglutarate, (Cer d18:1,C18:0) ratio to (Cer d18:1,C24:0), Lysophosphatidylethanolamine (C18:2), | 0.918 | 0.925 |
| CELS_13 | Octenoylcarnitine, Coenzyme Q9, (Cer d18:1,C18:0) ratio to (Cer d18:1,C24:0), Lysophosphatidylethanolamine (C18:2), | 0.913 | 0.915 |
| CELS_14 | Octenoylcarnitine, alpha-Ketoglutarate, (Cer d18:1,C18:0) ratio to (Cer d18:1,C24:0), Lysophosphatidylethanolamine (C18:2), | 0.910 | 0.907 |
| CELS_17 | Propionylcarnitine, Coenzyme Q9, (Cer d18:1,C16:0 plus | 0.923 | 0.931 |

| | | | | |
|---|---|---|---|---|
| | Cer d18:1,C18:0) ratio to (Cer d18:1,C24:0), Lysophosphatidylethanolamine (C18:2), | | | |
| CELS_18 | Propionylcarnitine, alpha-Ketoglutarate, (Cer d18:1,C16:0 plus Cer d18:1,C18:0) ratio to (Cer d18:1,C24:0), Lysophosphatidylethanolamine (C18:2), | 0.931 | 0.943 | |
| CELS_19 | Butyrylcarnitine, Coenzyme Q9, (Cer d18:1,C16:0 plus Cer d18:1,C18:0) ratio to (Cer d18:1,C24:0), Lysophosphatidylethanolamine (C18:2), | 0.911 | 0.917 | |
| CELS_2 | Propionylcarnitine, alpha-Ketoglutarate, (Cer d18:1,C16:0) ratio to (Cer d18:1,C24:0), Lysophosphatidylethanolamine (C18:2), | 0.929 | 0.943 | |
| CELS_20 | Butyrylcarnitine, alpha-Ketoglutarate, (Cer d18:1,C16:0 plus Cer d18:1,C18:0) ratio to (Cer d18:1,C24:0), Lysophosphatidylethanolamine (C18:2), | 0.918 | 0.929 | |
| CELS_21 | Octenoylcarnitine, Coenzyme Q9, (Cer d18:1,C16:0 plus Cer d18:1,C18:0) ratio to (Cer d18:1,C24:0), Lysophosphatidylethanolamine (C18:2), | 0.915 | 0.915 | |
| CELS_22 | Octenoylcarnitine, alpha-Ketoglutarate, (Cer d18:1,C16:0 plus Cer d18:1,C18:0) ratio to (Cer d18:1,C24:0), Lysophosphatidylethanolamine (C18:2), | 0.912 | 0.912 | |
| CELS_25 | Propionylcarnitine, Coenzyme Q9, (SM d17:1,C18:0) ratio to (SM d17:1,C24:0), Lysophosphatidylethanolamine (C18:2), | 0.917 | 0.927 | |
| CELS_26 | Propionylcarnitine, alpha-Ketoglutarate, (SM d17:1,C18:0) ratio to (SM d17:1,C24:0), Lysophosphatidylethanolamine (C18:2), | 0.923 | 0.939 | |
| CELS_27 | Butyrylcarnitine, Coenzyme Q9, (SM d17:1,C18:0) ratio to (SM d17:1,C24:0), Lysophosphatidylethanolamine (C18:2), | 0.902 | 0.914 | |
| CELS_28 | Butyrylcarnitine, alpha-Ketoglutarate, (SM d17:1,C18:0) ratio to (SM d17:1,C24:0), Lysophosphatidylethanolamine (C18:2), | 0.908 | 0.923 | |
| CELS_29 | Octenoylcarnitine, Coenzyme Q9, (SM d17:1,C18:0) ratio to (SM d17:1,C24:0), Lysophosphatidylethanolamine (C18:2), | 0.902 | 0.913 | |
| CELS_3 | Butyrylcarnitine, Coenzyme Q9, (Cer d18:1,C16:0) ratio to (Cer d18:1,C24:0), Lysophosphatidylethanolamine (C18:2), | 0.906 | 0.913 | |
| CELS_30 | Octenoylcarnitine, alpha-Ketoglutarate, (SM d17:1,C18:0) ratio to (SM d17:1,C24:0), Lysophosphatidylethanolamine (C18:2), | 0.897 | 0.911 | |
| CELS_33 | Propionylcarnitine, Coenzyme Q9, (SM d18:2,C18:0) ratio to (SM d18:2,C24:0), Lysophosphatidylethanolamine (C18:2), | 0.909 | 0.923 | |

| CELS_34 | Propionylcarnitine, alpha-Ketoglutarate, (SM d18:2,C18:0) ratio to (SM d18:2,C24:0), Lysophosphatidylethanolamine (C18:2), | 0.919 | 0.936 |
|---|---|---|---|
| CELS_35 | Butyrylcarnitine, Coenzyme Q9, (SM d18:2,C18:0) ratio to (SM d18:2,C24:0), Lysophosphatidylethanolamine (C18:2), | 0.890 | 0.904 |
| CELS_36 | Butyrylcarnitine, alpha-Ketoglutarate, (SM d18:2,C18:0) ratio to (SM d18:2,C24:0), Lysophosphatidylethanolamine (C18:2), | 0.894 | 0.913 |
| CELS_37 | Octenoylcarnitine, Coenzyme Q9, (SM d18:2,C18:0) ratio to (SM d18:2,C24:0), Lysophosphatidylethanolamine (C18:2), | 0.891 | 0.906 |
| CELS_38 | Octenoylcarnitine, alpha-Ketoglutarate, (SM d18:2,C18:0) ratio to (SM d18:2,C24:0), Lysophosphatidylethanolamine (C18:2), | 0.887 | 0.900 |
| CELS_4 | Butyrylcarnitine, alpha-Ketoglutarate, (Cer d18:1,C16:0) ratio to (Cer d18:1,C24:0), Lysophosphatidylethanolamine (C18:2), | 0.913 | 0.929 |
| CELS_41 | Propionylcarnitine, Coenzyme Q9, (SM d16:1,C18:0) ratio to (SM d16:1,C24:0), Lysophosphatidylethanolamine (C18:2), | 0.914 | 0.925 |
| CELS_42 | Propionylcarnitine, alpha-Ketoglutarate, (SM d16:1,C18:0) ratio to (SM d16:1,C24:0), Lysophosphatidylethanolamine (C18:2), | 0.922 | 0.937 |
| CELS_43 | Butyrylcarnitine, Coenzyme Q9, (SM d16:1,C18:0) ratio to (SM d16:1,C24:0), Lysophosphatidylethanolamine (C18:2), | 0.894 | 0.907 |
| CELS_44 | Butyrylcarnitine, alpha-Ketoglutarate, (SM d16:1,C18:0) ratio to (SM d16:1,C24:0), Lysophosphatidylethanolamine (C18:2), | 0.902 | 0.917 |
| CELS_45 | Octenoylcarnitine, Coenzyme Q9, (SM d16:1,C18:0) ratio to (SM d16:1,C24:0), Lysophosphatidylethanolamine (C18:2), | 0.898 | 0.909 |
| CELS_46 | Octenoylcarnitine, alpha-Ketoglutarate, (SM d16:1,C18:0) ratio to (SM d16:1,C24:0), Lysophosphatidylethanolamine (C18:2), | 0.895 | 0.903 |
| CELS_5 | Octenoylcarnitine, Coenzyme Q9, (Cer d18:1,C16:0) ratio to (Cer d18:1,C24:0), Lysophosphatidylethanolamine (C18:2), | 0.911 | 0.917 |
| CELS_6 | Octenoylcarnitine, alpha-Ketoglutarate, (Cer d18:1,C16:0) ratio to (Cer d18:1,C24:0), Lysophosphatidylethanolamine (C18:2), | 0.911 | 0.913 |
| CELS_9 | Propionylcarnitine, Coenzyme Q9, (Cer d18:1,C18:0) ratio to (Cer d18:1,C24:0), Lysophosphatidylethanolamine (C18:2), | 0.925 | 0.932 |

| CES_1 | Propionylcarnitine, Coenzyme Q9, (Cer d18:1,C16:0) ratio to (Cer d18:1,C24:0), | 0.925 | 0.930 |
|---|---|---|---|
| CES_10 | Propionylcarnitine, alpha-Ketoglutarate, (Cer d18:1,C18:0) ratio to (Cer d18:1,C24:0), | 0.939 | 0.946 |
| CES_11 | Butyrylcarnitine, Coenzyme Q9, (Cer d18:1,C18:0) ratio to (Cer d18:1,C24:0), | 0.920 | 0.910 |
| CES_12 | Butyrylcarnitine, alpha-Ketoglutarate, (Cer d18:1,C18:0) ratio to (Cer d18:1,C24:0), | 0.926 | 0.931 |
| CES_13 | Octenoylcarnitine, Coenzyme Q9, (Cer d18:1,C18:0) ratio to (Cer d18:1,C24:0), | 0.911 | 0.911 |
| CES_14 | Octenoylcarnitine, alpha-Ketoglutarate, (Cer d18:1,C18:0) ratio to (Cer d18:1,C24:0), | 0.910 | 0.915 |
| CES_17 | Propionylcarnitine, Coenzyme Q9, (Cer d18:1,C16:0 plus Cer d18:1,C18:0) ratio to (Cer d18:1,C24:0), | 0.926 | 0.930 |
| CES_18 | Propionylcarnitine, alpha-Ketoglutarate, (Cer d18:1,C16:0 plus Cer d18:1,C18:0) ratio to (Cer d18:1,C24:0), | 0.938 | 0.947 |
| CES_19 | Butyrylcarnitine, Coenzyme Q9, (Cer d18:1,C16:0 plus Cer d18:1,C18:0) ratio to (Cer d18:1,C24:0), | 0.919 | 0.910 |
| CES_2 | Propionylcarnitine, alpha-Ketoglutarate, (Cer d18:1,C16:0) ratio to (Cer d18:1,C24:0), | 0.935 | 0.946 |
| CES_20 | Butyrylcarnitine, alpha-Ketoglutarate, (Cer d18:1,C16:0 plus Cer d18:1,C18:0) ratio to (Cer d18:1,C24:0), | 0.928 | 0.934 |
| CES_21 | Octenoylcarnitine, Coenzyme Q9, (Cer d18:1,C16:0 plus Cer d18:1,C18:0) ratio to (Cer d18:1,C24:0), | 0.914 | 0.912 |
| CES_22 | Octenoylcarnitine, alpha-Ketoglutarate, (Cer d18:1,C16:0 plus Cer d18:1,C18:0) ratio to (Cer d18:1,C24:0), | 0.914 | 0.917 |
| CES_25 | Propionylcarnitine, Coenzyme Q9, (SM d17:1,C18:0) ratio to (SM d17:1,C24:0), | 0.923 | 0.927 |
| CES_26 | Propionylcarnitine, alpha-Ketoglutarate, (SM d17:1,C18:0) ratio to (SM d17:1,C24:0), | 0.933 | 0.944 |
| CES_27 | Butyrylcarnitine, Coenzyme Q9, (SM d17:1,C18:0) ratio to (SM d17:1,C24:0), | 0.909 | 0.906 |
| CES_28 | Butyrylcarnitine, alpha-Ketoglutarate, (SM d17:1,C18:0) ratio to (SM d17:1,C24:0), | 0.918 | 0.929 |
| CES_29 | Octenoylcarnitine, Coenzyme Q9, (SM d17:1,C18:0) ratio to (SM d17:1,C24:0), | 0.899 | 0.914 |
| CES_3 | Butyrylcarnitine, Coenzyme Q9, (Cer d18:1,C16:0) ratio to (Cer d18:1,C24:0), | 0.915 | 0.907 |
| CES_30 | Octenoylcarnitine, alpha-Ketoglutarate, (SM d17:1,C18:0) ratio to (SM d17:1,C24:0), | 0.898 | 0.915 |
| CES_33 | Propionylcarnitine, Coenzyme Q9, (SM d18:2,C18:0) ratio to (SM d18:2,C24:0), | 0.913 | 0.923 |

| CES_34 | Propionylcarnitine, alpha-Ketoglutarate, (SM d18:2,C18:0) ratio to (SM d18:2,C24:0), | 0.928 | 0.942 |
|---|---|---|---|
| CES_35 | Butyrylcarnitine, Coenzyme Q9, (SM d18:2,C18:0) ratio to (SM d18:2,C24:0), | 0.889 | 0.900 |
| CES_36 | Butyrylcarnitine, alpha-Ketoglutarate, (SM d18:2,18:0) ratio to (SM d18:2,C24:0), | 0.901 | 0.924 |
| CES_37 | Octenoylcarnitine, Coenzyme Q9, (SM d18:2,C18:0) ratio to (SM d18:2,C24:0), | 0.886 | 0.904 |
| CES_38 | Octenoylcarnitine, alpha-Ketoglutarate, (SM d18:2,C18:0) ratio to (SM d18:2,C24:0), | 0.887 | 0.904 |
| CES_4 | Butyrylcarnitine, alpha-Ketoglutarate, (Cer d18:1,C16:0) ratio to (Cer d18:1,C24:0), | 0.926 | 0.934 |
| CES_41 | Propionylcarnitine, Coenzyme Q9, (SM d16:1,C18:0) ratio to (SM d16:1,C24:0), | 0.917 | 0.924 |
| CES_42 | Propionylcarnitine, alpha-Ketoglutarate, (SM d16:1,C18:0) ratio to (SM d16:1,C24:0), | 0.929 | 0.942 |
| CES_43 | Butyrylcarnitine, Coenzyme Q9, (SM d16:1,C18:0) ratio to (SM d16:1,C24:0), | 0.898 | 0.901 |
| CES_44 | Butyrylcarnitine, alpha-Ketoglutarate, (SM d16:1,C18:0) ratio to (SM d16:1,C24:0), | 0.910 | 0.924 |
| CES_45 | Octenoylcarnitine, Coenzyme Q9, (SM d16:1,C18:0) ratio to (SM d16:1,C24:0), | 0.892 | 0.906 |
| CES_46 | Octenoylcarnitine, alpha-Ketoglutarate, (SM d16:1,C18:0) ratio to (SM d16:1,C24:0), | 0.894 | 0.905 |
| CES_5 | Octenoylcarnitine, Coenzyme Q9, (Cer d18:1,C16:0) ratio to (Cer d18:1,C24:0), | 0.912 | 0.912 |
| CES_6 | Octenoylcarnitine, alpha-Ketoglutarate, (Cer d18:1,C16:0) ratio to (Cer d18:1,C24:0), | 0.913 | 0.917 |
| CES_9 | Propionylcarnitine, Coenzyme Q9, (Cer d18:1,C18:0) ratio to (Cer d18:1,C24:0), | 0.929 | 0.930 |
| CLS_1 | Propionylcarnitine, (Cer d18:1,C16:0) ratio to (Cer d18:1,C24:0), Lysophosphatidylethanolamine (C18:2), | 0.914 | 0.930 |
| CLS_10 | Butyrylcarnitine, (SM d17:1,C18:0) ratio to (SM d17:1,C24:0), Lysophosphatidylethanolamine (C18:2), | 0.891 | 0.910 |
| CLS_11 | Butyrylcarnitine, (SM d18:2,C18:0) ratio to (SM d18:2,C24:0), Lysophosphatidylethanolamine (C18:2), | 0.856 | 0.900 |
| CLS_12 | Butyrylcarnitine, (SM d16:1,C18:0) ratio to (SM d16:1,C24:0), Lysophosphatidylethanolamine (C18:2), | 0.872 | 0.903 |
| CLS_13 | Octenoylcarnitine, (Cer d18:1,C16:0) ratio to (Cer d18:1,C24:0), Lysophosphatidylethanolamine (C18:2), | 0.898 | 0.912 |
| CLS_14 | Octenoylcarnitine, (Cer d18:1,C18:0) ratio to (Cer d18:1,C24:0), Lysophosphatidylethanolamine (C18:2), | 0.895 | 0.910 |

| CLS_15 | Octenoylcarnitine, (Cer d18:1,C16:0 plus Cer d18:1,C18:0) ratio to (Cer d18:1,C24:0), Lysophosphatidylethanolamine (C18:2), | 0.899 | 0.911 |
|---|---|---|---|
| CLS_16 | Octenoylcarnitine, (SM d17:1,C18:0) ratio to (SM d17:1,C24:0), Lysophosphatidylethanolamine (C18:2), | 0.876 | 0.908 |
| CLS_17 | Octenoylcarnitine, (SM d18:2,C18:0) ratio to (SM d18:2,C24:0), Lysophosphatidylethanolamine (C18:2), | 0.857 | 0.897 |
| CLS_18 | Octenoylcarnitine, (SM d16:1,C18:0) ratio to (SM d16:1,C24:0), Lysophosphatidylethanolamine (C18:2), | 0.865 | 0.898 |
| CLS_2 | Propionylcarnitine, (Cer d18:1,C18:0) ratio to (Cer d18:1,C24:0), Lysophosphatidylethanolamine (C18:2), | 0.922 | 0.931 |
| CLS_3 | Propionylcarnitine, (Cer d18:1,C16:0 plus Cer d18:1,C18:0) ratio to (Cer d18:1,C24:0), Lysophosphatidylethanolamine (C18:2), | 0.921 | 0.931 |
| CLS_4 | Propionylcarnitine, (SM d17:1,C18:0) ratio to (SM d17:1,C24:0), Lysophosphatidylethanolamine (C18:2), | 0.913 | 0.926 |
| CLS_5 | Propionylcarnitine, (SM d18:2,C18:0) ratio to (SM d18:2,C24:0), Lysophosphatidylethanolamine (C18:2), | 0.899 | 0.920 |
| CLS_6 | Propionylcarnitine, (SM d16:1,C18:0) ratio to (SM d16:1,C24:0), Lysophosphatidylethanolamine (C18:2), | 0.904 | 0.922 |
| CLS_7 | Butyrylcarnitine, (Cer d18:1,C16:0) ratio to (Cer d18:1,C24:0), Lysophosphatidylethanolamine (C18:2), | 0.896 | 0.915 |
| CLS_8 | Butyrylcarnitine, (Cer d18:1,C18:0) ratio to (Cer d18:1,C24:0), Lysophosphatidylethanolamine (C18:2), | 0.903 | 0.916 |
| CLS_9 | Butyrylcarnitine, (Cer d18:1,C16:0 plus Cer d18:1,C18:0) ratio to (Cer d18:1,C24:0), Lysophosphatidylethanolamine (C18:2), | 0.906 | 0.918 |
| ELS_1 | Coenzyme Q9, (Cer d18:1,C16:0) ratio to (Cer d18:1,C24:0), Lysophosphatidylethanolamine (C18:2), | 0.894 | 0.895 |
| ELS_10 | alpha-Ketoglutarate, (SM d17:1,C18:0) ratio to (SM d17:1,C24:0), Lysophosphatidylethanolamine (C18:2), | 0.893 | 0.903 |
| ELS_11 | alpha-Ketoglutarate, (SM d18:2,C18:0) ratio to (SM d18:2,C24:0), Lysophosphatidylethanolamine (C18:2), | 0.874 | 0.896 |
| ELS_12 | alpha-Ketoglutarate, (SM d16:1,C18:0) ratio to (SM d16:1,C24:0), Lysophosphatidylethanolamine (C18:2), | 0.882 | 0.896 |
| ELS_2 | Coenzyme Q9, (Cer d18:1,C18:0) ratio to (Cer d18:1,C24:0), Lysophosphatidylethanolamine (C18:2), | 0.904 | 0.892 |
| ELS_3 | Coenzyme Q9, (Cer d18:1,C16:0 plus Cer d18:1,C18:0) ratio to (Cer d18:1,C24:0), Lysophosphatidylethanolamine (C18:2), | 0.905 | 0.895 |
| ELS_4 | Coenzyme Q9, (SM d17:1,C18:0) ratio to (SM d17:1,C24:0), Lysophosphatidylethanolamine (C18:2), | 0.887 | 0.897 |
| ELS_5 | Coenzyme Q9, (SM d18:2,C18:0) ratio to (SM | 0.869 | 0.888 |

| | | | |
|---|---|---|---|
| | d18:2,C24:0), Lysophosphatidylethanolamine (C18:2), | | |
| ELS_6 | Coenzyme Q9, (SM d16:1,C18:0) ratio to (SM d16:1,C24:0), Lysophosphatidylethanolamine (C18:2), | 0.879 | 0.890 |
| ELS_7 | alpha-Ketoglutarate, (Cer d18:1,C16:0) ratio to (Cer d18:1,C24:0), Lysophosphatidylethanolamine (C18:2), | 0.900 | 0.903 |
| ELS_8 | alpha-Ketoglutarate, (Cer d18:1,C18:0) ratio to (Cer d18:1,C24:0), Lysophosphatidylethanolamine (C18:2), | 0.901 | 0.901 |
| ELS_9 | alpha-Ketoglutarate, (Cer d18:1,C16:0 plus Cer d18:1,C18:0) ratio to (Cer d18:1,C24:0), Lysophosphatidylethanolamine (C18:2), | 0.904 | 0.903 |
| LC | PropionylcarnitineButyrylcarnitineOctenoylcarnitine, alpha-Ketoglutarate, Coenzyme Q9, Proline, (Cer d18:1,C18:0) ratio to (Cer d18:1,C24:0), (Cer d18:1,C16:0 plus Cer d18:1,C18:0) ratio to (Cer d18:1,C24:0), (SM d17:1,C18:0) ratio to (SM d17:1,C24:0), | 0.933 | 0.933 |
| Nom_3 | Propionylcarnitine, alpha-Ketoglutarate, Histidine, (Cer d18:1,C18:0) ratio to (Cer d18:1,C24:0), (SM d16:1,C18:0) ratio to (SM d16:1,C24:0), (SM d18:2,C18:0) ratio to (SM d18:2,C24:0), PG ratio of phosphoglycerides to total glycerides, Lysophosphatidylethanolamine (C18:2), Tryptophan | 0.934 | 0.935 |
| Nom_2 | Propionylcarnitine, alpha-Ketoglutarate, (SM d18:2,C18:0) ratio to (SM d18:2,C24:0), PG ratio of phosphoglycerides to total glycerides, | 0.922 | 0.939 |

Moreover, in Table 2, preferred scaling, weighting, and bias parameters are provided for preferred panels of Table 1. Preferably, the indicated values $\pm$ 50% of their respective values are used, more preferably the indicated values $\pm$ 20% of their respective values are used, even more preferably the indicated values $\pm$ 10% of their respective values are used, most preferably, the values indicated in Table 2 are used.

Table 2: Preferred parameters for preferred panels of table 1. NA=not applicable

| Panel designation | metabolite/feature | bias | coefficient | scaling factors | |
|---|---|---|---|---|---|
| | | $\omega_0$ | $\omega_i$ | $m_i$ | $s_i$ |
| ACELS_29 | CA19_9 | | 0.850 | 1.871 | 0.921 |
| ACELS_29 | NA | 2.246 | NA | NA | NA |
| ACELS_29 | Propionylcarnitine | | -0.669 | -0.225 | 0.226 |
| ACELS_29 | alpha-Ketoglutarate | | 0.331 | 0.020 | 0.274 |
| ACELS_29 | Histidine | | 0.000 | -0.157 | 0.110 |
| ACELS_29 | (Cer d18:1,C18:0) ratio to (Cer d18:1,C24:0) | | 0.423 | 0.469 | 0.349 |
| ACELS_29 | Lysophosphatidylethanolamine (C18:2) | | -0.037 | -0.237 | 0.180 |
| ACELS_41 | CA19_9 | | 0.861 | 1.871 | 0.921 |

(continued)

| Panel designation | metabolite/feature | bias | coefficient | scaling factors | |
|---|---|---|---|---|---|
| | | $\omega_0$ | $\omega_i$ | $m_i$ | $s_i$ |
| ACELS_41 | NA | 2.250 | NA | NA | NA |
| ACELS_41 | Propionylcarnitine | | -0.620 | -0.225 | 0.226 |
| ACELS_41 | alpha-Ketoglutarate | | 0.324 | 0.020 | 0.274 |
| ACELS_41 | Proline | | -0.111 | -0.152 | 0.158 |
| ACELS_41 | (Cer d18:1,C16:0 plus Cer d18:1,C18:0) ratio to (Cer d18:1,C24:0) | | 0.410 | 0.685 | 0.321 |
| ACELS_41 | Lysophosphatidylethanolamine (C18:2) | | -0.024 | -0.237 | 0.180 |
| ACELS_45 | CA19_9 | | 0.885 | 1.871 | 0.921 |
| ACELS_45 | NA | 2.298 | NA | NA | NA |
| ACELS_45 | Propionylcarnitine | | -0.699 | -0.225 | 0.226 |
| ACELS_45 | alpha-Ketoglutarate | | 0.362 | 0.020 | 0.274 |
| ACELS_45 | Histidine | | 0.000 | -0.157 | 0.110 |
| ACELS_45 | (Cer d18:1,C16:0 plus Cer d18:1,C18:0) ratio to (Cer d18:1,C24:0) | | 0.426 | 0.685 | 0.321 |
| ACELS_45 | Lysophosphatidylethanolamine (C18:2) | | -0.050 | -0.237 | 0.180 |
| ACELS_9 | CA19_9 | | 0.840 | 1.871 | 0.921 |
| ACELS_9 | NA | 2.186 | NA | NA | NA |
| ACELS_9 | Propionylcarnitine | | -0.595 | -0.225 | 0.226 |
| ACELS_9 | alpha-Ketoglutarate | | 0.319 | 0.020 | 0.274 |
| ACELS_9 | Proline | | -0.108 | -0.152 | 0.158 |
| ACELS_9 | (Cer d18:1,C16:0) ratio to (Cer d18:1,C24:0) | | 0.321 | 0.268 | 0.284 |
| ACELS_9 | Lysophosphatidylethanolamine (C18:2) | | -0.046 | -0.237 | 0.180 |
| ACES_13 | CA19_9 | | 0.633 | 1.871 | 0.921 |
| ACES_13 | NA | 1.891 | NA | NA | NA |
| ACES_13 | Propionylcarnitine | | -0.464 | -0.225 | 0.226 |
| ACES_13 | alpha-Ketoglutarate | | 0.199 | 0.020 | 0.274 |
| ACES_13 | Histidine | | 0.000 | -0.157 | 0.110 |
| ACES_13 | (Cer d18:1,C16:0) ratio to (Cer d18:1,C24:0) | | 0.196 | 0.268 | 0.284 |
| ACES_25 | CA19_9 | | 0.624 | 1.871 | 0.921 |
| ACES_25 | NA | 1.905 | NA | NA | NA |
| ACES_25 | Propionylcarnitine | | -0.431 | -0.225 | 0.226 |
| ACES_25 | alpha-Ketoglutarate | | 0.166 | 0.020 | 0.274 |
| ACES_25 | Proline | | -0.060 | -0.152 | 0.158 |
| ACES_25 | (Cer d18:1,C18:0) ratio to (Cer d18:1,C24:0) | | 0.260 | 0.469 | 0.349 |
| ACES_29 | CA19_9 | | 0.623 | 1.871 | 0.921 |
| ACES_29 | NA | 1.902 | NA | NA | NA |
| ACES_29 | Propionylcarnitine | | -0.455 | -0.225 | 0.226 |

(continued)

| Panel designation | metabolite/feature | bias | coefficient | scaling factors | |
|---|---|---|---|---|---|
| | | $\omega_0$ | $\omega_i$ | $m_i$ | $s_i$ |
| ACES_29 | alpha-Ketoglutarate | | 0.173 | 0.020 | 0.274 |
| ACES_29 | Histidine | | 0.000 | -0.157 | 0.110 |
| ACES_29 | (Cer d18:1,C18:0) ratio to (Cer d18:1,C24:0) | | 0.263 | 0.469 | 0.349 |
| ACES_41 | CA19_9 | | 0.628 | 1.871 | 0.921 |
| ACES_41 | NA | 1.902 | NA | NA | NA |
| ACES_41 | Propionylcarnitine | | -0.432 | -0.225 | 0.226 |
| ACES_41 | alpha-Ketoglutarate | | 0.173 | 0.020 | 0.274 |
| ACES_41 | Proline | | -0.064 | -0.152 | 0.158 |
| ACES_41 | (Cer d18:1,C16:0 plus Cer d18:1,C18:0) ratio to (Cer d18:1,C24:0) | | 0.241 | 0.685 | 0.321 |
| ACES_45 | CA19_9 | | 0.626 | 1.871 | 0.921 |
| ACES_45 | NA | 1.899 | NA | NA | NA |
| ACES_45 | Propionylcarnitine | | -0.458 | -0.225 | 0.226 |
| ACES_45 | alpha-Ketoglutarate | | 0.181 | 0.020 | 0.274 |
| ACES_45 | Histidine | | 0.000 | -0.157 | 0.110 |
| ACES_45 | (Cer d18:1,C16:0 plus Cer d18:1,C18:0) ratio to (Cer d18:1,C24:0) | | 0.243 | 0.685 | 0.321 |
| ACES_61 | CA19_9 | | 0.627 | 1.871 | 0.921 |
| ACES_61 | NA | 1.900 | NA | NA | NA |
| ACES_61 | Propionylcarnitine | | -0.445 | -0.225 | 0.226 |
| ACES_61 | alpha-Ketoglutarate | | 0.194 | 0.020 | 0.274 |
| ACES_61 | Histidine | | 0.000 | -0.157 | 0.110 |
| ACES_61 | (SM d17:1,C18:0) ratio to (SM d 17:1,C24:0) | | 0.262 | 0.255 | 0.186 |
| ACES_9 | CA19_9 | | 0.635 | 1.871 | 0.921 |
| ACES_9 | NA | 1.895 | NA | NA | NA |
| ACES_9 | Propionylcarnitine | | -0.436 | -0.225 | 0.226 |
| ACES_9 | alpha-Ketoglutarate | | 0.190 | 0.020 | 0.274 |
| ACES_9 | Proline | | -0.068 | -0.152 | 0.158 |
| ACES_9 | (Cer d18:1,C16:0) ratio to (Cer d18:1,C24:0) | | 0.195 | 0.268 | 0.284 |
| CEL_2 | CA19_9 | | 0.864 | 1.871 | 0.921 |
| CEL_2 | NA | 2.128 | NA | NA | NA |
| CEL_2 | Propionylcarnitine | | -0.657 | -0.225 | 0.226 |
| CEL_2 | alpha-Ketoglutarate | | 0.397 | 0.020 | 0.274 |
| CEL_2 | Lysophosphatidylethanolamine (C18:2) | | -0.139 | -0.237 | 0.180 |
| CELS_10 | CA19_9 | | 0.622 | 1.871 | 0.921 |
| CELS_10 | NA | 1.903 | NA | NA | NA |
| CELS_10 | Propionylcarnitine | | -0.454 | -0.225 | 0.226 |

(continued)

| Panel designation | metabolite/feature | bias | coefficient | scaling factors | |
|---|---|---|---|---|---|
| | | $\omega_0$ | $\omega_i$ | $m_i$ | $s_i$ |
| CELS_10 | alpha-Ketoglutarate | | 0.171 | 0.020 | 0.274 |
| CELS_10 | (Cer d18:1,C18:0) ratio to (Cer d18:1,C24:0) | | 0.259 | 0.469 | 0.349 |
| CELS_10 | Lysophosphatidylethanolamine (C18:2) | | -0.012 | -0.237 | 0.180 |
| CELS_18 | CA19_9 | | 0.626 | 1.871 | 0.921 |
| CELS_18 | NA | 1.900 | NA | NA | NA |
| CELS_18 | Propionylcarnitine | | -0.455 | -0.225 | 0.226 |
| CELS_18 | alpha-Ketoglutarate | | 0.177 | 0.020 | 0.274 |
| CELS_18 | (Cer d18:1,C16:0 plus Cer d18:1,C18:0) ratio to (Cer d18:1,C24:0) | | 0.238 | 0.685 | 0.321 |
| CELS_18 | Lysophosphatidylethanolamine (C18:2) | | -0.019 | -0.237 | 0.180 |
| CELS_2 | CA19_9 | | 0.632 | 1.871 | 0.921 |
| CELS_2 | NA | 1.893 | NA | NA | NA |
| CELS_2 | Propionylcarnitine | | -0.459 | -0.225 | 0.226 |
| CELS_2 | alpha-Ketoglutarate | | 0.192 | 0.020 | 0.274 |
| CELS_2 | (Cer d18:1,C16:0) ratio to (Cer d18:1,C24:0) | | 0.186 | 0.268 | 0.284 |
| CELS_2 | Lysophosphatidylethanolamine (C18:2) | | -0.034 | -0.237 | 0.180 |
| CES_10 | CA19_9 | | 0.850 | 1.871 | 0.921 |
| CES_10 | NA | 2.244 | NA | NA | NA |
| CES_10 | Propionylcarnitine | | -0.675 | -0.225 | 0.226 |
| CES_10 | alpha-Ketoglutarate | | 0.338 | 0.020 | 0.274 |
| CES_10 | (Cer d18:1,C18:0) ratio to (Cer d18:1,C24:0) | | 0.437 | 0.469 | 0.349 |
| CES_18 | CA19_9 | | 0.824 | 1.871 | 0.921 |
| CES_18 | NA | 2.188 | NA | NA | NA |
| CES_18 | Propionylcarnitine | | -0.647 | -0.225 | 0.226 |
| CES_18 | alpha-Ketoglutarate | | 0.324 | 0.020 | 0.274 |
| CES_18 | (Cer d18:1,C16:0 plus Cer d18:1,C18:0) ratio to (Cer d18:1,C24:0) | | 0.391 | 0.685 | 0.321 |
| CES_18 | CA19_9 | | 0.824 | 1.871 | 0.921 |
| CES_18 | NA | 2.188 | NA | NA | NA |
| CES_18 | Propionylcarnitine | | -0.647 | -0.225 | 0.226 |
| CES_18 | alpha-Ketoglutarate | | 0.324 | 0.020 | 0.274 |
| CES_18 | (Cer d18:1,C16:0 plus Cer d18:1,C18:0) ratio to (Cer d18:1,C24:0) | | 0.391 | 0.685 | 0.321 |
| CES_2 | CA19_9 | | 0.836 | 1.871 | 0.921 |
| CES_2 | NA | 2.174 | NA | NA | NA |
| CES_2 | Propionylcarnitine | | -0.653 | -0.225 | 0.226 |
| CES_2 | alpha-Ketoglutarate | | 0.347 | 0.020 | 0.274 |
| CES_2 | (Cer d18:1,C16:0) ratio to (Cer d18:1,C24:0) | | 0.330 | 0.268 | 0.284 |

(continued)

| Panel designation | metabolite/feature | bias | coefficient | scaling factors | |
|---|---|---|---|---|---|
| | | $\omega_0$ | $\omega_i$ | $m_i$ | $s_i$ |
| CES_26 | CA19_9 | | 0.830 | 1.871 | 0.921 |
| CES_26 | NA | 2.175 | NA | NA | NA |
| CES_26 | Propionylcarnitine | | -0.629 | -0.225 | 0.226 |
| CES_26 | alpha-Ketoglutarate | | 0.345 | 0.020 | 0.274 |
| CES_26 | (SM d17:1,C18:0) ratio to (SM d 17:1,C24:0) | | 0.380 | 0.255 | 0.186 |
| CES_34 | CA19_9 | | 0.842 | 1.871 | 0.921 |
| CES_34 | NA | 2.134 | NA | NA | NA |
| CES_34 | Propionylcarnitine | | -0.648 | -0.225 | 0.226 |
| CES_34 | alpha-Ketoglutarate | | 0.392 | 0.020 | 0.274 |
| CES_34 | (SM d18:2,C18:0) ratio to (SM d18:2,C24:0) | | 0.218 | 0.220 | 0.169 |
| CES_42 | CA19_9 | | 0.847 | 1.871 | 0.921 |
| CES_42 | NA | 2.154 | NA | NA | NA |
| CES_42 | Propionylcarnitine | | -0.638 | -0.225 | 0.226 |
| CES_42 | alpha-Ketoglutarate | | 0.365 | 0.020 | 0.274 |
| CES_42 | (SM d16:1,C18:0) ratio to (SM d16:1,C24:0) | | 0.273 | 0.289 | 0.180 |

The term "sample" as used herein refers to a sample of a body fluid, preferably, blood, plasma, serum, saliva or urine, or a sample derived by lavage from tissues or organs, in particular from the bile duct. More preferably, the sample is a blood, plasma, serum or urine sample. Even more preferably, the sample is a blood or plasma sample or is a serum or plasma sample, most preferably, a plasma sample. Preferably, if the sample is a blood sample, the method of the present invention comprises a further step of obtaining a serum or plasma sample from said blood sample. Preferably, the sample is a citrate plasma sample, a heparin plasma sample, or an EDTA plasma sample. More preferably, the sample is an EDTA plasma sample. Techniques for obtaining the aforementioned different types of biological samples are well known in the art. For example, blood samples may be obtained by blood taking while tissue or organ samples are to be obtained, e.g., by biopsy. Preferably, the sample is a fasting sample, in particular a fasting blood, plasma or serum sample. Thus, preferably, the sample is obtained from a fasting subject. A fasting subject, in particular, is a subject who refrained from food and beverages, except for water, prior to obtaining the sample to be tested. Preferably, a fasting subject refrained from food and beverages, except for water, for at least eight hours prior to obtaining the sample to be tested. More preferably, the sample has been obtained from the subject after an overnight fast. Preferably said fasting continued up to at least one hour before sample taking, more preferably up to at least 30 min before sample taking, still more preferable up to at least 15 min before sample taking, most preferably until the sample was taken. The aforementioned samples are, preferably, pre-treated before they are used for the method of the present invention, preferably as specified herein above.

In a preferred embodiment, at least one internal standard compound can be added to the sample. As used herein, the term "internal standard compound" refers to a compound which is added to the sample and which is determined (i.e. the amount of the internal standard compound is determined). The at least one internal standard compound can be added before, during, or after extraction of the sample to be tested. In an embodiment, the at least one internal standard compound is added before mixing an aliquot of the sample with the extraction solvent. Preferably, the at least one internal standard compound is dissolved separately in a suitable solvent before addition to the sample and, subsequent addition of the extraction solvent. In an embodiment, the extraction solvent is an extraction solvent as described elsewhere herein, such as methanol/dichloromethane (2:1 v/v). The determination of the amount of the internal standard shall preferably allow for a normalization of the amounts of at least one biomarker as referred to herein. Preferably, the determined peak areas for the diagnostic biomarkers are divided by the peak area of the at least one internal standard compound. In particular, it is possible to calculate a correction factor for the test samples (samples from subjects to be tested, but also calibration samples). This correction factor can be used in order to correct the peak area of the at least three biomarkers for variations of the devices, inherent system errors, or the like. For each biomarker determined as peak area, the area

EP 3 502 699 A1

ratio of the biomarker peak area to the internal standard peak area can be determined. Preferably, the determination of the at least one standard compound does not interfere with the determination of the amounts of the diagnostic biomarkers. In a preferred embodiment, the internal standard solution is a solution not comprising sample, more preferably is a solution consisting of standard compound(s) and solvent(s) as specified herein above. Thus, in a preferred embodiment, the present invention relates to an internal standard solution as specified herein above.

The term "subject", as used herein, relates to an animal, preferably, to a mammal. More preferably, the subject is a primate and, most preferably, a human. Preferably, the subject is an apparently healthy subject. Preferably, the subject is a subject at risk of suffering from pancreatic cancer. Risk factors for developing pancreatic cancer are known in the art, e.g. from Brand et al., Gut. 2007;56:1460-9; or Del Chiaro et al., World J Gastroenterol 2014; 20:12118-12131 and include new-onset diabetes, genetic factors, chronic disease, and age; more preferably, the risk factor is new-onset diabetes. Thus, preferably, the subject at risk of suffering from pancreatic cancer is a subject having a genetic predisposition, preferably familiar pancreatic cancer, including Peutz-Jeghers Syndrome, BRCA1 positivity, or a genetic predisposition for developing pancreatitis. Also preferably, the subject at risk of suffering from pancreatic cancer is a subject at least 40 years old, most preferably, at least 50 years old. More preferably, the subject at risk of suffering from pancreatic cancer is a subject suffering from chronic pancreatitis. Most preferably, the subject at risk of suffering from pancreatic cancer is a subject with new-onset diabetes. Preferably, the subject is a subject in whom new-onset diabetes was diagnosed at most three years, more preferably at most two years, most preferably at most one year, before the method as specified herein is performed on a sample of said subject. Thus, the method preferably is a method for diagnosing pancreatic cancer in a subject suffering from new-onset diabetes.

Preferably, the subject is a subject suspected to suffer from pancreatic cancer. Suspicion that a subject may suffer from pancreatic cancer, preferably, arises from at least one clinical symptom known to the skilled person to be associated with pancreatic cancer. Thus, preferably, the subject suspected to suffer from pancreatic cancer, preferably, is a subject having at least one clinical symptom of pancreatic cancer, more preferably selected from the list consisting of abdominal pain, lower back pain, nausea, unexplained weight loss, vomiting, and in some cases, jaundice. Also preferably, the subject suspected to suffer from pancreatic cancer is a subject requiring differential diagnosis between pancreatic cancer and chronic pancreatitis, i.e., preferably, a subject suspected to suffer from pancreatic cancer is a subject suspected to suffer from pancreatic cancer or from chronic pancreatitis. Also preferably, the subject suspected to suffer from pancreatic cancer is a subject having an increased CA19-9 concentration in the blood as compared to a healthy control, preferably more than 37 U/mL, more preferably more than 500 U/mL, most preferably more than 1000 U/mL. Also preferably, the subject is a subject with a low CA19-9 value. Preferably, a low CA19-9 value is a blood CA19-9 value of less than 5 U/ml, preferably less than 2 U/mL, more preferably 0. As will be appreciated by the skilled person, Lewis a/b antigen negative subjects have low CA19-9 values (Tian et al., 1992 Annals of Surgery 215 350-355) or a CA19-9 value below the detection limit, preferably, a value of zero. Thus, preferably, the subject with a low CA19-9 value is a Lewis a/b antigen negative subject. In a preferred embodiment, the subject is a subject having an abdominal cystic lesion, preferably a subject diagnosed with an unclear abdominal expansive lesion. In another preferred embodiment, the subject is a subject having a pancreatic cystic lesion, preferably a subject diagnosed with an unclear pancreatic expansive lesion.

The term "new-onset diabetes" is known to the skilled person and relates to diabetes diagnosed in a subject not previously having been diagnosed with diabetes and, preferably, not having previously documented symptoms of diabetes. Preferably, diagnosis of diabetes is according to WHO guidelines, more preferably includes diagnosing an 8-h fasting blood glucose value of >125 mg/dL.

The term "reference" in connection with diagnostic methods is well known in the art. The reference in accordance with the present invention shall allow for the diagnosis of pancreatic cancer. A suitable reference may be established by the skilled person without further ado. The term reference, preferably, refers to values of characteristic features which can be correlated to a medical condition, i.e. the presence or absence of the disease, diseases status or an effect referred to herein, or a calculated value or calculated values derived from said values. Preferably, the reference will be stored in a suitable data storage medium such as a database and are, thus, also available for future assessments. Preferably, the reference is a value determined and/or calculated from a subject or group of subjects known to suffer from pancreatic cancer or is a value determined and/or calculated from an apparently healthy subject or group thereof, i.e. a "reference amount". The reference to be applied may be an individual reference for each of the biomarkers to be determined in the method of the present invention. Accordingly, the amount of each of the biomarkers as referred to in step a) of the method of the present invention is compared to a reference amount for each of the biomarkers. For example, if four diagnostic biomarkers are determined in step a), four reference amounts (a reference amount for the first, a reference amount for the second, a reference amount for the third, and a reference amount for the fourth biomarker) are applied in step b). Based on the comparison of the amounts of the diagnostic biomarkers with the reference amounts, a diagnosis of pancreatic cancer, i.e. whether the subject as referred to herein suffers from pancreatic cancer, or not, is established. It is understood by the skilled person that a reference amount is not required to be an amount as determined in the determination step, but may also be a value derived therefrom by mathematical calculations well known to the skilled person. Preferably, a reference amount is a threshold value for a biomarker, preferably, a diagnostic biomarker, as referred to in connection with the present invention, whereby values found in a sample to be investigated which are higher than (or depending on the marker lower than) the threshold are indicative for the presence of pancreatic cancer while those being lower (or depending on the marker higher than) are indicative for the absence of pancreatic cancer.

The diagnostic algorithm, i.e. the specific set of calculation rules applied to obtain the diagnosis, may depend on the reference. If the reference amount is e.g. derived from a subject or group of subjects known to suffer from pancreatic cancer, the presence of pancreatic cancer is preferably indicated by amounts in the test sample which are essentially identical to the reference(s). If the reference amount is e.g. derived from an apparently healthy subject or group thereof, the presence of pancreatic cancer is preferably indicated by amounts of the diagnostic biomarkers in the test sample which are different from (e.g. increased ("up") or decreased ("down") as compared to the reference(s).

In accordance with the method, a reference amount (or reference amounts) is, preferably, a reference amount (or reference amounts) obtained from a sample from a subject or group of subjects known to suffer from pancreatic cancer. In such a case, a value for each of the diagnostic biomarkers found in the at least one test sample being essentially identical is indicative for the presence of the disease, i.e. of pancreatic cancer. Moreover, the reference amount, also preferably, could be from a subject or group of subjects known not to suffer from pancreatic cancer, preferably, an apparently healthy subject or group of subjects. In such a case, a value for each of the diagnostic biomarkers found in the test sample being altered with respect to the reference amount is indicative for the presence of the disease. Alternatively, a value for each of the diagnostic biomarkers found in the test sample being essentially identical with respect to the reference amount is indicative for the absence of the disease. The same applies mutatis mutandis for a calculated reference, most preferably the average or median, for the relative or absolute value of the diagnostic biomarkers in a population of individuals comprising the subject to be investigated. The absolute or relative values of the biomarkers of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

The value for a biomarker of the test sample and the reference amounts are essentially identical if the values for the characteristic features and, in the case of quantitative determination, the intensity values, or values derived therefrom, for the biomarker and the reference are essentially identical. Essentially identical means that the difference between two values is, preferably, not significant and shall be characterized in that the values for the intensity are within at least the interval between 1st and 99th percentile, 5th and 95th percentile, 10th and 90th percentile, 20th and 80th percentile, 30th and 70th percentile, 40th and 60th percentile of the reference value, preferably, the 50th, 60th, 70th, 80th, 90th or 95th percentile of the reference value. Statistical test for determining whether two amounts or values are essentially identical are well known in the art and are also described elsewhere herein. An observed difference for two values, on the other hand, shall be statistically significant. A difference in the relative or absolute value is, preferably, significant outside of the interval between 45th and 55th percentile, 40th and 60th percentile, 30th and 70th percentile, 20th and 80th percentile, 10th and 90th percentile, 5th and 95th percentile, 1 st and 99th percentile of the reference value. In a preferred embodiment, the value for the characteristic feature can also be a calculated output such as score of a classification algorithm like "elastic net" as set forth elsewhere herein.

More preferably, the reference is derived from two subjects or groups of subjects known to differ in a property to be diagnosed, e.g. a subject or group of subjects known to suffer from pancreatic cancer and an apparently healthy subject or group thereof, e.g. as a reference score or as a cutoff value distinguishing between said two subjects or groups, as specified herein below. The skilled person understands that other subject groups which shall be distinguished can be used as well for establishing e.g. a cutoff value. Preferably, a group of subjects known to suffer from pancreatic cancer is distinguished from a group of subjects known to suffer from chronic pancreatitis; or a group of subjects known to suffer from pancreatic cancer is distinguished from non-pancreatic controls; or a group of subjects known to suffer from pancreatic cancer is distinguished from group of subjects known to suffer from chronic pancreatitis and/or non-pancreatic controls; or a group of subjects known to suffer from pancreatic cancer is distinguished from all non-cancer subjects, preferably including chronic pancreatitis patients, non-pancreatic controls, diabetes patients, and/or non-diabetic subjects; or a group of subjects known to suffer from pancreatic cancer is distinguished from diabetic subjects. Moreover, more than one cutoff value may be provided, e.g., two cutoff values may be determined, wherein the interval between the first and the second cutoff may define, preferably diagnosis of increased risk of suffering from a disease to be diagnosed, warranting, e.g., closer monitoring of the patient.

The term "comparing", preferably, refers to determining whether the determined values of the biomarkers or score are/is essentially identical to a reference or differs therefrom. Based on the comparison referred to above, a subject can be assessed to suffer from pancreatic cancer, or not. It is to be understood that the diagnostic algorithm may be adjusted to the reference or references to be applied. This is taken into account by the skilled person who can establish suitable reference values and/or diagnostic algorithms based on the diagnosis provided herein. The comparison is, preferably, assisted by automation. For example, a suitable computer program comprising algorithms for the comparison of two different data sets (e.g., data sets comprising the values of the characteristic feature(s)) may be used. Such computer programs and algorithms are well known in the art. Notwithstanding the above, a comparison can also be carried out manually.

Advantageously, it was found in the work underlying the present invention that the biomarker combinations provided herein allow for an improved diagnosis of pancreatic cancer, in particular in subjects with new onset diabetes.

The definitions made above apply mutatis mutandis to the following. Additional definitions and explanations made further below also apply for all embodiments described in this specification mutatis mutandis.

The present invention further relates to a method for identifying whether a subject is in need of a pancreatic cancer therapy comprising the steps of the method for diagnosing pancreatic cancer disclosed herein and the further step of identifying a subject in need of a pancreatic cancer therapy if said subject is diagnosed to suffer from pancreatic cancer.

The phrase "in need for a therapy of pancreatic cancer" as used herein means that the disease in the subject is in a status where therapeutic intervention is necessary or beneficial in order to ameliorate or treat pancreatic cancer or the symptoms associated therewith. Accordingly, the findings of the studies underlying the present invention do not only allow diagnosing pancreatic cancer in a subject but also allow for identifying subjects which should be treated by a pancreatic cancer therapy or whose pancreatic cancer therapy needs adjustment. Once the subject has been identified, the method may further include a step of making recommendations for a therapy of pancreatic cancer. Preferably, the method further comprises the step of recommending a therapeutic or patient health management measure for the subject based on whether the subject is diagnosed to suffer from pancreatic cancer. The term "recommending", as used herein, refers to making suggestions for therapeutic measures and/or patient health management measures which are specifically applicable to the patient. Recommending does, preferably, not encompass the actual application of the recommended therapeutic or patient health management measure.

The term "therapeutic or patient health management measure" as used herein refers to therapeutic measures aiming to cure or ameliorate pancreatic cancer or aiming at preventing progression of the said diseases as well as patient health management measures such as monitoring including selection of monitoring measures and monitoring frequency and hospitalization. Preferably, the said therapeutic or patient health management measure is selected from the group consisting of: surgery, administration of anti-cancer drugs, patient monitoring, active surveillance, and hospitalization. Suitable cancer therapies include surgery, low- and high-dose irradiation, and systemic chemotherapy, e.g., cytostatic drugs, alone, or in combination with other drugs. Preferred surgery-based therapies include resection of the pancreas or parts thereof, such as pancreaticoduodenectomy, tail pancreatectomy, total or partial pancreatectomy, and palliative bridging procedures. Drug-based therapies ("drug treatment"), preferably, include the administration of one or more drugs with anti-tumour properties including but not exclusive to platinum derivatives, e.g., oxaliplatin, fluoropyrimidines, pyrimidine analogues, Gemcitabine, antimetabolites, alkylating agents, anthracyclines, plant alkaloids, topoisomerase inhibitors, targeted antibodies and tyrosine kinase inhibitors. Particularly preferred drugs include but are not limited to gemcitabine alone or in combination with erlotinib and/or oxaliplatin. It will be understood that the method can also be applied to determine whether a subject will benefit from or is in need of a therapy against the aforementioned diseases. Such a method can be applied in therapeutic approaches like "active surveillance". In this approach, a subject suffering from, e.g., new-onset diabetes is subjected to a method for diagnosing pancreatic cancer as set forth above on a short regular basis in order to early detect pancreatic cancer. Only after pancreatic cancer becomes detectable, the subject will be treated by a suitable therapy, such as surgery or irradiation. Thus, "active surveillance" prevents the harmful side effects of a therapy in subjects which are not in an immediate need for a therapy. By avoiding the therapy at this stage, it will be understood that the harmful side effects of the therapy can be avoided as well.

In a more preferred embodiment of the method of the present invention, said method also comprises the step of applying the said therapeutic or patient health management measure as identified by the aforementioned method to the subject. Thus, the present invention also relates to a method of diagnosing and treating pancreatic cancer in a subject, said method comprising

a) obtaining a sample from a patient;
b) determining a panel of pancreatic cancer markers according to the method for diagnosing pancreatic cancer in the sample;
c) diagnosing the patient with pancreatic cancer depending on the determination of step b); and
d) administering a pancreatic cancer treatment to the diagnosed subject.

Furthermore, the present invention relates to a device for diagnosing pancreatic cancer in a sample of a subject comprising:

(a) an analyzing unit for the sample of the subject comprising a detector for the amounts of at least one biomarker each of the categories carnitines, amino acids, energy metabolites, and sphingolipids; said detector allowing for the determination of the amounts of the biomarkers of said groups of biomarkers in the sample; and operatively linked thereto,

(b) an evaluation unit comprising a data processing unit and a data base, said data base comprising a stored reference and said data processing unit having tangibly embedded an algorithm for carrying out a comparison of the amounts of the biomarkers of the group of biomarkers determined by the analyzing unit and the stored reference and for generating an output information based on which the diagnosis can be established.

A "device", as the term is used herein, comprises at least the aforementioned units. The units of the device are operatively linked to each other. How to link the means in an operating manner will depend on the type of units included into the device. For example, where the detector allows for automatic qualitative or quantitative determination of the biomarker, the data obtained by said automatically operating analyzing unit can be processed by, e.g., a computer program in order to facilitate the assessment in the evaluation unit. Preferably, the units are comprised by a single device in such a case. Preferably, the device includes an analyzing unit for the biomarkers and a computer or data processing device as an evaluation unit for processing the resulting data for the assessment and for establishing the output information. Preferably, the analyzing unit comprises at least one detector for at least the biomarkers of a group according to the present invention, said at least one detector determining the amounts of said biomarkers in said sample. Preferred devices are those which can be applied without the particular knowledge of a specialized clinician, e.g., electronic devices which merely require loading with a sample. The output information of the device, preferably, is a numerical value which allows drawing conclusions on the quality of the sample and, thus, is an aid for the reliability of a diagnosis or for troubleshooting. Preferred references to be used in accordance with the device of the present invention are values for the biomarkers analyzed or values derived therefrom as specified above. Preferably, the device is a device for diagnosing pancreatic cancer. Preferably, the device further comprises, operatively linked to the evaluation unit, at least one input unit for inputting the amount of the marker CA 19-9 and/or an analyzing unit for the sample of the subject comprising a detector for the amount of CA 19-9 in said sample. Preferably, the device further comprises an input unit adapted to receive input data, preferably a value of an amount of CA19-9.

The units of the device, also preferably, can be implemented into a system comprising several devices which are operatively linked to each other. Depending on the units to be used for the system of the present invention, said means may be functionally linked by connecting each means with the other by means which allow data transport in between said means, e.g., glass fiber cables, and other cables for high throughput data transport. Nevertheless, wireless data transfer between the means is also envisaged by the present invention, e.g., via LAN (Wireless LAN, W-LAN). A preferred system comprises means for determining biomarkers. Means for determining biomarkers as used herein encompass means for separating biomarkers, such as chromatographic devices, and means for metabolite determination, such as mass spectrometry devices. Suitable devices have been described in detail above. Preferred means for compound separation to be used in the system of the present invention include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or LC-MS/MS are used in the system of the present invention as described in detail elsewhere in the specification. Further comprised shall be means for comparing and/or analyzing the results obtained from the means for determination of biomarkers. The means for comparing and/or analyzing the results may comprise at least one databases and an implemented computer program for comparison of the values measured with corresponding references. Preferred embodiments of the aforementioned systems and devices are also described in detail below. Furthermore, the present invention relates to a data collection comprising characteristic values of at least the markers of at least one panel disclosed herein, being indicative for a subject suffering from pancreatic cancer, or not.

The term "data collection" refers to a collection of data which may be physically and/or logically grouped together. Accordingly, the data collection may be implemented in a single data storage medium or in physically separated data storage media being operatively linked to each other. Preferably, the data collection is implemented by means of a database. Thus, a database as used herein comprises the data collection on a suitable storage medium. Moreover, the database, preferably, further comprises a database management system. The database management system is, preferably, a network-based, hierarchical or object-oriented database management system. Furthermore, the database may be a federal or integrated database. More preferably, the database will be implemented as a distributed (federal) system, e.g. as a Client-Server-System. More preferably, the database is structured as to allow a search algorithm to compare a test data set with the data sets comprised by the data collection. Specifically, by using such an algorithm, the database can be searched for similar or identical data sets being indicative for pancreatic cancer as set forth above (e.g. a query search). Thus, if an identical or similar data set can be identified in the data collection, the test data set will be associated with the presence of disease, or not. Consequently, the information obtained from the data collection can be used, e.g.,

as a reference for the methods of the present invention described above. More preferably, the data collection comprises characteristic values of all diagnostic biomarkers comprised by any one of the groups recited above.

In light of the foregoing, the present invention encompasses a data storage medium comprising the aforementioned data collection.

The term "data storage medium" as used herein encompasses data storage media which are based on single physical entities such as a CD, a CD-ROM, a hard disk, optical storage media, or a diskette. Moreover, the term further includes data storage media consisting of physically separated entities which are operatively linked to each other in a manner as to provide the aforementioned data collection, preferably, in a suitable way for a query search.

The present invention also relates to a use of a panel of biomarkers comprising at least one biomarker each of the categories carnitines, amino acids, energy metabolites, sphingolipids, and CA 19-9 or detection agents therefor in a sample of a subject suspected to suffer from pancreatic cancer for diagnosing pancreatic cancer.

The present invention further relates to a use of a group of biomarkers comprising at least one biomarker each of the categories carnitines, amino acids, energy metabolites, sphingolipids, and CA 19-9 or detection agents therefor for the manufacture of a compound or device for diagnosing pancreatic cancer.

All references cited in this specification are herewith incorporated by reference with respect to their entire disclosure content and the disclosure content specifically mentioned in this specification.

In view of the above, the following embodiments are preferred:

1. A method for diagnosing pancreatic cancer in a subject suspected to suffer from pancreatic cancer comprising the steps of:

(a) determining in a sample of said subject the value of (i) at least one biomarker of the categories carnitines, amino acids, and mitochondrial energy metabolites, and (ii) a first and a second sphingolipid, wherein the fatty acid residue of the first sphingolipid is at least four carbon atoms longer than the fatty acid residue of the second sphingolipid, and wherein the sphingosine backbone of the first sphingolipid and the second sphingolipid are identical; and

(b) comparing the values of the biomarkers with references, whereby pancreatic cancer is diagnosed.

2. The method of embodiment 1, wherein at least one biomarker each of at least two, preferably at least three, most preferably of all of the categories carnitines, amino acids, mitochondrial energy metabolites is determined.

3. The method of embodiment 1 or 2, wherein said subject is a subject at risk of suffering from pancreatic cancer, preferably wherein said subject is a subject suffering from new-onset diabetes.

4. The method of any one of embodiments 1 to 3, wherein said carnitine biomarker is selected from the list consisting of Propionyl-carnitine, Butyryl-carnitine, and Octenoyl-carnitine.

5. The method of any one of embodiments 1 to 4, wherein said amino acid biomarker is selected from Histidine and Proline.

6. The method of any one of embodiments 1 to 5, wherein said mitochondrial energy metabolite biomarker is selected from 2-Oxoglutarate and Coenzyme Q9.

7. The method of any one of embodiments 1 to 6, wherein said sphingolipid biomarker comprises a value of a sphingolipid comprising a short-chain fatty acid and/or a sphingolipid comprising a long-chain fatty acid, preferably wherein said sphingolipid biomarker is a ratio of a sphingolipid comprising a short-chain fatty acid to a sphingolipid comprising a long-chain fatty acid.

8. The method of any one of embodiments 1 to 7, wherein said first and second sphingolipid biomarker is selected from the list consisting of (i) Ceramide (Cer) d18:1,C16:0) and Cer (d18:1,C24:0); (ii) Cer (d18:1,C18:0) and Cer (d18:1,C24:0); (iii) (Cer (d18:1,C16:0) plus Cer d18:1,C18:0)) and Cer (d18:1,C24:0); (iv) Sphingomyelin (SM) (d16:1,C18:0) and SM (d16:1,C24:0); (v) SM (d17:1,C18:0) and SM (d17:1,C24:0); and (vi) SM (d18:2,C18:0) to SM (d18:2,C24:0) ratio.

9. The method of any one of embodiments 1 to 8, wherein a further lipid biomarker, preferably selected from total phospholipids to total glycerolipids ratio and Lysophosphatidyl-ethanolamine (C18:2), is determined.

10. The method of any one of embodiments 1 to 9, wherein said value of a biomarker is an amount of a biomarker or is a ratio of amounts of at least two biomarkers.

11. The method of any one of embodiments 1 to 10, wherein said method further comprises determining the amount of CA 19-9 in said sample or comprises providing a value of CA19-9 of said subject in step a) and comparing said value of CA19-9 to a reference in step b).

12. The method of any one of embodiments 1 to 11, wherein step a) comprises determining at least one marker panel from Table 1.

13. The method of any one of embodiments 1 to 12, wherein said references are derived from (i) a sample of a subject or from samples of a group of subjects known to suffer from pancreatic cancer and/or (ii) a sample of a subject or from samples of a group of subjects known not to suffer from pancreatic cancer or from a population of apparently healthy subjects.

14. The method of any one of embodiments 1 to 13, wherein the biomarkers determined in step a) comprise the biomarkers of at least one panel of Table 1.

15. The method of any one of embodiments 1 to 14, wherein the biomarkers determined in step a) comprise the biomarkers of a panel selected from panels ACES_1, ACES_25, ACELS_9, ACELS_97 of Table 1.

16. The method of any one of embodiments 1 to 15, wherein determining the value of a biomarker comprises determining the amount of said biomarker of said sample.

17. The method of any one of embodiments 1 to 16, wherein said determining the amount of said biomarker of said sample comprises mass spectrometry.

18. The method of any one of embodiments 1 to 17, wherein determining the value of a biomarker comprises determining the amount of said biomarker in said sample, scaling the $\log_{10}$-transformed amount of said biomarker x to a scaled amount $\hat{x}$ according to formula (III):

$$\hat{x} = \frac{x-m}{s} \quad \text{(III).}$$

with m = a biomarker-specific constant; and s = a biomarker-specific divisor.

19. The method embodiment 18, wherein step b) comprises calculating a prediction probability calculated according to the following formula (II)

$$p = \frac{1}{1+e^{-(\omega_0 + \Sigma_i^n \omega_i \hat{x}_i)}} \quad \text{(II),}$$

wherein

  p = prediction score;
  e = Euler's number;
  $\omega_0$ = bias value;
  $\omega_i$ = diagnostic biomarker-specific weight value for diagnostic biomarker i; and
  $\hat{x}_i$ = scaled amount for biomarker i.

20. The method of embodiment 19, wherein the values of parameters $m$, $s$, $\omega_i$, and $\omega_0$ are selected from Table 2.

21. A method for identifying whether a subject is in need of a pancreatic cancer therapy comprising the steps of the method of any one of embodiments 1 to 20 and the further step of identifying a subject in need of a pancreatic cancer therapy if said subject is diagnosed to suffer from pancreatic cancer.

22. The method of embodiment 21, wherein said pancreatic cancer therapy comprises surgery, radiotherapy, and/or drug treatment.

23. A method of diagnosing and treating pancreatic cancer in a subject, said method comprising

  a) obtaining a sample from a patient;
  b) determining a panel of pancreatic cancer markers according to the method according to any one of embodiments 1 to 20 in the sample;
  c) diagnosing the patient with pancreatic cancer depending on the determination of step b); and
  d) administering a pancreatic cancer treatment to the diagnosed subject.

24. The method of any one of embodiments 1 to 23, wherein said sample is a plasma, blood or se-um sample.

25. A device for diagnosing pancreatic cancer in a sample of a subject comprising:

  (a) an analyzing unit for the sample of the subject comprising a detector for the amounts of at least one biomarker each of the categories carnitines, amino acids, energy metabolites, and sphingolipids; said detector allowing for the determination of the amounts of the biomarkers of the said group of biomarkers in the sample; and operatively linked thereto,
  (b) an evaluation unit comprising a data processing unit and a data base, said data base comprising a stored reference and said data processing unit having tangibly embedded an algorithm for carrying out a comparison of the amounts of the biomarkers of the group of biomarkers determined by the analyzing unit and the stored reference and for generating an output information based on which the diagnosis can be established.

26. The device of embodiment 25, further comprising, operatively linked to the evaluation unit, at least one input unit for inputting the amount of the marker CA 19-9 and/or an analyzing unit for the sample of the subject comprising a detector for the amount of CA 19-9 in said sample.

27. A data collection comprising characteristic values of at least the markers of at least one panel according to any one of embodiments 1 to 20, being indicative for a subject suffering from pancreatic cancer, or not.

28. Use of a panel of biomarkers comprising at least one biomarker each of the categories carnitines, amino acids, energy metabolites, sphingolipids, and CA 19-9 or detection agents therefor in a sample of a subject suspected to suffer from pancreatic cancer for diagnosing pancreatic cancer.

29. Use of a group of biomarkers comprising at least one biomarker each of the categories carnitines, amino acids, energy metabolites, sphingolipids, and CA 19-9 or detection agents therefor for the manufacture of a compound or device for diagnosing pancreatic cancer.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

**Example 1:** Patient characteristics, plasma preparation

A total of 402 patients with pancreatic cancer or diabetic non-pancreatic controls (hernia repair and thyroid resections) were enrolled in the clinical study. In this retrospective case control study, samples of 361 patients suffering from pancreatic ductal adenocarcinoma (PDAC) (198 of them in a resectable tumor stage (IA-IIB)), samples of 41 diabetic non-pancreatic control patients matched for age, gender and BMI were included. The mean age of the pancreatic cancer patients was 67 years. The mean age of the diabetic non-pancreatic control patients was 67 years. Patients were overnight fasted and consecutively recruited from four clinical centers. Exclusion criteria were a concomitant malignant disease, curative treatment of malignant disease less than 2 years of recruitment to the trial, concomitant cystic diseases of the pancreas, pregnancy or patients unable to give informed consent. All patients or their legal representatives gave their written informed consent and the local ethics review boards approved the protocol. After blood drawing and centrifugation according to the blood draw tube manufacturer's instruction, EDTA plasma was collected in Eppendorf tubes and stored at -80°C for further analysis.

**Example 2:** Metabolite profiling

2.1 MxP® Broad Profiling

Two types of mass spectrometry analyses were applied to all samples. GC-MS (gas chromatography-mass spectrometry; Agilent 6890 GC coupled to an Agilent 5973 MSSystem, Agilent, Waldbronn, Germany) and LC-MS/MS [liquid chromatography-MS/MS; Agilent 1100 HPLC-System (Agilent, Waldbronn, Germany) coupled to an Applied Biosystems API4000 MS/MS-System (Applied Biosystems, Darmstadt, Germany)] were used for broad profiling [van Ravenzwaay, B. et al. The use of metabolomics for the discovery of new biomarkers of effect. Toxicol Lett 172, 21-8 (2007). Solid phase extraction-LC-MS/MS [SPE-LC-MS/MS; Symbiosis Pharma (Spark, Emmen, Netherlands) coupled to an Applied Biosystems API4000 MS/MS-System (Applied Biosystems, Darmstadt, Germany)] was used for the determination steroid levels. Fractionation and derivatisation of samples and detection technologies have been previously described [van Ravenzwaay, B. et al. The use of metabolomics for the discovery of new biomarkers of effect. Toxicol Lett 172, 21-8 (2007, Roessner, U., Wagner, C., Kopka, J., Trethewey, R.N. & Willmitzer, L. Technical advance: simultaneous analysis of metabolites in potato tuber by gas chromatography-mass spectrometry. Plant J 23, 131-42 (2000), Mutch, D.M. et al. Metabolite profiling identifies candidate markers reflecting the clinical adaptations associated with Roux-en-Y gastric bypass surgery. PLoS One 4, e7905 (2009)]. Proteins were removed from plasma samples by solvent precipitation by adding the triple amount of acetonitrile. Subsequently polar and non-polar fractions were separated for both GC-MS and LC-MS/MS analyses by adding water and a mixture of ethanol and dichloromethane. For GC-MS analyses, the non-polar fraction was treated with methanol under acidic conditions to yield the fatty acid methyl esters derived from both free fatty acids and hydrolyzed complex lipids. The polar and non-polar fractions were further derivatized with O-methyl-hydroxyamine hydrochloride (20 mg/ml in pyridine, 50 II) to convert oxo-groups to O-methyloximes and subsequently with a silylating agent (MSTFA, 50 II) before GC-MS analysis. For LC-MS/MS analyses, both fractions were reconstituted in appropriate solvent mixtures. High performance LC (HPLC) was performed by gradient elution using methanol/water/formic acid on reversed phase separation columns. Mass spectrometric detection technology was applied as described in the patent US 7,196,323, which allows targeted and high sensitivity "Multiple Reaction Monitoring" profiling in parallel to a full screen analysis.

2.2 MxP® Lipids

Total lipids were extracted from plasma by liquid/liquid extraction using chloroform/methanol. The lipid extracts were subsequently fractionated by normal phase liquid chromatography (NPLC) according to Christie. Rapid separation and quantification of lipid classes by high performance liquid chromatography and mass (light-scattering) detection. J Lipid Res 26, 507-12 (1985) into different lipid groups comprising sphingomyelins, ceramides, sterol esters, free fatty acids, phosphatidylcholines, phosphatidylethanolamines, phosphatidylserines, phosphatidylinositols, lysophosphatidylcholines, triacylglycerides, diacylglycerides and monoacylglycerides. The sphingomyelins and ceramides fractions were analyzed by LC-MS/MS using electrospray ionization (ESI) and atmospheric pressure chemical ionization (APCI) with detection of specific multiple reaction monitoring (MRM) transitions for sphingomyelins (SM) and ceramides (CER) respectively.

Carbohydrate antigen 19-9 (CA 19-9) was analyzed in blood plasma or serum by a radioimmunoassay (RIA) in clinical chemistry laboratories. The normal range of CA 19-9 in the blood of a healthy individual is 0-37 U/mL (Units per milliliter).

**Example 3:** Data analysis, normalization and statistical evaluation

Metabolite profiling based on a semi-quantitative analytical platform results in relative metabolite levels ("ratio") to a defined reference. To support this concept and to allow an alignment of different analytical batches, two different reference sample types were run in parallel throughout the whole process. First, a project pool was generated from aliquots of all samples and measured with four replicates within each analytical sequence that comprised 24 samples. For all semi-quantitatively analyzed metabolites, the results of each analyte from each sample were normalized against the median of the corresponding analyte in the pool reference samples within each analytical sequence to provide pool-normalized ratios. This process step compensated for inter- and intra-instrumental variation, i.e. variability that occurs when different analytical sequences are analyzed by different devices. This corresponds to a single-point calibration using multiple aliquots of the same level. As the calibration samples are of the same matrix as the project samples, they account best for all sequence to sequence deviations that may occur within sample preparation and measurement. Furthermore, metabolite levels of the pooled sample are representative for metabolite levels of the study, leading to most accurate semi-quantitative values. Moreover, the slope of each metabolite signal, including unknown analytes, relative to sample amount has been determined during method development. Only metabolites with a slope justifying a single-point calibration approach were taken into account for the presented study.

Second, to allow for an experiment-to-experiment alignment of semi-quantitative data, MxPool™ (a large pool of a commercial human EDTA plasma suited for alignment of MxP® studies) was analyzed with 12 replicated samples in the identification and validation studies, but not in the exploratory study and the pool-normalized ratios were further normalized to the median of the MxPool™ samples, i.e. ratios from this study are on the same level and therefore comparable with data from other studies normalized to other aliquots of the same MxPool™.

Prior to statistical analysis, $\log_{10}$ transformation of ratios was conducted to assure normal distribution of data. The software R 2.8.1 (package nlme) was used for data analyses and visualizations. Statistical analysis was done by a simple linear model (ANOVA) with "disease", "age", "gender", "BMI", and "center", if appropriate, as fixed effects.

Classification using the Elastic Net algorithm (Zou and Hastie (2005) Regularization and variable selection via the elastic net, Journal of the Royal Statistical Society, Series B: 67, 301-320) as implemented in the R (version 3.0.1) package glmnet (version 1.9-8) was calculated to obtain a logistic regression model on $\log_{10}$ transformed data including CA 19-9. The L1 and the L2 penalties were given equal weight. The log-transformed biomarker data including CA19-9 were also centered and scaled to unit variance before the analysis. This logistic regression models allows the calculation of predicted probabilities for each of the patients having pancreatic cancer.

To analyze the performance of our selected panels, a classifier was built with an elastic net analysis with these sets of metabolites and the cross validated classification performance was estimated with the area under the curve (AUC) of a receiver operating characteristic (ROC) analysis.

A10-fold cross-validation was used to obtain an unbiased estimate of the area under the curve (AUC) on the remaining fold. The 95% confidence intervals for the AUC were calculated using the binormal model of the receiver operating characteristic (ROC) curve as described in Zhou, Obuchowski and McClish (Statistical Methods in Diagnostic Medicine (2011), 2nd Edition, by Zhou, Obuchowski and McClish). The assumption of the binormality of the logit-transformed prediction scores was visually checked with a QQ-Plot. Afterwards the final model coefficients were determined by retraining the classifier on the entire data.

The ANOVA results and AUC of univariate ROC analysis of all small molecule biomarkers that were subsequently used for biomarker panel definition are given in the Table 3, below.

Table 3: ANOVA results and AUC of univariate ROC analysis of all small molecule biomarkers. Up-regulations are indicated by t-values >0, downregulations are indicated by t-values <0.

| Metabolite | Pancreatic cancer relative to diabetes | | | | Resectable pancreatic cancer relative to diabetes | | | |
|---|---|---|---|---|---|---|---|---|
| | fold change | p-value | t-value | AUC | fold change | p-value | t-value | AUC |
| (Cer d18:1,C18:0) ratio to (Cer d18:1,C24:0) | 2.07 | 1.38E-12 | 7.18 | 0.81 | 2.13 | 1.47E-12 | 7.18 | 0.80 |
| Lysophosphatidylcholine (C18:2) | 0.65 | 5.74E-12 | -6.98 | 0.78 | 0.65 | 1.90E-11 | -6.80 | 0.78 |
| (Cer d18:1,C16:0 plus Cer | 1.90 | 1.14E-11 | 6.87 | 0.81 | 1.95 | 1.20E-11 | 6.87 | 0.80 |

| | | | | | | | | |
|---|---|---|---|---|---|---|---|---|
| d18:1,C18:0) ratio to (Cer d18:1,C24:0) | | | | | | | | |
| (SM d17:1,C18:0) ratio to (SM d17:1,C24:0) | 1.50 | 6.58E-11 | 6.61 | 0.77 | 1.51 | 1.04E-10 | 6.54 | 0.77 |
| (SM d16:1,C18:0) ratio to (SM d16:1,C24:0) | 1.45 | 6.21E-10 | 6.25 | 0.75 | 1.47 | 8.99E-10 | 6.19 | 0.75 |
| Coenzyme Q9 | 0.48 | 6.26E-10 | -6.25 | 0.78 | 0.47 | 2.10E-09 | -6.05 | 0.78 |
| Propionylcarnitine | 0.64 | 6.46E-10 | -6.24 | 0.81 | 0.62 | 6.82E-10 | -6.24 | 0.81 |
| 3-Hydroxybutyrate | 3.71 | 1.65E-09 | 6.09 | 0.77 | 3.48 | 3.53E-08 | 5.56 | 0.75 |
| Taurocholic acid | 7.73 | 2.52E-09 | 6.02 | 0.73 | 10.35 | 5.53E-11 | 6.63 | 0.75 |
| (Cer d18:1,C16:0) ratio to (Cer d18:1,C24:0) | 1.67 | 3.22E-09 | 5.98 | 0.77 | 1.70 | 3.75E-09 | 5.95 | 0.76 |
| Alanine | 0.75 | 5.33E-09 | -5.89 | 0.80 | 0.74 | 7.38E-09 | -5.84 | 0.81 |
| Ceramide (d16:1,C24:0) | 0.58 | 7.27E-09 | -5.84 | 0.78 | 0.59 | 1.35E-07 | -5.31 | 0.77 |
| (SM d18:2,C18:0) ratio to (SM d18:2,C24:0) | 1.38 | 2.02E-08 | 5.66 | 0.72 | 1.41 | 1.35E-08 | 5.73 | 0.73 |
| Ceramide (d18:1,C18:0) | 1.62 | 3.56E-08 | 5.56 | 0.71 | 1.66 | 3.22E-08 | 5.58 | 0.70 |
| alpha-Ketoglutarate | 1.55 | 5.17E-08 | 5.49 | 0.71 | 1.67 | 5.70E-10 | 6.27 | 0.73 |
| Cryptoxanthin | 0.43 | 6.20E-08 | -5.46 | 0.77 | 0.41 | 2.35E-08 | -5.63 | 0.78 |
| Sphingomyelin (d16:1,C24:0) | 0.66 | 6.29E-08 | -5.45 | 0.77 | 0.66 | 2.46E-07 | -5.20 | 0.77 |
| Ceramide (d16:1,C23:0) | 0.59 | 2.76E-07 | -5.18 | 0.76 | 0.62 | 8.48E-06 | -4.48 | 0.75 |
| Citrulline | 0.76 | 3.95E-07 | -5.11 | 0.70 | 0.76 | 1.20E-06 | -4.89 | 0.69 |
| Lysophosphatidylethanolamine (C18:2) | 0.73 | 4.65E-07 | -5.08 | 0.70 | 0.72 | 3.52E-07 | -5.13 | 0.70 |
| Sphingomyelin (d16:1,C23:0) | 0.64 | 6.35E-07 | -5.01 | 0.76 | 0.66 | 1.11E-05 | -4.42 | 0.74 |
| Sphingomyelin (d18:1,C20:1) | 1.39 | 8.08E-07 | 4.97 | 0.71 | 1.41 | 9.13E-07 | 4.94 | 0.69 |
| Glycocholic acid | 4.20 | 9.51E-07 | 4.93 | 0.69 | 5.27 | 4.39E-08 | 5.52 | 0.70 |
| Mannose | 1.34 | 1.28E-06 | 4.88 | 0.69 | 1.34 | 4.10E-06 | 4.63 | 0.69 |
| Ceramide (d18:2,C26:0) | 0.60 | 1.79E-06 | -4.83 | 0.74 | 0.60 | 6.10E-06 | -4.57 | 0.73 |
| Lysophosphatidylcholine (C18:0) | 0.77 | 2.34E-06 | -4.75 | 0.69 | 0.78 | 1.40E-05 | -4.37 | 0.68 |
| 2-Hydroxybutyrate | 1.48 | 8.02E-06 | 4.49 | 0.66 | 1.39 | 2.82E-04 | 3.65 | 0.64 |
| Sphingomyelin (d18:2,C22:1) | 1.30 | 1.12E-05 | 4.42 | 0.66 | 1.32 | 6.71E-06 | 4.53 | 0.66 |
| Ceramide (d19:1,C18:0) | 1.91 | 1.17E-05 | 4.42 | 0.74 | 1.87 | 5.53E-05 | 4.06 | 0.72 |
| Ceramide (d18:1,C22:1) | 1.55 | 1.25E-05 | 4.39 | 0.68 | 1.56 | 2.27E-05 | 4.26 | 0.65 |
| Taurochenodeoxycholic acid | 3.44 | 1.26E-05 | 4.39 | 0.67 | 4.62 | 1.76E-07 | 5.26 | 0.71 |
| Ceramide (d18:1,C24:1) | 1.27 | 1.52E-05 | 4.35 | 0.67 | 1.25 | 7.37E-05 | 3.98 | 0.65 |
| Sphingomyelin (d18:1,C24:1) | 1.21 | 1.70E-05 | 4.32 | 0.69 | 1.20 | 9.27E-05 | 3.93 | 0.67 |
| 3-Indoxylsulfate | 0.49 | 1.81E-05 | -4.31 | 0.72 | 0.48 | 2.36E-05 | -4.25 | 0.74 |
| Sphingomyelin (d18:1,C22:1) | 1.36 | 1.82E-05 | 4.31 | 0.68 | 1.37 | 2.35E-05 | 4.25 | 0.66 |
| beta-Carotene | 0.52 | 1.88E-05 | -4.30 | 0.69 | 0.52 | 3.63E-05 | -4.15 | 0.69 |
| Ceramide (d18:1,C25:0) | 0.63 | 1.93E-05 | -4.31 | 0.78 | 0.64 | 6.07E-05 | -4.04 | 0.78 |
| Lutein | 0.67 | 2.17E-05 | -4.27 | 0.70 | 0.63 | 4.03E-06 | -4.64 | 0.73 |
| Lycopene | 0.48 | 2.29E-05 | -4.26 | 0.73 | 0.51 | 1.83E-04 | -3.76 | 0.71 |

| | | | | | | | | |
|---|---|---|---|---|---|---|---|---|
| Proline | 0.80 | 2.34E-05 | -4.25 | 0.74 | 0.80 | 7.48E-05 | -3.98 | 0.74 |
| Tryptophan | 0.79 | 2.68E-05 | -4.22 | 0.74 | 0.79 | 3.22E-05 | -4.18 | 0.74 |
| Ceramide (d18:2,C24:0) | 0.72 | 2.74E-05 | -4.22 | 0.71 | 0.72 | 3.71E-05 | -4.14 | 0.71 |
| Ceramide (d18:2,C18:0) | 1.37 | 2.84E-05 | 4.21 | 0.62 | 1.40 | 1.76E-05 | 4.32 | 0.63 |
| Lysophosphatidylcholine (C18:1) | 0.91 | 4.18E-05 | -4.12 | 0.66 | 0.90 | 5.80E-05 | -4.04 | 0.66 |
| Glycerol, polar fraction | 1.35 | 4.27E-05 | 4.11 | 0.67 | 1.35 | 7.54E-05 | 3.98 | 0.67 |
| Sphingomyelin (d16:1,C22:0) | 0.73 | 4.49E-05 | -4.10 | 0.73 | 0.75 | 3.57E-04 | -3.58 | 0.71 |
| Sphingomyelin (d19:1,C18:0) | 1.63 | 6.86E-05 | 4.01 | 0.71 | 1.59 | 3.26E-04 | 3.62 | 0.71 |
| Ceramide (d18:1,C16:0) | 1.31 | 7.11E-05 | 3.99 | 0.65 | 1.32 | 6.95E-05 | 4.00 | 0.63 |
| Ceramide (d18:0,C18:0) | 1.87 | 8.10E-05 | 3.97 | 0.67 | 1.97 | 4.83E-05 | 4.10 | 0.67 |
| Creatine | 0.77 | 8.21E-05 | -3.96 | 0.68 | 0.75 | 5.56E-05 | -4.05 | 0.68 |
| Biliverdin | 1.74 | 9.01E-05 | 3.93 | 0.66 | 1.88 | 1.62E-05 | 4.34 | 0.68 |
| Ceramide (d18:1,C24:0) | 0.78 | 9.22E-05 | -3.93 | 0.71 | 0.78 | 1.10E-04 | -3.88 | 0.72 |
| Ceramide (d17:1,C23:0) | 0.70 | 1.00E-04 | -3.91 | 0.72 | 0.72 | 6.87E-04 | -3.41 | 0.70 |
| Ceramide (d18:2,C23:0) | 0.72 | 1.01E-04 | -3.91 | 0.71 | 0.73 | 3.28E-04 | -3.61 | 0.70 |
| gamma-Linolenic acid (C18:cis[6,9,12]3) | 0.63 | 1.17E-04 | -3.87 | 0.72 | 0.69 | 3.44E-03 | -2.93 | 0.69 |
| Ceramide (d17:1,C24:0) | 0.72 | 1.20E-04 | -3.86 | 0.71 | 0.73 | 3.49E-04 | -3.59 | 0.70 |
| Sphingomyelin (d18:1,C18:1) | 1.24 | 1.22E-04 | 3.86 | 0.66 | 1.24 | 3.23E-04 | 3.61 | 0.64 |
| Ceramide (d18:1,C23:1) | 1.38 | 1.35E-04 | 3.83 | 0.62 | 1.40 | 1.06E-04 | 3.89 | 0.61 |
| Ceramide (d18:1,C20:1) | 1.95 | 1.41E-04 | 3.83 | 0.71 | 1.90 | 4.42E-04 | 3.54 | 0.71 |
| Indole-3-lactic acid | 0.82 | 1.60E-04 | -3.79 | 0.72 | 0.81 | 1.68E-04 | -3.78 | 0.72 |
| Sphingomyelin (d20:2,C24:1) | 1.43 | 1.66E-04 | 3.79 | 0.74 | 1.39 | 1.10E-03 | 3.28 | 0.71 |
| Xylose | 0.70 | 2.04E-04 | -3.73 | 0.69 | 0.72 | 1.26E-03 | -3.24 | 0.68 |
| Coenzyme Q10 | 0.74 | 2.11E-04 | -3.72 | 0.69 | 0.77 | 1.93E-03 | -3.11 | 0.67 |
| Lysophosphatidylethanolamine (C18:0) | 0.80 | 2.12E-04 | -3.72 | 0.67 | 0.81 | 6.56E-04 | -3.42 | 0.67 |
| Ceramide (d18:1,C20:0) | 1.29 | 2.46E-04 | 3.68 | 0.62 | 1.30 | 3.07E-04 | 3.62 | 0.60 |
| Ceramide (d18:1,C26:0) | 0.68 | 2.78E-04 | -3.66 | 0.73 | 0.68 | 5.30E-04 | -3.49 | 0.73 |
| Cholesterylester C18:2 | 0.78 | 3.25E-04 | -3.61 | 0.61 | 0.74 | 1.79E-05 | -4.31 | 0.64 |
| Ceramide (d17:1,C16:0) | 1.27 | 3.33E-04 | 3.60 | 0.63 | 1.30 | 1.59E-04 | 3.79 | 0.63 |
| Sphingomyelin (d17:1,C23:0) | 0.75 | 3.49E-04 | -3.59 | 0.69 | 0.75 | 7.21E-04 | -3.39 | 0.69 |
| Tricosanoic acid (C23:0) | 0.73 | 4.11E-04 | -3.55 | 0.66 | 0.78 | 8.12E-03 | -2.65 | 0.62 |
| Sphingomyelin (d18:1,C18:0) | 1.20 | 4.50E-04 | 3.52 | 0.62 | 1.19 | 1.17E-03 | 3.26 | 0.60 |
| Ceramide (d17:1,C18:0) | 1.46 | 4.89E-04 | 3.51 | 0.67 | 1.47 | 6.84E-04 | 3.42 | 0.66 |
| Ceramide (d18:1,C19:0) | 1.42 | 5.01E-04 | 3.50 | 0.70 | 1.44 | 5.35E-04 | 3.48 | 0.68 |
| Sphingomyelin (d18:2,C23:0) | 0.79 | 5.12E-04 | -3.49 | 0.68 | 0.79 | 7.48E-04 | -3.38 | 0.68 |
| Sphingomyelin (d18:2,C20:1) | 1.21 | 5.28E-04 | 3.48 | 0.63 | 1.24 | 2.45E-04 | 3.68 | 0.64 |
| Sphingomyelin (d19:1,C24:1) | 1.40 | 5.82E-04 | 3.46 | 0.70 | 1.38 | 1.69E-03 | 3.16 | 0.70 |
| Ceramide (d18:1,C24:2) | 1.33 | 6.02E-04 | 3.44 | 0.59 | 1.36 | 3.97E-04 | 3.55 | 0.60 |
| Lysophosphatidylethanolamine (C22:5) | 0.87 | 6.27E-04 | -3.43 | 0.61 | 0.84 | 1.11E-04 | -3.88 | 0.63 |
| Hypoxanthine | 1.39 | 6.76E-04 | 3.41 | 0.65 | 1.44 | 3.00E-04 | 3.63 | 0.67 |

| | | | | | | | | |
|---|---|---|---|---|---|---|---|---|
| Eicosapentaenoic acid (C20:cis[5,8,11,14,17]5) | 0.61 | 6.80E-04 | -3.42 | 0.72 | 0.65 | 4.65E-03 | -2.84 | 0.69 |
| Glucuronic acid | 2.18 | 6.91E-04 | 3.42 | 0.71 | 2.13 | 1.71E-03 | 3.16 | 0.67 |
| Cholesterol, free | 1.19 | 7.15E-04 | 3.40 | 0.63 | 1.22 | 1.30E-04 | 3.84 | 0.65 |
| Glutamate | 1.38 | 7.23E-04 | 3.39 | 0.62 | 1.46 | 1.57E-04 | 3.80 | 0.64 |
| Glutamine | 0.83 | 8.57E-04 | -3.34 | 0.67 | 0.79 | 7.91E-05 | -3.97 | 0.70 |
| Sphingomyelin (d18:1,C23:1) | 1.24 | 8.75E-04 | 3.34 | 0.63 | 1.28 | 2.84E-04 | 3.64 | 0.64 |
| Ceramide (d18:1,C23:0) | 0.80 | 9.34E-04 | -3.32 | 0.69 | 0.82 | 2.94E-03 | -2.98 | 0.68 |
| 3-Hydroxyindole | 0.59 | 1.04E-03 | -3.30 | 0.71 | 0.53 | 2.17E-04 | -3.73 | 0.76 |
| Paraxanthine | 0.58 | 1.11E-03 | -3.29 | 0.75 | 0.61 | 5.51E-03 | -2.79 | 0.73 |
| Phosphatidylcholine (C16:0,C16:0) | 1.10 | 1.13E-03 | 3.27 | 0.66 | 1.11 | 6.31E-04 | 3.43 | 0.66 |
| Threitol | 0.67 | 1.13E-03 | -3.27 | 0.70 | 0.68 | 3.27E-03 | -2.95 | 0.69 |
| Ceramide (d18:2,C16:0) | 1.22 | 1.14E-03 | 3.26 | 0.61 | 1.22 | 1.45E-03 | 3.19 | 0.60 |
| Catechol | 0.70 | 1.16E-03 | -3.27 | 0.72 | 0.70 | 1.86E-03 | -3.13 | 0.72 |
| Ribonic acid | 0.80 | 1.17E-03 | -3.26 | 0.69 | 0.83 | 1.22E-02 | -2.51 | 0.66 |
| 4-Hydroxyphenylpyruvate | 0.61 | 1.18E-03 | -3.25 | 0.74 | 0.52 | 3.74E-05 | -4.14 | 0.78 |
| Fructosamine | 0.63 | 1.43E-03 | -3.21 | 0.74 | 0.66 | 5.85E-03 | -2.77 | 0.72 |
| Sphingomyelin (d16:1,C18:2) | 0.72 | 1.44E-03 | -3.20 | 0.72 | 0.76 | 8.17E-03 | -2.66 | 0.69 |
| Ceramide (d19:1,C20:0) | 1.44 | 1.69E-03 | 3.15 | 0.68 | 1.43 | 3.74E-03 | 2.91 | 0.67 |
| Octenoylcarnitine | 0.65 | 1.82E-03 | -3.13 | 0.72 | 0.64 | 1.82E-03 | -3.13 | 0.72 |
| Indole-3-acetic acid | 0.69 | 2.06E-03 | -3.09 | 0.66 | 0.71 | 5.88E-03 | -2.76 | 0.65 |
| Ceramide (d19:1,C25:0) | 0.67 | 2.06E-03 | -3.10 | 0.68 | 0.66 | 2.47E-03 | -3.04 | 0.67 |
| Sphingomyelin (d17:1,C24:0) | 0.80 | 2.12E-03 | -3.08 | 0.66 | 0.80 | 3.27E-03 | -2.95 | 0.66 |
| Sphingomyelin (d18:1,C24:2) | 1.23 | 2.23E-03 | 3.07 | 0.62 | 1.23 | 3.43E-03 | 2.93 | 0.61 |
| Plasmanyl-lysophosphatidylcholine (alkyl-C22) | 0.85 | 2.24E-03 | -3.07 | 0.65 | 0.83 | 8.44E-04 | -3.35 | 0.66 |
| Sphingomyelin (d20:1,C24:1) | 1.34 | 2.34E-03 | 3.06 | 0.65 | 1.31 | 7.63E-03 | 2.68 | 0.64 |
| Ketoleucine | 0.82 | 2.43E-03 | -3.04 | 0.66 | 0.75 | 3.66E-05 | -4.15 | 0.71 |
| Ceramide (d18:2,C20:0) | 1.24 | 2.48E-03 | 3.03 | 0.57 | 1.27 | 1.37E-03 | 3.21 | 0.57 |
| Sphingomyelin (d18:1,C22:2) | 1.35 | 2.54E-03 | 3.03 | 0.62 | 1.35 | 3.61E-03 | 2.92 | 0.61 |
| Sphingomyelin (d18:1,C23:0) | 0.83 | 3.06E-03 | -2.97 | 0.67 | 0.83 | 4.96E-03 | -2.82 | 0.67 |
| 3-O-Galactosylglycerol | 0.64 | 3.40E-03 | -2.95 | 0.78 | 0.68 | 1.37E-02 | -2.48 | 0.76 |
| Campesterol | 0.73 | 3.80E-03 | -2.90 | 0.64 | 0.70 | 1.49E-03 | -3.19 | 0.66 |
| Ceramide (d18:1,C18:1) | 1.53 | 3.86E-03 | 2.90 | 0.66 | 1.59 | 2.69E-03 | 3.01 | 0.67 |
| Sphingomyelin (d18:2,C16:0) | 1.11 | 3.87E-03 | 2.90 | 0.62 | 1.11 | 4.85E-03 | 2.82 | 0.64 |
| Ceramide (d16:1,C22:0) | 0.80 | 4.41E-03 | -2.85 | 0.69 | 0.83 | 2.61E-02 | -2.23 | 0.67 |
| Caffeine | 0.46 | 4.52E-03 | -2.85 | 0.73 | 0.53 | 2.79E-02 | -2.21 | 0.70 |
| Threonic acid | 0.77 | 4.53E-03 | -2.85 | 0.65 | 0.79 | 1.10E-02 | -2.55 | 0.65 |
| Ergothioneine | 1.66 | 4.53E-03 | 2.85 | 0.70 | 1.60 | 1.28E-02 | 2.50 | 0.67 |
| Phosphatidylcholine (C16:0,C20:5) | 0.91 | 4.58E-03 | -2.84 | 0.69 | 0.92 | 8.76E-03 | -2.63 | 0.69 |

| | | | | | | | | |
|---|---|---|---|---|---|---|---|---|
| Sphingomyelin (d19:1,C20:0) | 1.34 | 4.68E-03 | 2.84 | 0.66 | 1.33 | 1.03E-02 | 2.57 | 0.66 |
| Cystine | 1.21 | 4.74E-03 | 2.83 | 0.56 | 1.21 | 5.50E-03 | 2.78 | 0.57 |
| Sphingomyelin (d18:2,C24:1) | 1.15 | 4.77E-03 | 2.83 | 0.63 | 1.14 | 9.93E-03 | 2.58 | 0.62 |
| Sphingomyelin (d17:1,C18:0) | 1.19 | 4.81E-03 | 2.83 | 0.59 | 1.21 | 3.67E-03 | 2.91 | 0.60 |
| Ethanolamine plasmalogen (C39:5) | 0.85 | 4.85E-03 | -2.82 | 0.60 | 0.83 | 1.03E-03 | -3.29 | 0.62 |
| Uric acid | 0.84 | 4.87E-03 | -2.82 | 0.68 | 0.86 | 1.66E-02 | -2.40 | 0.67 |
| Sphingomyelin (d18:1,C20:2) | 1.33 | 4.90E-03 | 2.83 | 0.60 | 1.28 | 2.00E-02 | 2.33 | 0.57 |
| Guanidinoacetic acid | 0.82 | 4.95E-03 | -2.82 | 0.66 | 0.83 | 1.12E-02 | -2.54 | 0.65 |
| Xanthurenic acid | 0.84 | 4.95E-03 | -2.82 | 0.71 | 0.85 | 1.36E-02 | -2.47 | 0.69 |
| Allantoin | 0.78 | 4.98E-03 | -2.82 | 0.70 | 0.82 | 3.18E-02 | -2.15 | 0.67 |
| Sphingomyelin (d18:2,C18:0) | 1.17 | 5.04E-03 | 2.81 | 0.58 | 1.18 | 5.83E-03 | 2.76 | 0.58 |
| Sarcosine | 0.89 | 5.04E-03 | -2.81 | 0.66 | 0.89 | 6.84E-03 | -2.71 | 0.66 |
| Lysophosphatidylcholine (C16:0) | 0.94 | 5.13E-03 | -2.81 | 0.66 | 0.94 | 1.25E-02 | -2.51 | 0.66 |
| Sphingomyelin (d16:1,C21:0) | 0.81 | 5.47E-03 | -2.78 | 0.68 | 0.85 | 3.85E-02 | -2.07 | 0.65 |
| Histidine | 0.89 | 5.52E-03 | -2.78 | 0.64 | 0.90 | 1.81E-02 | -2.37 | 0.63 |
| Quinic acid | 0.61 | 5.68E-03 | -2.77 | 0.66 | 0.61 | 7.81E-03 | -2.67 | 0.65 |
| Glyoxylate | 0.78 | 6.10E-03 | -2.75 | 0.67 | 0.76 | 3.56E-03 | -2.93 | 0.69 |
| Butyrylcarnitine | 0.79 | 6.21E-03 | -2.74 | 0.69 | 0.77 | 2.93E-03 | -2.98 | 0.70 |
| Sphingomyelin (d18:2,C18:1) | 1.18 | 6.53E-03 | 2.73 | 0.60 | 1.19 | 6.11E-03 | 2.75 | 0.60 |
| Thiamine phosphate | 0.65 | 6.92E-03 | -2.71 | 0.64 | 0.63 | 5.76E-03 | -2.77 | 0.65 |
| Hippuric acid | 0.62 | 7.20E-03 | -2.69 | 0.64 | 0.62 | 9.75E-03 | -2.59 | 0.64 |
| Spermidine | 0.72 | 7.55E-03 | -2.68 | 0.63 | 0.70 | 6.16E-03 | -2.75 | 0.64 |
| Phosphate (inorganic and from organic phosphates) | 1.11 | 8.39E-03 | 2.64 | 0.66 | 1.13 | 3.09E-03 | 2.97 | 0.69 |
| Sphingomyelin (d18:1,C19:0) | 1.21 | 8.54E-03 | 2.64 | 0.62 | 1.20 | 1.16E-02 | 2.53 | 0.61 |
| Phosphatidylcholine (C18:0,C20:3) | 0.97 | 8.75E-03 | -2.63 | 0.66 | 0.97 | 3.24E-02 | -2.14 | 0.64 |
| Arginine | 0.86 | 8.95E-03 | -2.62 | 0.63 | 0.83 | 3.52E-03 | -2.93 | 0.66 |
| TAG (C18:2,C18:3) | 0.73 | 8.97E-03 | -2.62 | 0.65 | 0.82 | 9.98E-02 | -1.65 | 0.61 |
| Sphingomyelin (d18:2,C24:0) | 0.85 | 9.01E-03 | -2.62 | 0.64 | 0.84 | 6.58E-03 | -2.72 | 0.66 |
| Hexadecenoylcarnitine | 1.34 | 9.83E-03 | 2.59 | 0.67 | 1.31 | 2.08E-02 | 2.32 | 0.66 |
| Sphingosine (d16:1) | 0.79 | 1.04E-02 | -2.57 | 0.65 | 0.81 | 3.44E-02 | -2.12 | 0.63 |
| gamma-Tocopherol | 0.82 | 1.04E-02 | -2.57 | 0.66 | 0.80 | 7.59E-03 | -2.68 | 0.67 |
| Ceramide (d18:2,C22:1) | 1.35 | 1.08E-02 | 2.56 | 0.61 | 1.36 | 1.31E-02 | 2.49 | 0.60 |
| Tiglylcarnitine | 0.78 | 1.16E-02 | -2.53 | 0.66 | 0.76 | 7.90E-03 | -2.67 | 0.67 |
| 7-Ketocholesterol | 0.77 | 1.22E-02 | -2.51 | 0.67 | 0.79 | 2.99E-02 | -2.17 | 0.66 |
| Sphingosine-1-phosphate (d16:1) | 0.85 | 1.24E-02 | -2.51 | 0.63 | 0.88 | 6.07E-02 | -1.88 | 0.61 |
| Ceramide (d18:1,C21:0) | 1.19 | 1.24E-02 | 2.51 | 0.54 | 1.22 | 4.95E-03 | 2.82 | 0.55 |
| Dihydrocholesterol | 1.53 | 1.40E-02 | 2.47 | 0.66 | 1.68 | 4.07E-03 | 2.89 | 0.68 |
| Triacylglyceride hydroperoxide (C16:0,C18:1,C18:2-OOH) | 0.66 | 1.43E-02 | -2.46 | 0.63 | 0.71 | 4.80E-02 | -1.98 | 0.61 |
| Ornithine | 0.88 | 1.47E-02 | -2.44 | 0.61 | 0.88 | 1.58E-02 | -2.42 | 0.60 |

| | | | | | | | | |
|---|---|---|---|---|---|---|---|---|
| Glycerol-3-phosphate, polar fraction | 0.86 | 1.50E-02 | -2.44 | 0.60 | 0.91 | 1.34E-01 | -1.50 | 0.56 |
| Sulfate, lipid fraction | 0.79 | 1.52E-02 | -2.44 | 0.61 | 0.76 | 6.96E-03 | -2.71 | 0.64 |
| Choline phosphate | 0.80 | 1.53E-02 | -2.43 | 0.63 | 0.78 | 9.98E-03 | -2.58 | 0.64 |
| Dodecanol | 1.24 | 1.53E-02 | 2.43 | 0.59 | 1.37 | 7.47E-04 | 3.38 | 0.64 |
| Ceramide (d18:0,C16:0) | 1.34 | 1.55E-02 | 2.43 | 0.59 | 1.41 | 6.22E-03 | 2.75 | 0.59 |
| Choline | 0.88 | 1.56E-02 | -2.42 | 0.62 | 0.89 | 3.57E-02 | -2.10 | 0.62 |
| Glycerate | 0.89 | 1.64E-02 | -2.40 | 0.63 | 0.89 | 1.45E-02 | -2.45 | 0.64 |
| Hexadecanol | 0.92 | 1.68E-02 | -2.40 | 0.63 | 0.92 | 3.17E-02 | -2.15 | 0.62 |
| Sphingomyelin (d18:2,C24:2) | 1.15 | 1.69E-02 | 2.39 | 0.57 | 1.16 | 1.89E-02 | 2.35 | 0.57 |
| Oleoylcarnitine | 1.17 | 1.73E-02 | 2.39 | 0.61 | 1.15 | 4.98E-02 | 1.96 | 0.59 |
| alpha-Tocopherol | 0.83 | 1.76E-02 | -2.38 | 0.64 | 0.84 | 2.87E-02 | -2.19 | 0.63 |
| Uridine | 0.87 | 1.85E-02 | -2.36 | 0.63 | 0.92 | 1.91E-01 | -1.31 | 0.58 |
| Lysophosphatidylcholine (C17:0) | 0.87 | 1.86E-02 | -2.36 | 0.57 | 0.87 | 3.05E-02 | -2.17 | 0.56 |
| Sphingomyelin (d18:2,C18:2) | 0.81 | 1.90E-02 | -2.35 | 0.69 | 0.80 | 2.39E-02 | -2.27 | 0.68 |
| Xylitol | 1.49 | 1.91E-02 | 2.35 | 0.70 | 1.55 | 1.35E-02 | 2.48 | 0.72 |
| Phosphatidylethanolamine (C18:0,C22:6) | 1.24 | 2.05E-02 | 2.32 | 0.57 | 1.29 | 9.24E-03 | 2.61 | 0.60 |
| Sphingomyelin (d18:2,C14:0) | 0.87 | 2.06E-02 | -2.32 | 0.64 | 0.90 | 1.01E-01 | -1.64 | 0.61 |
| Hydroxyoctadecenoylcarnitine | 1.31 | 2.11E-02 | 2.31 | 0.65 | 1.29 | 3.76E-02 | 2.08 | 0.64 |
| Sorbitol | 0.74 | 2.13E-02 | -2.31 | 0.59 | 0.76 | 4.01E-02 | -2.06 | 0.59 |
| N-Acetylcytidine | 1.17 | 2.14E-02 | 2.31 | 0.58 | 1.18 | 1.82E-02 | 2.37 | 0.58 |
| Sphingomyelin (d18:1,C25:1) | 1.21 | 2.34E-02 | 2.27 | 0.64 | 1.26 | 9.73E-03 | 2.59 | 0.67 |
| Decenoylcarnitine | 0.75 | 2.52E-02 | -2.25 | 0.66 | 0.70 | 8.12E-03 | -2.66 | 0.69 |
| 1,5-Anhydrosorbitol | 0.75 | 2.57E-02 | -2.23 | 0.64 | 0.71 | 1.10E-02 | -2.55 | 0.65 |
| Isocitrate | 1.12 | 2.62E-02 | 2.23 | 0.57 | 1.14 | 1.01E-02 | 2.58 | 0.59 |
| Ceramide (d18:0,C23:0) | 0.78 | 2.70E-02 | -2.22 | 0.69 | 0.84 | 1.49E-01 | -1.44 | 0.65 |
| Sphingomyelin (d18:1,C24:0) | 0.87 | 2.79E-02 | -2.20 | 0.61 | 0.87 | 2.59E-02 | -2.23 | 0.62 |
| 3-Deoxyglucosone | 0.83 | 2.84E-02 | -2.20 | 0.66 | 0.84 | 4.32E-02 | -2.03 | 0.68 |
| Mannosamine | 0.79 | 2.86E-02 | -2.19 | 0.65 | 0.77 | 1.72E-02 | -2.39 | 0.66 |
| Ceramide (d19:1,C24:0) | 0.79 | 2.98E-02 | -2.18 | 0.64 | 0.78 | 2.76E-02 | -2.21 | 0.64 |
| Valine | 0.92 | 3.04E-02 | -2.17 | 0.65 | 0.91 | 1.52E-02 | -2.43 | 0.66 |
| Gluconic acid | 0.86 | 3.16E-02 | -2.15 | 0.62 | 0.86 | 3.21E-02 | -2.15 | 0.63 |
| Ceramide (d18:0,C24:1) | 1.28 | 3.52E-02 | 2.11 | 0.56 | 1.35 | 1.23E-02 | 2.51 | 0.59 |
| dihomo-gamma-Linolenic acid (C20:cis[8,11,14]3) | 0.85 | 3.53E-02 | -2.11 | 0.63 | 0.92 | 3.19E-01 | -1.00 | 0.58 |
| Threonine | 0.90 | 3.61E-02 | -2.10 | 0.62 | 0.89 | 3.02E-02 | -2.17 | 0.64 |
| Furoylglycine | 0.60 | 3.65E-02 | -2.10 | 0.63 | 0.66 | 1.03E-01 | -1.63 | 0.62 |
| O-Acetylcarnitine | 1.16 | 3.67E-02 | 2.09 | 0.59 | 1.12 | 1.09E-01 | 1.61 | 0.57 |
| Cholesterylester C16:1 | 0.80 | 3.68E-02 | -2.09 | 0.59 | 0.84 | 1.25E-01 | -1.54 | 0.57 |
| Nervonic acid (C24:cis[15]1) | 1.18 | 3.71E-02 | 2.09 | 0.63 | 1.28 | 3.54E-03 | 2.92 | 0.67 |
| Sphingomyelin (d17:1,C24:1) | 1.13 | 3.82E-02 | 2.08 | 0.60 | 1.14 | 2.93E-02 | 2.18 | 0.60 |
| Ceramide (d19:1,C23:0) | 0.78 | 3.83E-02 | -2.08 | 0.67 | 0.79 | 5.81E-02 | -1.90 | 0.66 |

| | | | | | | | | |
|---|---|---|---|---|---|---|---|---|
| Phenylacetylglutamine | 0.74 | 4.00E-02 | -2.06 | 0.58 | 0.73 | 3.87E-02 | -2.07 | 0.59 |
| Sphingomyelin (d18:1,C17:0) | 1.17 | 4.02E-02 | 2.06 | 0.64 | 1.17 | 5.16E-02 | 1.95 | 0.64 |
| Sphinganine-1-phosphate (d18:0) | 0.86 | 4.07E-02 | -2.05 | 0.62 | 0.84 | 2.08E-02 | -2.32 | 0.63 |
| erythro-Dihydrosphingosine (d18:0) | 1.23 | 4.07E-02 | 2.05 | 0.56 | 1.38 | 2.00E-03 | 3.10 | 0.61 |
| myo-Inositol | 0.90 | 4.22E-02 | -2.03 | 0.64 | 0.91 | 8.96E-02 | -1.70 | 0.63 |
| 1-Methylxanthine | 0.76 | 4.36E-02 | -2.02 | 0.68 | 0.80 | 1.12E-01 | -1.59 | 0.65 |
| 5-O-Methylsphingosine (d16:1) | 0.83 | 4.43E-02 | -2.01 | 0.59 | 0.91 | 3.43E-01 | -0.95 | 0.54 |
| Thiamine | 0.86 | 4.44E-02 | -2.01 | 0.62 | 0.86 | 6.12E-02 | -1.87 | 0.61 |
| N-Phenylacetylglycine | 0.75 | 4.50E-02 | -2.01 | 0.60 | 0.72 | 2.62E-02 | -2.23 | 0.61 |
| 5-Methyl-2'-deoxycytidine | 0.84 | 4.73E-02 | -1.99 | 0.64 | 0.83 | 5.01E-02 | -1.96 | 0.63 |
| Indole-3-propionic acid | 0.59 | 4.82E-02 | -1.98 | 0.59 | 0.62 | 9.11E-02 | -1.69 | 0.58 |
| conjugated Octadecadienoic acid (C18:2) No 02 | 0.75 | 4.83E-02 | -1.98 | 0.64 | 0.76 | 7.55E-02 | -1.78 | 0.64 |
| Creatinine | 0.84 | 4.85E-02 | -1.98 | 0.65 | 0.82 | 2.56E-02 | -2.24 | 0.66 |
| Ceramide (d20:1,C24:1) | 1.22 | 4.86E-02 | 1.98 | 0.60 | 1.17 | 1.41E-01 | 1.48 | 0.56 |
| Carnitine | 0.90 | 4.98E-02 | -1.96 | 0.62 | 0.90 | 8.03E-02 | -1.75 | 0.60 |
| N,N-Dimethylarginine (ADMA) | 0.79 | 5.09E-02 | -1.95 | 0.62 | 0.76 | 2.68E-02 | -2.22 | 0.64 |
| Capsanthin | 0.73 | 5.12E-02 | -1.95 | 0.62 | 0.66 | 1.26E-02 | -2.50 | 0.64 |
| Ceramide (d17:1,C20:0) | 1.20 | 5.30E-02 | 1.94 | 0.58 | 1.21 | 5.52E-02 | 1.92 | 0.57 |
| Behenic acid (C22:0) | 0.87 | 5.30E-02 | -1.94 | 0.58 | 0.92 | 3.00E-01 | -1.04 | 0.54 |
| Ceramide (d18:2,C24:1) | 1.13 | 5.30E-02 | 1.94 | 0.54 | 1.13 | 5.49E-02 | 1.92 | 0.54 |
| N-Acetylglycine | 1.37 | 5.57E-02 | 1.92 | 0.69 | 1.22 | 2.48E-01 | 1.16 | 0.66 |
| Sphingomyelin (d18:2,C26:0) | 0.86 | 5.64E-02 | -1.91 | 0.59 | 0.85 | 6.09E-02 | -1.88 | 0.60 |
| Eicosapentaenoic acid (C20:5) No 01 | 0.68 | 5.76E-02 | -1.90 | 0.66 | 0.66 | 4.80E-02 | -1.98 | 0.66 |
| Ceramide (d18:0,C24:0) | 0.79 | 5.85E-02 | -1.90 | 0.68 | 0.85 | 2.02E-01 | -1.28 | 0.65 |
| erythro-Dihydrosphingosine (d16:0) | 0.82 | 5.85E-02 | -1.89 | 0.59 | 0.92 | 4.16E-01 | -0.81 | 0.54 |
| beta-Sitosterol | 0.81 | 5.91E-02 | -1.89 | 0.61 | 0.83 | 1.20E-01 | -1.56 | 0.58 |
| Serine, lipid fraction | 0.86 | 6.09E-02 | -1.88 | 0.58 | 0.85 | 5.41E-02 | -1.93 | 0.59 |
| Ceramide (d16:1,C21:0) | 0.81 | 6.27E-02 | -1.86 | 0.66 | 0.86 | 1.84E-01 | -1.33 | 0.65 |
| Cholic acid | 0.62 | 6.29E-02 | -1.86 | 0.60 | 0.62 | 7.98E-02 | -1.75 | 0.60 |
| Canthaxanthin | 0.79 | 6.31E-02 | -1.86 | 0.62 | 0.79 | 7.55E-02 | -1.78 | 0.61 |
| Ketovaline | 0.88 | 6.35E-02 | -1.86 | 0.66 | 0.85 | 2.34E-02 | -2.27 | 0.69 |
| Lysine | 0.92 | 6.51E-02 | -1.85 | 0.64 | 0.92 | 4.84E-02 | -1.98 | 0.65 |
| Isoguanine | 0.73 | 6.56E-02 | -1.85 | 0.70 | 0.71 | 6.63E-02 | -1.84 | 0.70 |
| Sebacic acid (Dicarboxylic acid; C10:0) | 1.43 | 6.63E-02 | 1.84 | 0.65 | 1.37 | 1.24E-01 | 1.54 | 0.64 |
| Sphingomyelin (d16:1,C20:0) | 0.89 | 6.64E-02 | -1.84 | 0.62 | 0.92 | 2.05E-01 | -1.27 | 0.59 |
| Ceramide (d16:1,C18:0) | 1.16 | 6.67E-02 | 1.84 | 0.51 | 1.22 | 1.76E-02 | 2.38 | 0.53 |
| Cholesta-1,3,5-triene | 0.86 | 6.76E-02 | -1.83 | 0.61 | 0.90 | 2.12E-01 | -1.25 | 0.58 |
| Ceramide (d18:0,C20:0) | 1.26 | 6.90E-02 | 1.82 | 0.55 | 1.33 | 3.61E-02 | 2.10 | 0.57 |

| | | | | | | | | |
|---|---|---|---|---|---|---|---|---|
| Phosphatidylcholine (C18:0,C22:6) | 1.08 | 6.90E-02 | 1.82 | 0.58 | 1.10 | 3.16E-02 | 2.15 | 0.61 |
| Cortisol | 1.17 | 7.08E-02 | 1.81 | 0.62 | 1.10 | 2.72E-01 | 1.10 | 0.58 |
| Cholesterol, total | 0.82 | 7.22E-02 | -1.80 | 0.63 | 0.86 | 1.98E-01 | -1.29 | 0.60 |
| Malonylcarnitine | 1.37 | 7.26E-02 | 1.80 | 0.60 | 1.35 | 9.75E-02 | 1.66 | 0.59 |
| Ethanolamine plasmalogen (C39:4) | 0.94 | 7.36E-02 | -1.79 | 0.58 | 0.92 | 4.08E-02 | -2.05 | 0.58 |
| Lauric acid (C12:0) | 0.79 | 7.39E-02 | -1.79 | 0.58 | 0.77 | 5.40E-02 | -1.93 | 0.59 |
| Lysophosphatidylcholine (C20:4) | 0.91 | 7.58E-02 | -1.78 | 0.55 | 0.91 | 7.80E-02 | -1.77 | 0.55 |
| 2-Methylserine | 0.91 | 7.64E-02 | -1.77 | 0.61 | 0.90 | 5.94E-02 | -1.89 | 0.61 |
| Lignoceric acid (C24:0) | 0.88 | 8.00E-02 | -1.75 | 0.58 | 0.90 | 1.61E-01 | -1.40 | 0.57 |
| Cholesta-3,5,7-triene | 0.83 | 8.12E-02 | -1.75 | 0.64 | 0.83 | 1.05E-01 | -1.63 | 0.64 |
| Sphingomyelin (d16:1,C20:1) | 1.17 | 8.25E-02 | 1.74 | 0.59 | 1.18 | 7.39E-02 | 1.79 | 0.61 |
| Phosphocreatine | 0.82 | 8.38E-02 | -1.73 | 0.59 | 0.77 | 3.30E-02 | -2.13 | 0.61 |
| Phosphatidylcholine hydroperoxide (C16:0,C18:2-OOH) | 1.25 | 8.43E-02 | 1.73 | 0.61 | 1.32 | 3.59E-02 | 2.10 | 0.64 |
| Hydroxyhexadecenoylcarnitine | 1.17 | 8.54E-02 | 1.72 | 0.61 | 1.18 | 7.46E-02 | 1.79 | 0.61 |
| Sphingomyelin (d16:1,C16:0) | 0.89 | 8.60E-02 | -1.72 | 0.62 | 0.92 | 2.49E-01 | -1.15 | 0.59 |
| Trimethylamine-N-oxide (TMAO) | 1.34 | 8.77E-02 | 1.71 | 0.59 | 1.38 | 7.14E-02 | 1.81 | 0.60 |
| Glycine | 0.93 | 8.96E-02 | -1.70 | 0.51 | 0.91 | 2.56E-02 | -2.24 | 0.54 |
| TAG (C16:0,C18:1,C18:2) | 1.14 | 9.12E-02 | 1.69 | 0.54 | 1.18 | 4.74E-02 | 1.99 | 0.56 |
| Ceramide (d17:1,C24:1) | 1.11 | 9.29E-02 | 1.68 | 0.53 | 1.13 | 6.28E-02 | 1.86 | 0.54 |
| Pipecolic acid | 0.90 | 9.72E-02 | -1.66 | 0.56 | 0.89 | 6.69E-02 | -1.83 | 0.58 |
| 5-Methylcytidine | 0.92 | 9.72E-02 | -1.66 | 0.61 | 0.90 | 7.05E-02 | -1.81 | 0.61 |
| Sphingomyelin (d18:2,C23:1) | 1.11 | 1.01E-01 | 1.64 | 0.55 | 1.14 | 5.08E-02 | 1.96 | 0.57 |
| Tyrosine | 0.94 | 1.02E-01 | -1.64 | 0.65 | 0.94 | 1.13E-01 | -1.59 | 0.65 |
| Glucose | 0.91 | 1.06E-01 | -1.62 | 0.62 | 0.91 | 1.29E-01 | -1.52 | 0.62 |
| Sphingomyelin (d18:2,C19:0) | 1.11 | 1.06E-01 | 1.62 | 0.56 | 1.10 | 1.36E-01 | 1.49 | 0.56 |
| Pyrogallol | 0.78 | 1.07E-01 | -1.62 | 0.63 | 0.72 | 4.46E-02 | -2.02 | 0.66 |
| Sphingomyelin (d18:2,C20:2) | 1.16 | 1.08E-01 | 1.61 | 0.54 | 1.14 | 1.67E-01 | 1.38 | 0.53 |
| Sphingomyelin (d18:1,C20:0) | 1.10 | 1.09E-01 | 1.60 | 0.56 | 1.10 | 1.26E-01 | 1.53 | 0.54 |
| 18-Hydroxy-11-deoxycorticosterone | 0.83 | 1.12E-01 | -1.59 | 0.55 | 0.77 | 3.17E-02 | -2.15 | 0.58 |
| trans-4-Hydroxyproline | 0.89 | 1.14E-01 | -1.58 | 0.58 | 0.86 | 5.12E-02 | -1.95 | 0.60 |
| Ceramide (d18:2,C14:0) | 0.87 | 1.15E-01 | -1.58 | 0.65 | 0.91 | 2.90E-01 | -1.06 | 0.62 |
| Octadecanedioic acid | 1.14 | 1.15E-01 | 1.58 | 0.59 | 1.20 | 2.83E-02 | 2.20 | 0.63 |
| Cholesterylester C20:4 | 0.90 | 1.17E-01 | -1.57 | 0.53 | 0.86 | 3.06E-02 | -2.16 | 0.56 |
| Sphingomyelin (d18:2,C26:1) | 1.15 | 1.22E-01 | 1.55 | 0.60 | 1.18 | 8.25E-02 | 1.74 | 0.63 |
| Cholesterylester, total | 0.98 | 1.26E-01 | -1.53 | 0.62 | 0.98 | 2.12E-01 | -1.25 | 0.62 |
| Sphingomyelin (d18:2,C17:0) | 1.12 | 1.26E-01 | 1.53 | 0.57 | 1.12 | 1.47E-01 | 1.45 | 0.57 |
| Sphingomyelin (d17:1,C16:0) | 1.10 | 1.26E-01 | 1.53 | 0.57 | 1.10 | 1.25E-01 | 1.54 | 0.58 |
| Myristic acid (C14:0) | 0.83 | 1.30E-01 | -1.52 | 0.57 | 0.96 | 7.28E-01 | -0.35 | 0.52 |

| | | | | | | | | |
|---|---|---|---|---|---|---|---|---|
| Ceramide (d20:1,C24:0) | 0.82 | 1.31E-01 | -1.51 | 0.60 | 0.79 | 8.60E-02 | -1.72 | 0.63 |
| Sphingomyelin (d18:1,C16:0) | 1.05 | 1.31E-01 | 1.51 | 0.57 | 1.04 | 3.06E-01 | 1.02 | 0.54 |
| Cysteine | 0.95 | 1.36E-01 | -1.49 | 0.64 | 0.94 | 6.36E-02 | -1.86 | 0.66 |
| Linolenic acid (C18:cis[9,12,15]3) | 0.87 | 1.36E-01 | -1.49 | 0.57 | 0.91 | 3.20E-01 | -0.99 | 0.55 |
| Phosphatidylcholine (C16:0,C20:4) | 0.99 | 1.39E-01 | -1.48 | 0.55 | 1.00 | 3.39E-01 | -0.96 | 0.54 |
| Tetradecenoylcarnitine | 1.28 | 1.39E-01 | 1.48 | 0.62 | 1.21 | 2.92E-01 | 1.05 | 0.60 |
| Adenine | 1.17 | 1.39E-01 | 1.48 | 0.61 | 1.23 | 7.22E-02 | 1.80 | 0.65 |
| Sphingomyelin (d18:2,C22:0) | 0.92 | 1.40E-01 | -1.48 | 0.60 | 0.91 | 1.37E-01 | -1.49 | 0.61 |
| Sphingomyelin (d18:1,C26:2) | 1.15 | 1.45E-01 | 1.46 | 0.60 | 1.16 | 1.41E-01 | 1.47 | 0.61 |
| scyllo-Inositol | 0.86 | 1.47E-01 | -1.45 | 0.61 | 0.86 | 1.62E-01 | -1.40 | 0.60 |
| Phosphatidylcholine No 02 | 0.97 | 1.47E-01 | -1.45 | 0.55 | 0.97 | 1.03E-01 | -1.63 | 0.56 |
| Phosphatidylcholine (C18:1,C18:2) | 0.99 | 1.49E-01 | -1.45 | 0.56 | 0.99 | 5.98E-02 | -1.88 | 0.59 |
| Sphingomyelin (d18:2,C22:2) | 1.14 | 1.63E-01 | 1.40 | 0.53 | 1.14 | 1.96E-01 | 1.29 | 0.54 |
| Cresol sulfate | 0.72 | 1.66E-01 | -1.38 | 0.56 | 0.69 | 1.34E-01 | -1.50 | 0.57 |
| Methylmalonylcarnitine | 0.87 | 1.69E-01 | -1.38 | 0.59 | 0.85 | 1.12E-01 | -1.59 | 0.60 |
| 3-Hydroxyhippuric acid | 0.88 | 1.73E-01 | -1.37 | 0.58 | 0.84 | 9.56E-02 | -1.67 | 0.59 |
| Choline plasmalogen (C18-vinyl,C20:4) | 0.94 | 1.74E-01 | -1.36 | 0.52 | 0.89 | 1.43E-02 | -2.45 | 0.57 |
| Linoleic acid (C18:cis[9,12]2) | 0.90 | 1.77E-01 | -1.35 | 0.56 | 0.95 | 5.40E-01 | -0.61 | 0.52 |
| Sphingomyelin (d18:1,C26:1) | 1.14 | 1.83E-01 | 1.33 | 0.62 | 1.17 | 1.31E-01 | 1.51 | 0.63 |
| Sphingomyelin (d18:2,C20:0) | 1.08 | 1.83E-01 | 1.33 | 0.51 | 1.08 | 1.83E-01 | 1.33 | 0.51 |
| Asparagine | 0.93 | 1.91E-01 | -1.31 | 0.59 | 0.92 | 1.84E-01 | -1.33 | 0.61 |
| Hexoses | 0.94 | 1.97E-01 | -1.29 | 0.59 | 0.95 | 3.05E-01 | -1.03 | 0.58 |
| Ketoisoleucine | 0.91 | 1.99E-01 | -1.29 | 0.57 | 0.86 | 5.18E-02 | -1.95 | 0.60 |
| Cholesterylester C16:0 | 0.88 | 2.15E-01 | -1.24 | 0.51 | 0.84 | 9.84E-02 | -1.66 | 0.53 |
| Pyruvate | 0.92 | 2.24E-01 | -1.22 | 0.58 | 0.89 | 1.21E-01 | -1.55 | 0.61 |
| Docosahexaenoic acid (C22:cis[4,7,10,13,16,19]6) | 1.12 | 2.25E-01 | 1.21 | 0.56 | 1.20 | 5.62E-02 | 1.91 | 0.60 |
| Sphingomyelin (d16:1,C22:1) | 1.09 | 2.27E-01 | 1.21 | 0.54 | 1.14 | 6.58E-02 | 1.84 | 0.57 |
| Urea | 0.93 | 2.35E-01 | -1.19 | 0.59 | 0.90 | 1.18E-01 | -1.56 | 0.61 |
| Hydroxypentanoylcarnitine | 0.89 | 2.37E-01 | -1.18 | 0.59 | 0.88 | 1.98E-01 | -1.29 | 0.59 |
| TAG (C18:1,C18:2) | 1.08 | 2.40E-01 | 1.18 | 0.50 | 1.11 | 1.47E-01 | 1.45 | 0.52 |
| Docosapentaenoic acid (C22:cis[4,7,10,13,16]5) | 1.14 | 2.40E-01 | 1.18 | 0.51 | 1.22 | 8.54E-02 | 1.72 | 0.54 |
| Carnosine | 1.20 | 2.60E-01 | 1.13 | 0.53 | 1.26 | 1.71E-01 | 1.37 | 0.54 |
| Benzoic acid | 1.10 | 2.85E-01 | 1.07 | 0.58 | 1.17 | 9.89E-02 | 1.65 | 0.63 |
| 5-O-Methylsphingosine (d18:1) | 1.09 | 3.11E-01 | 1.01 | 0.56 | 1.16 | 9.67E-02 | 1.66 | 0.58 |
| Octanoylcarnitine | 0.90 | 3.24E-01 | -0.99 | 0.58 | 0.82 | 6.17E-02 | -1.87 | 0.63 |
| Glycochenodeoxycholic acid | 1.22 | 3.58E-01 | 0.92 | 0.52 | 1.47 | 8.45E-02 | 1.73 | 0.57 |
| ratio of phospholipids to glycerolipids | 1.07 | 3.74E-01 | 0.89 | 0.57 | 1.03 | 7.50E-01 | 0.32 | 0.55 |

| | | | | | | | | |
|---|---|---|---|---|---|---|---|---|
| 2-Hydroxypalmitic acid (C16:0) | 1.05 | 3.88E-01 | 0.86 | 0.55 | 1.10 | 1.12E-01 | 1.59 | 0.57 |
| 3-O-Methylsphingosine (d18:1) | 1.08 | 4.16E-01 | 0.81 | 0.54 | 1.16 | 1.39E-01 | 1.48 | 0.57 |
| Decanoylcarnitine | 0.89 | 4.33E-01 | -0.79 | 0.57 | 0.81 | 1.90E-01 | -1.31 | 0.60 |
| erythro-Sphingosine (d18:1) | 1.05 | 5.02E-01 | 0.67 | 0.55 | 1.12 | 1.76E-01 | 1.35 | 0.58 |
| threo-Sphingosine (d18:1) | 1.05 | 5.24E-01 | 0.64 | 0.54 | 1.12 | 1.53E-01 | 1.43 | 0.57 |
| Oleic acid (C18:cis[9]1) | 1.05 | 5.25E-01 | 0.64 | 0.52 | 1.12 | 1.72E-01 | 1.37 | 0.56 |
| Ceramide (d16:1,C20:0) | 1.05 | 5.29E-01 | 0.63 | 0.54 | 1.11 | 2.00E-01 | 1.28 | 0.51 |
| Heptadecenoic acid (C17:cis[10]1) | 1.04 | 6.68E-01 | 0.43 | 0.51 | 1.16 | 1.11E-01 | 1.60 | 0.54 |
| Phosphate, lipid fraction | 1.02 | 7.56E-01 | 0.31 | 0.50 | 1.08 | 1.58E-01 | 1.41 | 0.55 |

**Example 4:** Biomarker panel for diagnosis of pancreatic cancer

The diagnostic biomarkers of the biomarker panels allowing for diagnosis of pancreatic cancer versus diabetic non-pancreatic control, were manually selected and optimized according to their discriminating performance both multivariate and univariate and the feasibility of their concomitant analysis in a single analytical approach. These pre-defined panels were then tested for their discrimination performance using Elastic Net algorithm combined with ROC curve analysis. Preferred parameters obtained for preferred panels are shown in Table 2.

The biomarker panels that were identified for diagnosis of pancreatic cancer consist of a most preferred core panel that comprises in addition to CA19-9 at least one small molecule biomarker from each of the metabolite classes carnitines, amino acids, energy metabolism, sphingolipid ratios as shown in Table 4. Frequently, the biomarker panels comprised in addition to CA19-9 at least one small molecule biomarker from each of the metabolite classes carnitines, amino acids, energy metabolism, sphingolipid ratios, and lipids as shown in Table 4; optionally, in extended panels, other lipids, in particular ratio of phospholipids to glycerolipids or Lysophosphatidyl-ethanolamine (C18:2) were determined in addition.

Table 4: Core panel definition for diagnosis of pancreatic cancer. CA19-9 was analyzed in any panel.

| Carnitine ('C') | Amino acid ('A') | Energy metabolite ('E') | Sphingolipid ('S') |
|---|---|---|---|
| Propionyl-carnitine Butyryl-carnitine Octenoyl-carnitine | Histidine Proline | 2-Oxoglutarate CoQ9 | (Cer d18:1,C16:0) ratio to (Cer d18:1,C24:0) (Cer d18:1,C18:0) ratio to (Cer d18:1,C24:0) (Cerd18:1,C16:0 plus Cer d18:1,C18:0) ratio to (Cer d18:1,C24:0) (SM d16:1,C18:0) ratio to (SM d16:1,C24:0) (SM d17:1,C18:0) ratio to (SM d 17:1,C24:0) (SM d18:2,C18:0) ratio to (SM d18:2,C24:0) |

**Claims**

1. A method for diagnosing pancreatic cancer in a subject suspected to suffer from pancreatic cancer comprising the steps of:

(a) determining in a sample of said subject the value of (i) at least one biomarker of each of the categories carnitines, amino acids, and mitochondrial energy metabolites; and (ii) a first and a second sphingolipid, wherein the fatty acid residue of the first sphingolipid is at least four carbon atoms longer than the fatty acid residue of the second sphingolipid, and wherein the sphingosine backbone of the first sphingolipid and the second sphingolipid are identical; and

(b) comparing the values of the biomarkers with references, whereby pancreatic cancer is diagnosed.

2. The method of claim 1, wherein said subject is a subject suffering from new-onset diabetes.

3. The method of claim 1 or 2, wherein said carnitine biomarker is selected from the list consisting of Propionyl-carnitine, Butyryl-carnitine, and Octenoyl-carnitine;
wherein said amino acid biomarker is selected from Histidine and Proline;
wherein said mitochondrial energy metabolite biomarker is selected from 2-Oxoglutarate and Coenzyme Q9; and/or
wherein said first and second sphingolipid biomarker are selected from the list consisting of (i) Ceramide (Cer) d18:1,C16:0) and Cer (d18:1,C24:0); (ii) Cer (d18:1,C18:0) and Cer (d18:1,C24:0); (iii) (Cer (d18:1,C16:0) plus Cer d18:1,C18:0)) and Cer (d18:1,C24:0); (iv) Sphingomyelin (SM) (d16:1,C18:0) and SM (d16:1,C24:0); (v) SM (d17:1,C18:0) and SM (d17:1,C24:0); and (vi) SM (d18:2,C18:0) to SM (d18:2,C24:0) ratio.

4. The method of any one of claims 1 to 4, the biomarkers determined in step a) comprise the biomarkers of a panel selected from panels ACES_1, ACES_25, ACELS_9, ACELS_97 of Table 1.

5. The method of any one of claims 1 to 4, wherein a further lipid biomarker, preferably selected from total phospholipids to total glycerolipids ratio and Lysophosphatidyl-ethanolamine (C18:2), is determined.

6. The method of any one of claims 1 to 5, wherein said method further comprises determining the amount of CA 19-9 in said sample or comprises providing a value of CA19-9 of said subject in step a) and comparing said value of CA19-9 to a reference in step b).

7. The method of any one of claims 1 to 6, wherein step a) comprises determining at least one marker panel from Table 1.

8. The method of any one of claims 1 to 7, wherein said pancreatic cancer is resectable pancreatic cancer.

9. The method of any one of claims 1 to 8, wherein said determining the amount of said biomarker of said sample comprises mass spectrometry.

10. A method for identifying whether a subject is in need of a pancreatic cancer therapy comprising the steps of the method of any one of claims 1 to 9 and the further step of identifying a subject in need of a pancreatic cancer therapy if said subject is diagnosed to suffer from pancreatic cancer, preferably wherein said pancreatic cancer therapy comprises surgery, radiotherapy, and/or drug treatment.

11. The method of any one of claims 1 to 10, wherein said sample is a plasma, blood or serum sample.

12. A device for diagnosing pancreatic cancer in a sample of a subject comprising:

(a) an analyzing unit for the sample of the subject comprising a detector for the amounts of at least one biomarker each of the categories carnitines, amino acids, energy metabolites, and sphingolipids; said detector allowing for the determination of the amounts of the biomarkers of the said group of biomarkers in the sample; and operatively linked thereto,
(b) an evaluation unit comprising a data processing unit and a data base, said data base comprising a stored reference and said data processing unit having tangibly embedded an algorithm for carrying out a comparison of the amounts of the biomarkers of the group of biomarkers determined by the analyzing unit and the stored reference and for generating an output information based on which the diagnosis can be established.

13. The device of claim 12, further comprising, operatively linked to the evaluation unit, at least one input unit for inputting the amount of the marker CA 19-9 and/or an analyzing unit for the sample of the subject comprising a detector for the amount of CA 19-9 in said sample.

14. A data collection, preferably tangibly embedded on a data carrier, comprising characteristic values of at least the markers of at least one panel according to the method of any one of claims 1 to 11, being indicative for a subject suffering from pancreatic cancer, or not.

**15.** Use of a panel of biomarkers comprising at least one biomarker each of the categories carnitines, amino acids, energy metabolites, sphingolipids, and CA 19-9 or detection agents therefor in a sample of a subject suspected to suffer from pancreatic cancer for diagnosing pancreatic cancer.

Europäisches
Patentamt

European
Patent Office

Office européen
des brevets

# EUROPEAN SEARCH REPORT

Application Number

EP 17 20 9024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/091962 A1 (METANOMICS HEALTH GMBH) 25 June 2015 (2015-06-25) * page 17, line 31 - page 19; claims 1-15; tables 1-28 * | 1-15 | INV. G01N33/574 G01N33/68 |
| X,D | WO 2013/079594 A1 (METANOMICS HEALTH GMBH [DE]; UKSH SCHLESWIG HOLSTEIN [DE]; UNIV ERNST) 6 June 2013 (2013-06-06) * page 17, line 16 - page 19, line 4; claim 14 * | 12,13 | |
| X | WO 2016/207391 A1 (METANOMICS HEALTH GMBH [DE]) 29 December 2016 (2016-12-29) * page 44, line 1 - page 45, line 20; claim 14 * | 12,13 | |

TECHNICAL FIELDS
SEARCHED    (IPC)

G01N

The present search report has been drawn up for all claims

| Place of search | Date of completion of the search | Examiner |
|---|---|---|
| The Hague | 5 June 2018 | Wiesner, Martina |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

               

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 20 9024

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2018

| Patent document cited in search report | | | Publication date | Patent family member(s) | | | Publication date |
|---|---|---|---|---|---|---|---|
| WO 2015091962 | A1 | | 25-06-2015 | AU | 2014368412 | A1 | 07-07-2016 |
| | | | | CA | 2934012 | A1 | 25-06-2015 |
| | | | | EP | 3084443 | A1 | 26-10-2016 |
| | | | | JP | 2017504011 | A | 02-02-2017 |
| | | | | US | 2016313338 | A1 | 27-10-2016 |
| | | | | WO | 2015091962 | A1 | 25-06-2015 |
| WO 2013079594 | A1 | | 06-06-2013 | AU | 2012343843 | A1 | 12-06-2014 |
| | | | | CA | 2857401 | A1 | 06-06-2013 |
| | | | | EP | 2786152 | A1 | 08-10-2014 |
| | | | | JP | 6219300 | B2 | 25-10-2017 |
| | | | | JP | 2015502541 | A | 22-01-2015 |
| | | | | JP | 2018049016 | A | 29-03-2018 |
| | | | | US | 2014323352 | A1 | 30-10-2014 |
| | | | | WO | 2013079594 | A1 | 06-06-2013 |
| WO 2016207391 | A1 | | 29-12-2016 | AU | 2016282362 | A1 | 18-01-2018 |
| | | | | CA | 2990316 | A1 | 29-12-2016 |
| | | | | EP | 3314264 | A1 | 02-05-2018 |
| | | | | WO | 2016207391 | A1 | 29-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011151252 A
- WO 2013079594 A
- US 4540884 A
- US 5397894 A
- WO 03073464 A
- US 7196323 B

### Non-patent literature cited in the description

- **EVERHART.** *Gastroenterology,* 2009, vol. 136, 1134-11449
- **DEWITT.** *Gastroenterol Hepatol.,* 2006, vol. 4, 717-25
- **FRY.** *Langenbecks Arch Surg.,* 2008, vol. 393, 883-90
- **GUPTA et al.** *Cancer,* 1985, vol. 56, 277-283
- **HRUBAN.** *Am J Surg Path,* 2004, vol. 28, 977-987
- **LOWENFELS.** *N Engl J Med,* 1993, vol. 328, 1433-1437
- **HOWES.** *Clinical Gastroenterology and Hepatology,* 2004, vol. 2, 252-261
- **LOWENFELS.** *JAMA,* 2001, vol. 286, 169-170
- **LOWENFELS.** *J Natl Cancer Inst,* 1997, vol. 89, 442-44656
- **SAH et al.** *Nat Rev Gastroenterol Hepatol,* 2013, vol. 10, 423-433
- **MUNIRAJ ; CHARI.** *Minerva gastroenterologica e dietologica,* 2012, vol. 58, 331-345
- **CHARI et al.** *Gastroenterology,* 2008, vol. 134, 95-101
- **MUNIGALA et al.** *Clin Transl Gastroenterol,* 2015, vol. 6, e118
- **CHARI et al.** *Gastroenterology,* 2005, vol. 129, 504-511
- **CHOE et al.** *Pancreas,* 2016, vol. 45, 730-734
- **SCHRADER.** *Pancreas,* 2009, vol. 38, 416-421
- **PITSON.** *Trend Biochem Sci,* 2011, vol. 36, 97-107
- **MODRAK.** *Mol Cancer Res,* 2009, vol. 7, 890-896
- **DOWDY ; WEARDEN.** Statistics for Research. John Wiley & Sons, 1983
- **NISSEN.** *Journal of Chromatography A,* 1995, vol. 703, 37-57
- **CHRISTIE.** *Journal of Lipid Research,* 1985, vol. 26, 507-512
- **SCHMIDT H.** *Prostaglandins & other Lipid Mediators,* 2006, vol. 81, 162-170
- **BRAND et al.** *Gut,* 2007, vol. 56, 1460-9
- **DEL CHIARO et al.** *World J Gastroenterol,* 2014, vol. 20, 12118-12131
- **VAN RAVENZWAAY, B. et al.** The use of metabolomics for the discovery of new biomarkers of effect. *Toxicol Lett,* 2007, vol. 172, 21-8
- **ROESSNER, U. ; WAGNER, C. ; KOPKA, J. ; TRETHEWEY, R.N. ; WILLMITZER, L.** Technical advance: simultaneous analysis of metabolites in potato tuber by gas chromatography-mass spectrometry. *Plant J,* 2000, vol. 23, 131-42
- **MUTCH, D.M. et al.** Metabolite profiling identifies candidate markers reflecting the clinical adaptations associated with Roux-en-Y gastric bypass surgery. *PLoS One,* 2009, vol. 4, e7905
- **CHRISTIE.** Rapid separation and quantification of lipid classes by high performance liquid chromatography and mass (light-scattering) detection. *J Lipid Res,* 1985, vol. 26, 507-12
- **ZOU ; HASTIE.** Regularization and variable selection via the elastic net. *Journal of the Royal Statistical Society,* 2005, vol. 67, 301-320
- **ZHOU ; OBUCHOWSKI ; MCCLISH.** Statistical Methods in Diagnostic Medicine. 2011